# EUROPÄISCHE PATENTANMELDUNG

(11) **EP 1 774 953 A2**
(43) Veröffentlichungstag der Anmeldung: **18.04.2007**
(21) Anmeldenummer: 06011569.8
(22) Anmeldetag: 03.06.2006
(51) Int. Cl.: A61K 8/34, A61K 8/42, A61K 8/97, A61Q 11/00

(54) **Mundwasserkonzentrate**

(30) Priorität: 17.10.2005 DE 102005049974
(71) Anmelder: Henkel Kommanditgesellschaft auf Aktien, 40589 Düsseldorf (DE)
(72) Erfinder: Bernecker, Ullrich, 52074 Aachen (DE); Barth, Adolf Peter, 42781 Haan (DE)

(57) **Zusammenfassung**

Mundwasserkonzentrate, die Ethanol in bestimmten Mengen und mindestens eine scharf schmeckende und/oder ein Gefühl von Wärme erzeugende Substanz enthalten, vermitteln bei der Anwendung ein angenehmes Gefühl von Wärme und Schärfe, ohne daß dieses Gefühl als unangenehm empfunden wird. Zudem werden diese Produkte vom Verbraucher als leistungsstark empfunden.

## Beschreibung

Die Erfindung betrifft gebrauchsfertige Mundwasserkonzentrate, die aufgrund von speziellen Inhaltsstoffen ein besonderes Gefühl im Mund bewirken.

In der Mund- und Zahnpflege werden neben Zahnpasten vielfach flüssige Zubereitungen eingesetzt, bei welchen der Reinigungs- und Pflegeeffekt durch den Kontakt der Zubereitung mit der Mundhöhle erzielt wird, ohne daß es eines zusätzlichen Hilfsmittels wie einer Zahnbürste bedarf. Diese Zubereitungen, die üblicherweise als Mundwässer bezeichnet werden, bieten dadurch einen besonderen Anwendungskomfort und gewährleisten darüber hinaus, daß auch schwer zugängliche Bereiche der Mundhöhle, insbesondere an den Zähnen, mit der Zubereitung in Kontakt kommen.
Während Mundwässer in der Vergangenheit lediglich zur Erfrischung des Mund- und Rachenraumes und zur Verbesserung des Mundgeruchs dienten, übernehmen sie heute Aufgaben wie z. B. eine plaqueanlösende Wirkung, und sie dienen als Träger von Antikarieswirkstoffen oder antibakteriellen Wirkstoffen zur Plaquebekämpfung. Mundwässer werden als dünnflüssige wäßrige oder wäßrig-alkoholische Formulierungen angeboten bzw. als wasserverdünnbare Konzentrate. Durch die Applikation in Form einer dünnflüssigen Zubereitung wird eine gute Verteilbarkeit und ein guter Kontakt mit der Mundhöhle erreicht. Neben der Reinigung der Zähne erwartet der Verbraucher von den gattungsgemäßen Produkten auch eine Pflege der Zähne und der Mundhöhle. So sind insbesondere ein "sauberes" Gefühl, d.h. eine glatte und glänzende Zahnoberfläche sowie ein frisches Gefühl im Mund wesentliche Aspekte für den Kaufanreiz von Zubereitungen zur Mund- und Zahnpflege- und -reinigung. Ein erfolgreiches Mittel der gattungsgemäßen Art sollte daher die Zähne gründlich reinigen, ohne den Zahn oder die Zahnoberfläche zu schädigen und gleichzeitig Mundgeruch verringern und/oder verhindern.

Mundwässer sind im Stand der Technik breit beschrieben. Die DE 33 41 466 A1 offenbart wäßrige Mundpflegemittel, welche neben 1 bis 3 Gew.-% Wasserstoffperoxid 3 bis 15 Gew.-% Glycerin und/oder Sorbit sowie 3 bis 10 Gew.-% Ethanol enthalten.

Die europäische Patentanmeldung EP 0 497 476 A2 offenbart Antiplaque-Zusammensetzungen, welche in Form von Mundspülungen 1 bis 20 Gew.-% Glycerin, 1 bis 20 Gew.-% Propylenglycol, 1 bis 25 Gew.-% Sorbitol und 5 bis 20 Gew.-% Ethanol enthalten können.

Schließlich beschreibt das U.S. Patent 4,130,638 Mundwasserzusammensetzungen, welche 10-25% Ethanol, 0.5- 2.5% Pluronic^{®} Tensid sowie Thymol, Menthol und Eucalyptol enthalten.

Mundwässer werden oft in der Form von Konzentraten angeboten, die vom Verbraucher direkt vor der Anwendung mit Wasser verdünnt werden. Da dies den Zugang zu Wasser und einem Mischbehälter voraussetzt, stoßen gebrauchsfertige Mundwässer, die beispielsweise direkt aus der Verpackung in den Mund aufgenommen und nach der Benutzung ausgespuckt werden können, auf gesteigertes Verbraucherinteresse. Diese gebrauchsfertigen Lösungen besitzen aber den Nachteil, daß die Aktivkomponenten in relativ geringer Konzentration vorliegen, so daß das Verkaufsprodukt nur eine geringe Anzahl von Anwendungsvorgängen ermöglicht. Zusätzlich werden die anwendungsfertigen Mundwässer vom Verbraucher subjektiv oft als zu wenig leistungsstark empfunden, auch wenn dies objektiv nicht zutrifft.

Es ist bekannt, Zubereitungen zur Mund- und Zahnpflege- und -reinigung Geruchs- und/oder Geschmacksstoffe bzw. Aromen zuzusetzen, um ihnen einerseits einen besonderen Geruch bzw. Geschmack zu verleihen und andererseits um ein aromatisches Gefühl von Frische nach der Anwendung zu hinterlassen. Solche Aromen usw. sind im Bereich der Mund- und Zahnpflege und -reinigung weit verbreitet. In speziellen Zubereitungen ist auch der Einsatz von sogenannten Scharfstoffen bereits bekannt, um einen sensorisch interessanten und lang anhaltenden Geschmack und/oder Frischeeffekt zu bewirken. So offenbart die GB 1,438, 205 Zahnpasten oder Mundwässer, welche N-isobutyl-2,6,8-Decatrienamid enthalten, das in Mengen um 0,01 Gew.-%, bezogen auf die Zubereitung, eingesetzt wird.

Der Einsatz anderer Scharfstoffe aus der Gruppe der Alkadienamide wird beispielsweise in der DE 103 51 422 A1 offenbart.

Es hat sich gezeigt, daß der gewünschte scharfe, kribbelnde, mundwässernde oder wärmeerzeugende Effekt von einem Großteil der Verbraucher als unangenehm und zu stark empfunden wird, das aber die Reduzierung in der Einsatzkonzentration des Scharfstoffes zu verringerter Wahrnehmung des Effektes führt. Es bestand daher nach wie vor der Wunsch nach Zubereitungsformen, die ein sensorisch einzigartiges Empfinden hervorrufen, das vom Verbraucher akzeptiert und nicht als unangenehm empfunden wird.

Überraschenderweise wurde nun festgestellt, daß Zubereitungen, die Ethanol in bestimmtem Mengen und scharf schmeckende und/oder ein Gefühl von Wärme erzeugende Substanzen enthalten, dem Anwender ein angenehmes Gefühl von Wärme und Schärfe vermitteln, ohne daß dieses Gefühl als unangenehm empfunden wird. Zudem werden diese Produkte vom Verbraucher als leistungsstark empfunden.

Gegenstand der vorliegenden Erfindung sind demnach gebrauchsfertige Mundwasserzusammensetzungen, enthaltend bezogen auf ihr Gewicht
a) 10 bis 40 Gew.-% Ethanol;
b) mindestens eine scharf schmeckende und/oder ein Gefühl von Wärme erzeugende Substanz.

"Mundwasser" bzw. Mundwasserzusammensetzung" im Sinne der vorliegenden Erfindung sind auch sogenannte Mundspülungen, Anti-Plaque-Spülungen usw. "Gebrauchsfertig" im Rahmen der vorliegenden Erfindung bedeutet, daß die Zusammensetzung ohne weitere Verarbeitungsschritte (z.B. Verdünnung) aus der Verpackung entnommen und vom Verbraucher angewendet werden kann und soll.

Die erfindungsgemäße Mundwasserzusammensetzung enthält bezogen auf ihr Gewicht 10 bis 40 Gew.-% Ethanol, wobei sich die Angabe auf reinen Alkohol (100 %) bezieht, auch wenn dieser nicht als 100%iges Ethanol eingesetzt wurde, da Ethanol mit Wasser ein Azeotrop bildet, das aus 95,57 Gew.-% Ethanol und 4,43 Gew.-% Wasser besteht und konstant bei 78,2°C siedet.

Mit besonderem Vorzug enthalten die erfindungsgemäßen Mundwasserzusammensetzungen höhere Mengen an Ethanol, wobei erfindungsgemäße Mundwasserzusammensetzungen besonders bevorzugt sind, die bezogen auf ihr Gewicht 12,5 bis 35 Gew.-%, vorzugsweise 15 bis 30 Gew.-% und insbesondere 17,5 bis 25 Gew.-% Ethanol enthalten.

Als weiteren wesentlichen Inhaltsstoff enthalten die erfindungsgemäßen Mundwässer eine scharf schmeckende und/oder ein Gefühl von Wärme erzeugende Substanz. Diese Substanzen vermitteln dem Anwender einen scharfen, kribbelnden, mundwässernden oder wärmeerzeugenden Effekt, d.h. sie rufen sensorisch einen Wärmeeindruck oder ein Brennen, oder ein Prickeln, Perlen, Kitzeln oder Sprudeln hervor.

Erfindungsgemäß bevorzugte Mundwasserzusammensetzungen enthalten die scharf schmeckende(n) und/oder ein Gefühl von Wärme erzeugende(n) Substanz(en) in Mengen von 0,00001 bis 5 Gew.-%, vorzugsweise von 0,0005 bis 2,5 Gew.-%, weiter bevorzugt von 0,001 bis 1 Gew.-%, besonders bevorzugt von 0,005 bis 0,75 Gew.-% und insbesondere von 0,01 bis 0,5 Gew.-%, jeweils bezogen auf das Gewichts des gesamten Mittels.

Als scharf schmeckende oder ein Gefühl von Wärme erzeugende Substanz kann eine Reihe von Stoffen eingesetzt werden. Bevorzugt sind insbesondere N-Alkyl-substituierte Amide von ungesättigten Carbonsäuren, beispielsweise
- 2E,4E-Decadiensäure-N-Methylamid
- 2E,4E-Decadiensäure-N-Ethylamid
- 2E,4E-Decadiensäure-N-n-Propylamid
- 2E,4E-Decadiensäure-N-Isopropylamid
- 2E,4E-Decadiensäure-N-n-Butylamid
- 2E,4E-Decadiensäure-N-(1-Methylpropyl)-amid
- 2E,4E-Decadiensäure-N-Isobutylamid
- 2E,4E-Decadiensäure-N-tert-Butylamid

- 2E,4Z-Decadiensäure-N-Methylamid
- 2E,4Z-Decadiensäure-N-Ethylamid
- 2E,4Z-Decadiensäure-N-n-Propylamid
- 2E,4Z-Decadiensäure-N-Isopropylamid
- 2E,4Z-Decadiensäure-N-n-Butylamid
- 2E,4Z-Decadiensäure-N-(1-Methylpropyl)-amid
- 2E,4Z-Decadiensäure-N-Isobutylamid
- 2E,4Z-Decadiensäure-N-tert-Butylamid

- 2E,4E,8Z-Decatriensäure-N-Methylamid
- 2E,4E,8Z-Decatriensäure-N-Ethylamid
- 2E,4E,8Z-Decatriensäure-N-n-Propylamid
- 2E,4E,8Z-Decatriensäure-N-Isopropylamid
- 2E,4E,8Z-Decatriensäure-N-n-Butylamid
- 2E,4E,8Z-Decatriensäure-N-(1-Methylpropyl)-amid
- 2E,4E,8Z-Decatriensäure-N-Isobutylamid
- 2E,4E,8Z-Decatriensäure-N-tert-Butylamid

- 2E,4Z,8Z-Decatriensäure-N-Methylamid
- 2E,4Z,8Z-Decatriensäure-N-Ethylamid
- 2E,4Z,8Z-Decatriensäure-N-n-Propylamid
- 2E,4Z,8Z-Decatriensäure-N-Isopropylamid
- 2E,4Z,8Z-Decatriensäure-N-n-Butylamid
- 2E,4Z,8Z-Decatriensäure-N-(1-Methylpropyl)-amid
- 2E,4Z,8Z-Decatriensäure-N-Isobutylamid
- 2E,4Z,8Z-Decatriensäure-N-tert-Butylamid
- 2E,4E,8E-Decatriensäure-N-Methylamid
- 2E,4E,8E-Decatriensäure-N-Ethylamid
- 2E,4E,8E-Decatriensäure-N-n-Propylamid
- 2E,4E,8E-Decatriensäure-N-Isopropylamid
- 2E,4E,8E-Decatriensäure-N-n-Butylamid
- 2E,4E,8E-Decatriensäure-N-(1-Methylpropyl)-amid
- 2E,4E,8E-Decatriensäure-N-Isobutylamid
- 2E,4E,8E-Decatriensäure-N-tert-Butylamid

- 2E,4Z,8E-Decatriensäure-N-Methylamid
- 2E,4Z,8E-Decatriensäure-N-Ethylamid
- 2E,4Z,8E-Decatriensäure-N-n-Propylamid
- 2E,4Z,8E-Decatriensäure-N-Isopropylamid
- 2E,4Z,8E-Decatriensäure-N-n-Butylamid
- 2E,4Z,8E-Decatriensäure-N-(1-Methylpropyl)-amid
- 2E,4Z,8E-Decatriensäure-N-Isobutylamid
- 2E,4Z,8E-Decatriensäure-N-tert-Butylamid

- 2E,6Z,8E-Decatriensäure-N-Methylamid
- 2E,6Z,8E-Decatriensäure-N-Ethylamid
- 2E,6Z,8E-Decatriensäure-N-n-Propylamid
- 2E,6Z,8E-Decatriensäure-N-Isopropylamid
- 2E,6Z,8E-Decatriensäure-N-n-Butylamid
- 2E,6Z,8E-Decatriensäure-N-(1-Methylpropyl)-amid
- 2E,6Z,8E-Decatriensäure-N-Isobutylamid
- 2E,6Z,8E-Decatriensäure-N-tert-Butylamid

- 2E,6E,8E-Decatriensäure-N-Methylamid
- 2E,6E,8E-Decatriensäure-N-Ethylamid
- 2E,6E,8E-Decatriensäure-N-n-Propylamid
- 2E,6E,8E-Decatriensäure-N-Isopropylamid
- 2E,6E,8E-Decatriensäure-N-n-Butylamid
- 2E,6E,8E-Decatriensäure-N-(1-Methylpropyl)-amid
- 2E,6E,8E-Decatriensäure-N-Isobutylamid
- 2E,6E,8E-Decatriensäure-N-tert-Butylamid

- 2E,6Z,8Z-Decatriensäure-N-Methylamid
- 2E,6Z,8Z-Decatriensäure-N-Ethylamid
- 2E,6Z,8Z-Decatriensäure-N-n-Propylamid
- 2E,6Z,8Z-Decatriensäure-N-Isopropylamid
- 2E,6Z,8Z-Decatriensäure-N-n-Butylamid
- 2E,6Z,8Z-Decatriensäure-N-(1-Methylpropyl)-amid
- 2E,6Z,8Z-Decatriensäure-N-Isobutylamid
- 2E,6Z,8Z-Decatriensäure-N-tert-Butylamid

- 2E,6E,8Z-Decatriensäure-N-Methylamid
- 2E,6E,8Z-Decatriensäure-N-Ethylamid
- 2E,6E,8Z-Decatriensäure-N-n-Propylamid
- 2E,6E,8Z-Decatriensäure-N-Isopropylamid
- 2E,6E,8Z-Decatriensäure-N-n-Butylamid
- 2E,6E,8Z-Decatriensäure-N-(1-Methylpropyl)-amid
- 2E,6E,8Z-Decatriensäure-N-Isobutylamid
- 2E,6E,8Z-Decatriensäure-N-tert-Butylamid

- 2E,7Z,9E-Undecatriensäure -N-Methylamid
- 2E,7Z,9E-Undecatriensäure -N-Ethylamid
- 2E,7Z,9E-Undecatriensäure -N-n-Propylamid
- 2E,7Z,9E-Undecatriensäure -N-Isopropylamid
- 2E,7Z,9E-Undecatriensäure -N-n-Butylamid
- 2E,7Z,9E-Undecatriensäure -N-(1-Methylpropyl)-amid
- 2E,7Z,9E-Undecatriensäure-N-isobutylamid
- 2E,7Z,9E-Undecatriensäure -N-tert-Butylamid

- 2E,7E,9E-Undecatriensäure -N-Methylamid
- 2E,7E,9E-Undecatriensäure -N-Ethylamid
- 2E,7E,9E-Undecatriensäure -N-n-Propylamid
- 2E,7E,9E-Undecatriensäure -N-Isopropylamid
- 2E,7E,9E-Undecatriensäure -N-n-Butylamid
- 2E,7E,9E-Undecatriensäure -N-(1-Methylpropyl)-amid
- 2E,7E,9E-Undecatriensäure-N-isobutylamid
- 2E,7E,9E-Undecatriensäure -N-tert-Butylamid

- 2E,7Z,9Z-Undecatriensäure -N-Methylamid
- 2E,7Z,9Z-Undecatriensäure -N-Ethylamid
- 2E,7Z,9Z-Undecatriensäure -N-n-Propylamid
- 2E,7Z,9Z-Undecatriensäure -N-Isopropylamid
- 2E,7Z,9Z-Undecatriensäure -N-n-Butylamid
- 2E,7Z,9Z-Undecatriensäure -N-(1-Methylpropyl)-amid
- 2E,7Z,9Z-Undecatriensäure-N-isobutylamid
- 2E,7Z,9Z-Undecatriensäure -N-tert-Butylamid

- 2E,7Z,9E-Undecatriensäure -N-Methylamid
- 2E,7Z,9E-Undecatriensäure -N-Ethylamid
- 2E,7Z,9E-Undecatriensäure -N-n-Propylamid
- 2E,7Z,9E-Undecatriensäure -N-Isopropylamid
- 2E,7Z,9E-Undecatriensäure -N-n-Butylamid
- 2E,7Z,9E-Undecatriensäure -N-(1-Methylpropyl)-amid
- 2E,7Z,9E-Undecatriensäure-N-isobutylamid
- 2E,7Z,9E-Undecatriensäure -N-tert-Butylamid

Selbstverständlich sind auch andere Substitutionsmuster am Stickstoffatom möglich und bevorzugt, beispielsweise längerkettige n-Alkylreste (...-N-n-Pentylamid, ...-N-n-Pentylamid, ...-N-n-Pentylamid, ...-N-n-Pentylamid, ...-N-n-Pentylamid, ...-N-n-Hexylamid, ...-N-n-Heptylamid, ...-N-n-Octylamid, ...-N-n-Nonylamid, ...-N-n-Decylamid, ...-N-n-Undecylamid, ...-N-n-Dodecylamid, ...-N-n-Tridecylamid, usw.) oder disubstituierte ...-N,N-Dialkylamide wie ...-N,N-dimethylamid, ...-N,N-diethylamid, ...-N,N-di-n-propylamid, ...-N,N-düsopropylamid, ...-N,N-di-n-butylamid, ...-N,N-di(1-Methylpropyl)amid, ...-N,N-düsobutylamid, ...-N,N-di-tert-butylamid, ...-N,N-methyl-ethylamid, ...-N,N-methyl-n-propylamid, ...-N,N-methyl-isopropylamid, ...-N,N-ethyl-n-propylamid, ...-N,N-ethyl-isopropylamid, usw..

Unter den genannten Verbindungen sind einige im Rahmen der vorliegenden Erfindung besonders bevorzugt. Diese sind nachfolgend aufgeführt:

2E,6Z,8E-Decatriensäure-N-isobutylamid (N-Isobutyl-2E,6Z,8E-decatrienamid, auch Spilanthol oder Affinin genannt):

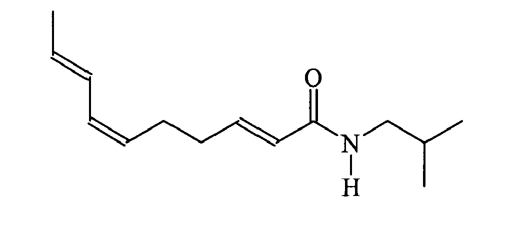

2E,4E,8Z-Decatriensäure-N-isobutylamid (N-Isobutyl-2E,4E,8Z-decatrienamid, auch Isoaffinin genannt):

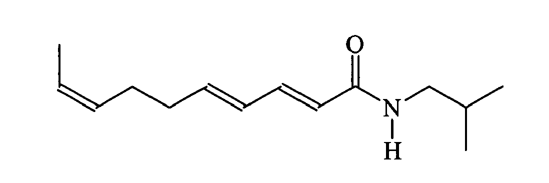

2E,7Z,9E-Undecatriensäure-N-isobutylamid (N-Isobutyl-2E,7Z,9E-undecatrienamid):

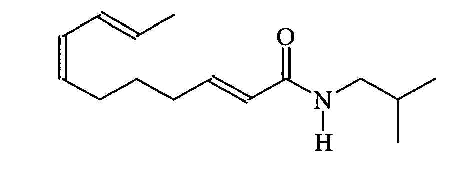

2E,4Z-Decadiensäure-N-isobutylamid (cis-Pellitorin):

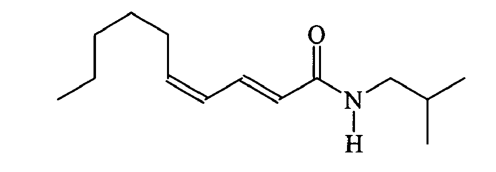

2E,4E-Decadiensäure-N-isobutylamid (trans-Pellitorin):

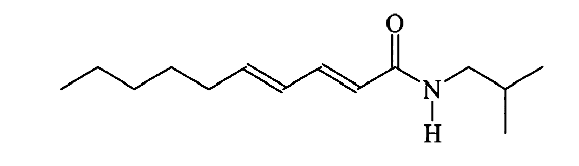

Ferulasäureamide, beispielsweise

Ferulasäure-N-Vanillylamid:

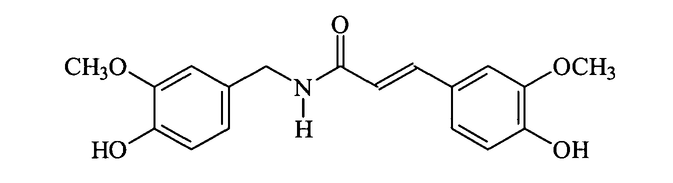

N-[2-(4-Hydroxy-3-methoxyphenyl)ethyl]-3-(4-hydroxy-3-methoxy-phenyl)- (2*E*)-propensäureamid (trans-Feruloylmethoxytyramin):

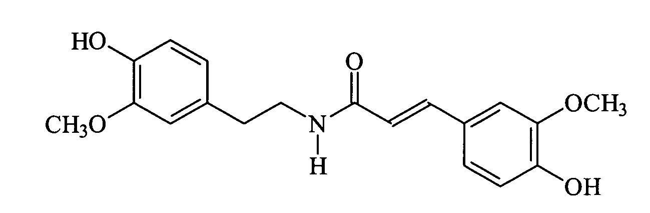

*N*-[2- (4-Hydroxy-3-methoxyphenyl)ethyl]-3-(4-hydroxy-3-methoxy-phenyl)-(2 Z)-propensäureamid (cis-Feruloylmethoxytyramin):

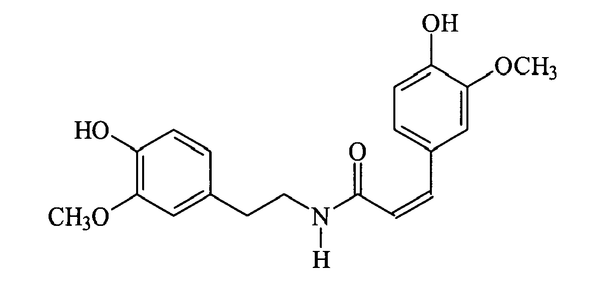

*N*-[2-(4- Hydroxy-3-methoxyphenyl)ethyl]-3-(4-hydroxy-3-methoxy-phenyl)- propansäureamid (Dihydroferuloylmethoxytyramin):

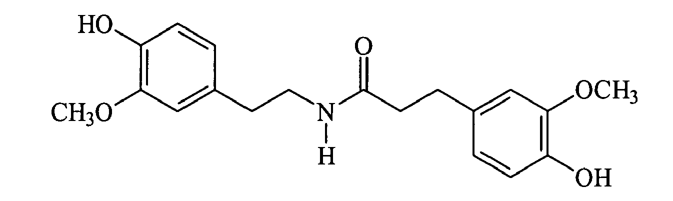

*N*-[2-(3,4- Dihydroxyphenyl)ethyl]-3-(4-hydroxy-3-methoxy-phenyl)-(2E)- propensäureamid (trans-Feruloyldopamin):

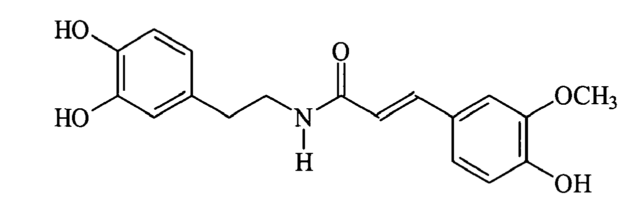

*N*-[2-(3,4-Dihydroxyphenyl) ethyl]-3-(4-hydroxy-3-methoxy-phenyl)-(2Z)-propensäureamid (cis-Feruloyldopamin):

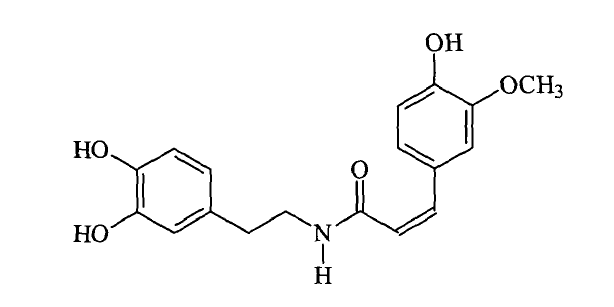

*N*-[2-(4-Hydroxyphenyl)ethyl]-3-(3,4- dihydroxyphenyl)-(2*E*)-propensäureamid (trans-Caffeoyltyramin):

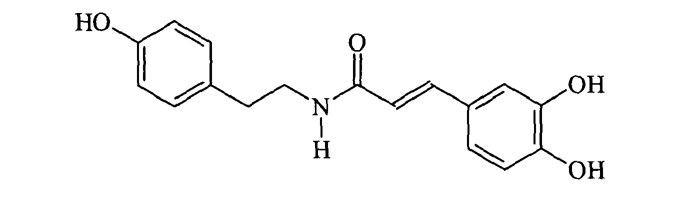

*N*-[2-(4-Hydroxyphenyl)ethyl]-3-(3,4-dihydroxyphenyl)-(2Z)- propensäureamid (cis-Caffeoyltyramin):

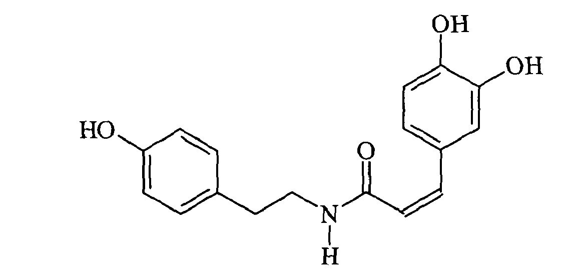

*N*-[2-(3,4-Dimethoxyphenyl) ethyl]-3-(3,4-dimethoxyphenyl)-(2*E*)-propensäureamid (trans-Rubenamin):

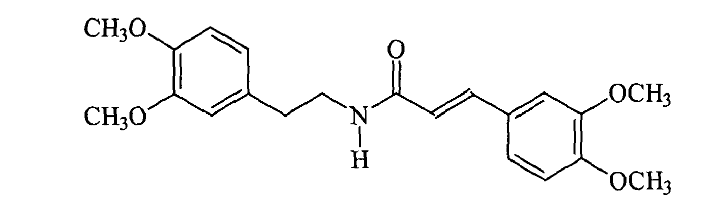

*N*-[2-(3,4-dimethoxyphenyl)ethyl]-3-(3,4- dimethoxy-phenyl)-(2Z)-propensäureamid (cis-Rubenamin):

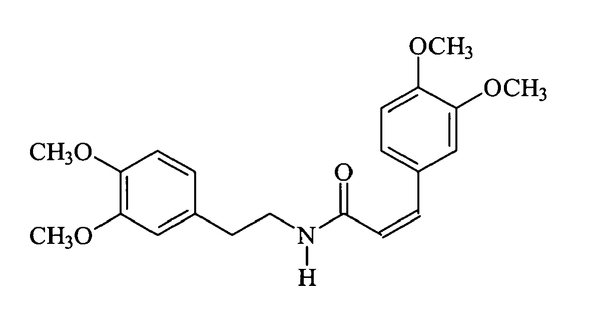

Weitere im Rahmen der vorliegenden Erfindung mit besonderem Vorzug einsetzbare Scharfstoffe sind beispielsweise Extrakte aus Naturpflanzen. Scharf schmeckende pflanzliche Extrakte können alle physiologisch unbedenklichen pflanzlichen Extrakte sein, die einen scharfen oder warmen sensorischen Eindruck hervorrufen. Bevorzugt als scharf schmeckende pflanzliche Extrakte sind beispielsweise Pfefferextrakt *(Piper ssp.,* insbesondere *Piper nigrum),* Wasserpfefferextrakt *(Polygonum ssp.* ,insbesondere *Polygonum hydropiper),* Extrakte aus *Allium* ssp.(insbesondere Zwiebel und Knoblauchextrakte), Extrakte aus Rettich (*Raphanus ssp.),* Meerrettichextrakte (*Cochlearia armoracia*), Extrakte aus schwarzem *(Brassica nigra),* wildem oder gelbem Senf *(Sinapis ssp.,* insbesondere *Sinapis arvensis* und *Sinapis alba*), Bertramwurzel-Extrakte (*Anacyclus ssp.,* insbesondere *Anacyclus pyrethrumL.),* Sonnenhutextrakte (*Echinaceae ssp.),* Extrakten aus Szechuan-Pfeffer *(Zanthoxylum ssp.,* insbesondere *Zanthoxylum piperitum),* Spilanthesextrakt (*Spilanthes ssp.,* insbesondere *Spilanthes acmella*), Chiliextrakt *(Capsicum ssp.,* insbesondere *Capsicum frutescens* ), Paradieskörner-Extrakt (*Aframomum ssp.,*insbesondere *Aframomum melegueta[Rose]* K. Schum.),Ingwerextrakt *(Zingiber ssp.,*insbesondere *Zingiber officinale*) und Galangaextrakt *(Kaempferia galanga* oder *Alpinia galanga*).

Eine besonders geeignete Substanz ist das aus dem Ingwerextrakt stammende Gingerol:

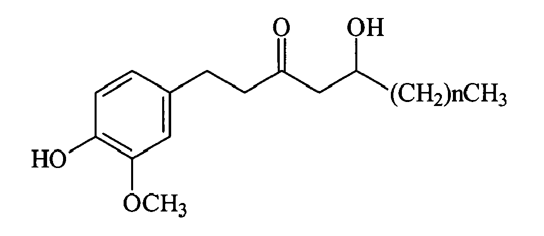

Einsetzbar ist auch N-Ethyl-p-menthan-3-carboxamid (N-Ethyl-5-Methyl-2-isopropylcyclohexancarboxamid):

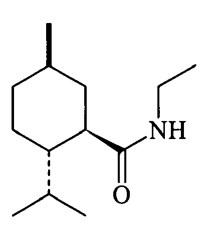

Andere scharf schmeckende oder ein Gefühl von Wärme erzeugende Substanzen können z.B. sein Capsaicin, Dihydrocapsaicin, Gingerol, Paradol, Shogaol, Piperin, Carbonsäure-N-vanillylamide, insbesondere Nonansäure-N- vanillylamid, 2-Alkensäureamide, insbesondere 2-Nonensäure-N-isobutylamid, 2- Nonensäure-N-4-hydroxy-3- methoxyphenylamid, Alkylether von 4-Hydroxy-3- methoxybenzylalkohol, insbesonders 4-Hydroxy-3-methoxybenzyl-n-butylether, Alkylether von 3- Hydroxy-4-methoxybenzylalkohol, Alkylether von 3,4- Dimethoxybenzylalkohol, Alkylether von 3-Ethoxy-4-hydroxybenzylalkohol, Alkylether von 3,4-Methylendioxybenzylalkohol, Nicotinaldehyd, Methylnicotinat, Propylnicotinat, 2-Butoxyethylnicotinat, Benzylnicotinat, 1-Acetoxychavicol, Polygodial oder Isodrimeninol.

Besonders bevorzugte erfindungsgemäße Mundwasserzusammensetzungen sind dadurch gekennzeichnet, daß sie mindestens einen Scharfstoff aus der Gruppe der N-Alkyl-substituierte Amide von ungesättigten Carbonsäuren, vorzugsweise
- 2E,6Z,8E-Decatriensäure-N-isobutylamid (Spilanthol) und/oder
- 2E,4E,8Z-Decatriensäure-N-isobutylamid und/oder
- 2E,7Z,9E-Undecatriensäure-N-isobutylamid und/oder
- 2E,4Z-Decadiensäure-N-isobutylamid (cis-Pellitorin) und/oder
- 2E,4E-Decadiensäure-N-isobutylamid (trans-Pellitorin) und/oder
- Ferulasäure-N-Vanillylamid und/oder
- N-[2-(4-Hydroxy-3-methoxyphenyl)ethyl]-3-(4-hydroxy-3-methoxyphenyl)- (2*E*)-propensäureamid (trans-Feruloylmethoxytyramin) und/oder
- *N*-[2- (4-Hydroxy-3-methoxyphenyl)ethyl]-3-(4-hydroxy-3-methoxyphenyl)-(2 Z)-propensäureamid (cis-Feruloylmethoxytyramin) und/oder
- *N*-[2-(4- Hydroxy-3-methoxyphenyl)ethyl]-3-(4-hydroxy-3-methoxyphenyl)- propansäureamid (Dihydroferuloylmethoxytyramin) und/oder
- *N*-[2-(3,4- Dihydroxyphenyl)ethyl]-3-(4-hydroxy-3-methoxy-phenyl)-(2*E*)-propensäureamid (trans-Feruloyldopamin) und/oder
- *N*-[2-(3,4-Dihydroxyphenyl) ethyl]-3-(4-hydroxy-3-methoxy-phenyl)-(2*Z*)-propensäureamid (cis- Feruloyldopamin) und/oder
- *N*-[2-(4-Hydroxyphenyl)ethyl]-3-(3,4- dihydroxyphenyl)-(2*E*)-propensäureamid (trans-Caffeoyltyramin) und/oder
- *N*-[2-(4-Hydroxyphenyl)ethyl]-3-(3,4-dihydroxyphenyl)-(2Z)-propensäureamid (cis-Caffeoyltyramin) und/oder
- *N*-[2-(3,4-Dimethoxyphenyl) ethyl]-3-(3,4-dimethoxyphenyl)-(2*E*)-propensäureamid (trans- Rubenamin) und/oder
- *N*-[2-(3,4-dimethoxyphenyl)ethyl]-3-(3,4- dimethoxy-phenyl)-(2*Z*)-propensäureamid (cis-Rubenamin)
enthalten.

Zusätzlich zu den genannten Scharfstoffen oder an ihrer Stelle können auch weitere scharf schmeckende und/oder ein Gefühl von Wärme erzeugende Substanzen in die erfindungsgemäßen Mundwässer eingearbeitet werden.

Als besonders geeignet haben sich im Rahmen der vorliegenden Erfindung alkylsubstituierte Dioxane der Formel 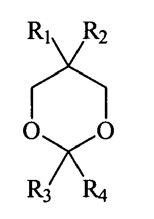 erwiesen, in der R1 und R2 unabhängig voneinander ausgewählt sind aus -H, -CH₃, -CH₂CH₃ und R3 und R4 unabhängig voneinander ausgewählt sind aus -H, -CH₃, -CH₂CH₃, -CH₂CH₂CH₃, - CH(CH₃)₂. Einzelne Spezies, die der vorstehend genannten Formel genügen, sind hinsichtlich ihrer Reste R1, R2, R3 und R4 in der nachstehenden Tabelle 1 aufgeführt. Diese sind erfindungsgemäß besonders bevorzugt.

**Tabelle 1: Bevorzugte alkylsubstituierte Dioxane:**

| **Nr.** | **R1** | **R2** | **R3** | **R4** |
|---|---|---|---|---|
| 1 | -H | -CH₃ | -H | -H |
| 2 | -H | -CH₂CH₃ | -H | -H |
| 3 | -CH₃ | -CH₃ | -H | -H |
| 4 | -CH₃ | -CH₂CH₃ | -H | -H |
| 5 | -CH₂CH₃ | -CH₂CH₃ | -H | -H |
| 6 | -H | -CH₃ | -H | -CH₃ |
| 7 | -H | -CH₃ | -H | -CH₂CH₃ |
| 8 | -H | -CH₃ | -H | -CH₂CH₂CH₃ |
| 9 | -H | -CH₃ | -H | -CH(CH₃)₂ |
| 10 | -H | -CH₃ | -CH₃ | -CH₃ |
| 11 | -H | -CH₃ | -CH₃ | -CH₂CH₃ |
| 12 | -H | -CH₃ | -CH₃ | -CH₂CH₂CH₃ |
| 13 | -H | -CH₃ | -CH₃ | -CH(CH₃)₂ |
| 14 | -H | -CH₃ | -CH₂CH₃ | -CH₂CH₃ |
| 15 | -H | -CH₃ | -CH₂CH₃ | -CH₂CH₂CH₃ |
| 16 | -H | -CH₃ | -CH₂CH₃ | -CH(CH₃)₂ |
| 17 | -H | -CH₃ | -CH₂CH₂CH₃ | -CH₂CH₂CH₃ |
| 18 | -H | -CH₃ | -CH₂CH₂CH₃ | -CH(CH₃)₂ |
| 19 | -H | -CH₃ | -CH(CH₃)₂ | -CH(CH₃)₂ |
| 20 | -H | -CH₂CH₃ | -H | -CH₃ |
| 21 | -H | -CH₂CH₃ | -H | -CH₂CH₃ |
| 22 | -H | -CH₂CH₃ | -H | -CH₂CH₂CH₃ |
| 23 | -H | -CH₂CH₃ | -H | -CH(CH₃)₂ |
| 24 | -H | -CH₂CH₃ | -CH₃ | -CH₃ |
| 25 | -H | -CH₂CH₃ | -CH₃ | -CH₂CH₃ |
| 26 | -H | -CH₂CH₃ | -CH₃ | -CH₂CH₂CH₃ |
| 27 | -H | -CH₂CH₃ | -CH₃ | -CH(CH₃)₂ |
| 28 | -H | -CH₂CH₃ | -CH₂CH₃ | -CH₂CH₃ |
| 29 | -H | -CH₂CH₃ | -CH₂CH₃ | -CH₂CH₂CH₃ |
| 30 | -H | -CH₂CH₃ | -CH₂CH₃ | -CH(CH₃)₂ |
| 31 | -H | -CH₂CH₃ | -CH₂CH₂CH₃ | -CH₂CH₂CH₃ |
| 32 | -H | -CH₂CH₃ | -CH₂CH₂CH₃ | -CH(CH₃)₂ |
| 33 | -H | -CH₂CH₃ | -CH(CH₃)₂ | -CH(CH₃)₂ |
| 34 | -CH₃ | -CH₃ | -H | -CH₃ |
| 35 | -CH₃ | -CH₃ | -H | -CH₂CH₃ |
| 36 | -CH₃ | -CH₃ | -H | -CH₂CH₂CH₃ |
| 37 | -CH₃ | -CH₃ | -H | -CH(CH₃)₂ |
| 38 | -CH₃ | -CH₃ | -CH₃ | -CH₃ |
| 39 | -CH₃ | -CH₃ | -CH₃ | -CH₂CH₃ |
| 40 | -CH₃ | -CH₃ | -CH₃ | -CH₂CH₂CH₃ |
| 41 | -CH₃ | -CH₃ | -CH₃ | -CH(CH₃)₂ |
| 42 | -CH₃ | -CH₃ | -CH₂CH₃ | -CH₂CH₃ |
| 43 | -CH₃ | -CH₃ | -CH₂CH₃ | -CH₂CH₂CH₃ |
| 44 | -CH₃ | -CH₃ | -CH₂CH₃ | -CH(CH₃)₂ |
| 45 | -CH₃ | -CH₃ | -CH₂CH₂CH₃ | -CH₂CH₂CH₃ |
| 46 | -CH₃ | -CH₃ | -CH₂CH₂CH₃ | -CH(CH₃)₂ |
| 47 | -CH₃ | -CH₃ | -CH(CH₃)₂ | -CH(CH₃)₂ |
| 48 | -CH₃ | -CH₂CH₃ | -H | -CH₃ |
| 49 | -CH₃ | -CH₂CH₃ | -H | -CH₂CH₃ |
| 50 | -CH₃ | -CH₂CH₃ | -H | -CH₂CH₂CH₃ |
| 51 | -CH₃ | -CH₂CH₃ | -H | -CH(CH₃)₂ |
| 52 | -CH₃ | -CH₂CH₃ | -CH₃ | -CH₃ |
| 53 | -CH₃ | -CH₂CH₃ | -CH₃ | -CH₂CH₃ |
| 54 | -CH₃ | -CH₂CH₃ | -CH₃ | -CH₂CH₂CH₃ |
| 55 | -CH₃ | -CH₂CH₃ | -CH₃ | -CH(CH₃)₂ |
| 56 | -CH₃ | -CH₂CH₃ | -CH₂CH₃ | -CH₂CH₃ |
| 57 | -CH₃ | -CH₂CH₃ | -CH₂CH₃ | -CH₂CH₂CH₃ |
| 58 | -CH₃ | -CH₂CH₃ | -CH₂CH₃ | -CH(CH₃)₂ |
| 59 | -CH₃ | -CH₂CH₃ | -CH₂CH₂CH₃ | -CH₂CH₂CH₃ |
| 60 | -CH₃ | -CH₂CH₃ | -CH₂CH₂CH₃ | -CH(CH₃)₂ |
| 61 | -CH₃ | -CH₂CH₃ | -CH(CH₃)₂ | -CH(CH₃)₂ |
| 62 | -CH₂CH₃ | -CH₂CH₃ | -H | -CH₃ |
| 63 | -CH₂CH₃ | -CH₂CH₃ | -H | -CH₂CH₃ |
| 64 | -CH₂CH₃ | -CH₂CH₃ | -H | -CH₂CH₂CH₃ |
| 65 | -CH₂CH₃ | -CH₂CH₃ | -H | -CH(CH₃)₂ |
| 66 | -CH₂CH₃ | -CH₂CH₃ | -CH₃ | -CH₃ |
| 67 | -CH₂CH₃ | -CH₂CH₃ | -CH₃ | -CH₂CH₃ |
| 68 | -CH₂CH₃ | -CH₂CH₃ | -CH₃ | -CH₂CH₂CH₃ |
| 69 | -CH₂CH₃ | -CH₂CH₃ | -CH₃ | -CH(CH₃)₂ |
| 70 | -CH₂CH₃ | -CH₂CH₃ | -CH₂CH₃ | -CH₂CH₃ |
| 71 | -CH₂CH₃ | -CH₂CH₃ | -CH₂CH₃ | -CH₂CH₂CH₃ |
| 72 | -CH₂CH₃ | -CH₂CH₃ | -CH₂CH₃ | -CH(CH₃)₂ |
| 73 | -CH₂CH₃ | -CH₂CH₃ | -CH₂CH₂CH₃ | -CH₂CH₂CH₃ |
| 74 | -CH₂CH₃ | -CH₂CH₃ | -CH₂CH₂CH₃ | -CH(CH₃)₂ |
| 75 | -CH₂CH₃ | -CH₂CH₃ | -CH(CH₃)₂ | -CH(CH₃)₂ |

Als weiterhin besonders geeignet haben sich im Rahmen der vorliegenden Erfindung Phenylester der Formel 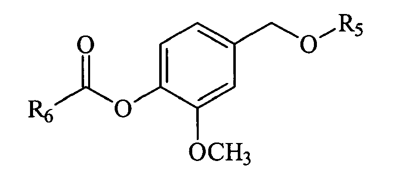 erwiesen, in der R5 für -CH₃ oder einen geradkettigen oder verzweigten Alkyl- oder Alkenylrest mit 2 bis 8 Kohlenstoffatomen und R6 für -CH₃ oder einen geradkettigen oder verzweigten Alkyl- oder Alkenylrest mit 2 bis 8 Kohlenstoffatomen oder eine Alkoxygruppe mit 1 bis 3 Kohlenstoffatomen steht

Einzelne Spezies, die der vorstehend genannten Formel genügen, sind hinsichtlich ihrer Reste R5 und R6 in der nachstehenden Tabelle 2 aufgeführt. Diese sind erfindungsgemäß besonders bevorzugt.

**Tabelle 2: Bevorzugte Phenylester**

| **Nr.** | **R5** | **R6** |
|---|---|---|
| 1 | -CH₃ | -CH₃ |
| 2 | -CH₂CH₃ | -CH₃ |
| 3 | -CH₂CH₂CH₃ | -CH₃ |
| 4 | -CH(CH₃)₂ | -CH₃ |
| 5 | -CH₂CH₂CH₂CH₃ | -CH₃ |
| 6 | -CH(CH₃)CH₂CH₃ | -CH₃ |
| 7 | -CH₂CH(CH₃)₂ | -CH₃ |
| 8 | -C(CH₃)₃ | -CH₃ |
| 9 | -CH₂(CH₂)₃CH₃ | -CH₃ |
| 10 | -CH(CH₃)(CH₂)₂CH₃ | -CH₃ |
| 11 | -CH₂CH(CH₃)CH₂CH₃ | -CH₃ |
| 12 | -(CH₂)₂CH(CH₃)₂ | -CH₃ |
| 13 | -CH₂(CH₂)₄CH₃ | -CH₃ |
| 14 | -CH₂(CH₂)₅CH₃ | -CH₃ |
| 15 | -CH₂(CH₂)₆CH₃ | -CH₃ |
| 16 | -CH=CH₂ | -CH₃ |
| 17 | -CH=CHCH₃ | -CH₃ |
| 18 | -CH=C(CH₃)₂ | -CH₃ |
| 19 | -C(CH₃)=CH₂ | -CH₃ |
| 20 | -C(CH₃)=CHCH₃ | -CH₃ |
| 21 | -C(CH₃)=C(CH₃)₂ | -CH₃ |
| 22 | -CH=CH-CH₂CH₃ | -CH₃ |
| 23 | -CH₂CH=CH-CH₃ | -CH₃ |
| 24 | -(CH₂)₂CH=CH₂ | -CH₃ |
| 25 | -CH=CH-(CH₂)₂CH₃ | -CH₃ |
| 26 | -CH₂CH=CH-CH₂CH₃ | -CH₃ |
| 27 | -(CH₂)₂CH=CH-CH₃ | -CH₃ |
| 28 | -(CH₂)₃CH=CH₂ | -CH₃ |
| 29 | -CH=CH-(CH₂)₃CH₃ | -CH₃ |
| 30 | -CH₂CH=CH-(CH₂)₂CH₃ | -CH₃ |
| 31 | -(CH₂)₂CH=CH-CH₂CH₃ | -CH₃ |
| 32 | -(CH₂)₃CH=CH-CH₃ | -CH₃ |
| 33 | -(CH₂)₄CH=CH₂ | -CH₃ |
| 34 | -CH=CH-(CH₂)₃CH₃ | -CH₃ |
| 35 | -CH₂CH=CH-(CH₂)₃CH₃ | -CH₃ |
| 36 | -(CH₂)₂CH=CH-(CH₂)₂CH₃ | -CH₃ |
| 37 | -(CH₂)₃CH=CH-CH₂CH₃ | -CH₃ |
| 38 | -(CH₂)₄CH=CH-CH₃ | -CH₃ |
| 39 | -(CH₂)₅CH=CH₂ | -CH₃ |
| 40 | -CH=CH-(CH₂)₅CH₃ | -CH₃ |
| 41 | -CH₂CH=CH-(CH₂)₄CH₃ | -CH₃ |
| 42 | -(CH₂)₂CH=CH-(CH₂)₃CH₃ | -CH₃ |
| 43 | -(CH₂)₃CH=CH-(CH₂)₂CH₃ | -CH₃ |
| 44 | -(CH₂)₄CH=CH-CH₂CH₃ | -CH₃ |
| 45 | -(CH₂)₅CH=CH-CH₃ | -CH₃ |
| 46 | -(CH₂)₆CH=CH₂ | -CH₃ |
| 47 | -CH₃ | -CH₂CH₃ |
| 48 | -CH₂CH₃ | -CH₂CH₃ |
| 49 | -CH₂CH₂CH₃ | -CH₂CH₃ |
| 50 | -CH(CH₃)₂ | -CH₂CH₃ |
| 51 | -CH₂CH₂CH₂CH₃ | -CH₂CH₃ |
| 52 | -CH(CH₃)CH₂CH₃ | -CH₂CH₃ |
| 53 | -CH₂CH(CH₃)₂ | -CH₂CH₃ |
| 54 | -C(CH₃)₃ | -CH₂CH₃ |
| 55 | -CH₂(CH₂)₃CH₃ | -CH₂CH₃ |
| 56 | -CH(CH₃)(CH₂)₂CH₃ | -CH₂CH₃ |
| 57 | -CH₂CH(CH₃)CH₂CH₃ | -CH₂CH₃ |
| 58 | -(CH₂)₂CH(CH₃)₂ | -CH₂CH₃ |
| 59 | -CH₂(CH₂)₄CH₃ | -CH₂CH₃ |
| 60 | -CH₂(CH₂)₅CH₃ | -CH₂CH₃ |
| 61 | -CH₂(CH₂)₆CH₃ | -CH₂CH₃ |
| 62 | -CH=CH₂ | -CH₂CH₃ |
| 63 | -CH=CHCH₃ | -CH₂CH₃ |
| 64 | -CH=C(CH₃)₂ | -CH₂CH₃ |
| 65 | -C(CH₃)=CH₂ | -CH₂CH₃ |
| 66 | -C(CH₃)=CHCH₃ | -CH₂CH₃ |
| 67 | -C(CH₃)=C(CH₃)₂ | -CH₂CH₃ |
| 68 | -CH=CH-CH₂CH₃ | -CH₂CH₃ |
| 69 | -CH₂CH=CH-CH₃ | -CH₂CH₃ |
| 70 | -(CH₂)₂CH=CH₂ | -CH₂CH₃ |
| 71 | -CH=CH-(CH₂)₂CH₃ | -CH₂CH₃ |
| 72 | -CH₂CH=CH-CH₂CH₃ | -CH₂CH₃ |
| 73 | -(CH₂)₂CH=CH-CH₃ | -CH₂CH₃ |
| 74 | -(CH₂)₃CH=CH₂ | -CH₂CH₃ |
| 75 | -CH=CH-(CH₂)₃CH₃ | -CH₂CH₃ |
| 76 | -CH₂CH=CH-(CH₂)₂CH₃ | -CH₂CH₃ |
| 77 | -(CH₂)₂CH=CH-CH₂CH₃ | -CH₂CH₃ |
| 78 | -(CH₂)₃CH=CH-CH₃ | -CH₂CH₃ |
| 79 | -(CH₂)₄CH=CH₂ | -CH₂CH₃ |
| 80 | -CH=CH-(CH₂)₃CH₃ | -CH₂CH₃ |
| 81 | -CH₂CH=CH-(CH₂)₃CH₃ | -CH₂CH₃ |
| 82 | -(CH₂)₂CH₌CH-(CH₂)₂CH₃ | -CH₂CH₃ |
| 83 | -(CH₂)₃CH=CH-CH₂CH₃ | -CH₂CH₃ |
| 84 | -(CH₂)₄CH=CH-CH₃ | -CH₂CH₃ |
| 85 | -(CH₂)₅CH=CH₂ | -CH₂CH₃ |
| 86 | -CH=CH-(CH₂)₅CH₃ | -CH₂CH₃ |
| 87 | -CH₂CH=CH-(CH₂)₄CH₃ | -CH₂CH₃ |
| 88 | -(CH₂)₂CH=CH-(CH₂)₃CH₃ | -CH₂CH₃ |
| 89 | -(CH₂)₃CH=CH-(CH₂)₂CH₃ | -CH₂CH₃ |
| 90 | -(CH₂)₄CH=CH-CH₂CH₃ | -CH₂CH₃ |
| 91 | -(CH₂)₅CH=CH-CH₃ | -CH₂CH₃ |
| 92 | -(CH₂)₆CH=CH₂ | -CH₂CH₃ |
| 93 | -CH₃ | -CH₂CH₂CH₃ |
| 94 | -CH₂CH₃ | -CH₂CH₂CH₃ |
| 95 | -CH₂CH₂CH₃ | -CH₂CH₂CH₃ |
| 96 | -CH(CH₃)₂ | -CH₂CH₂CH₃ |
| 97 | -CH₂CH₂CH₂CH₃ | -CH₂CH₂CH₃ |
| 98 | -CH(CH₃)CH₂CH₃ | -CH₂CH₂CH₃ |
| 99 | -CH₂CH(CH₃)₂ | -CH₂CH₂CH₃ |
| 100 | -C(CH₃)₃ | -CH₂CH₂CH₃ |
| 101 | -CH₂(CH₂)₃CH₃ | -CH₂CH₂CH₃ |
| 102 | -CH(CH₃)(CH₂)₂CH₃ | -CH₂CH₂CH₃ |
| 103 | -CH₂CH(CH₃)CH₂CH₃ | -CH₂CH₂CH₃ |
| 104 | -(CH₂)₂CH(CH₃)₂ | -CH₂CH₂CH₃ |
| 105 | -CH₂(CH₂)₄CH₃ | -CH₂CH₂CH₃ |
| 106 | -CH₂(CH₂)₅CH₃ | -CH₂CH₂CH₃ |
| 107 | -CH₂(CH₂)₆CH₃ | -CH₂CH₂CH₃ |
| 108 | -CH=CH₂ | -CH₂CH₂CH₃ |
| 109 | -CH=CHCH₃ | -CH₂CH₂CH₃ |
| 110 | -CH=C(CH₃)₂ | -CH₂CH₂CH₃ |
| 111 | -C(CH₃)=CH₂ | -CH₂CH₂CH₃ |
| 112 | -C(CH₃)=CHCH₃ | -CH₂CH₂CH₃ |
| 113 | -C(CH₃)=C(CH₃)₂ | -CH₂CH₂CH₃ |
| 114 | -CH=CH-CH₂CH₃ | -CH₂CH₂CH₃ |
| 115 | -CH₂CH=CH-CH₃ | -CH₂CH₂CH₃ |
| 116 | -(CH₂)₂CH=CH₂ | -CH₂CH₂CH₃ |
| 117 | -CH=CH-(CH₂)₂CH₃ | -CH₂CH₂CH₃ |
| 118 | -CH₂CH=CH-CH₂CH₃ | -CH₂CH₂CH₃ |
| 119 | -(CH₂)₂CH=CH-CH₃ | -CH₂CH₂CH₃ |
| 120 | -(CH₂)₃CH=CH₂ | -CH₂CH₂CH₃ |
| 121 | -CH=CH-(CH₂)₃CH₃ | -CH₂CH₂CH₃ |
| 122 | -CH₂CH=CH-(CH₂)₂CH₃ | -CH₂CH₂CH₃ |
| 123 | -(CH₂)₂CH=CH-CH₂CH₃ | -CH₂CH₂CH₃ |
| 124 | -(CH₂)₃CH=CH-CH₃ | -CH₂CH₂CH₃ |
| 125 | -(CH₂)₄CH=CH₂ | -CH₂CH₂CH₃ |
| 126 | -CH=CH-(CH₂)₃CH₃ | -CH₂CH₂CH₃ |
| 127 | -CH₂CH=CH-(CH₂)₃CH₃ | -CH₂CH₂CH₃ |
| 128 | -(CH₂)₂CH=CH-(CH₂)₂CH₃ | -CH₂CH₂CH₃ |
| 129 | -(CH₂)₃CH=CH-CH₂CH₃ | -CH₂CH₂CH₃ |
| 130 | -(CH₂)₄CH=CH-CH₃ | -CH₂CH₂CH₃ |
| 131 | -(CH₂)₅CH=CH₂ | -CH₂CH₂CH₃ |
| 132 | -CH=CH-(CH₂)₅CH₃ | -CH₂CH₂CH₃ |
| 133 | -CH₂CH=CH-(CH₂)₄CH₃ | -CH₂CH₂CH₃ |
| 134 | -(CH₂)₂CH=CH-(CH₂)₃CH₃ | -CH₂CH₂CH₃ |
| 135 | -(CH₂)₃CH=CH-(CH₂)₂CH₃ | -CH₂CH₂CH₃ |
| 136 | -(CH₂)₄CH=CH-CH₂CH₃ | -CH₂CH₂CH₃ |
| 137 | -(CH₂)₅CH=CH-CH₃ | -CH₂CH₂CH₃ |
| 138 | -(CH₂)₆CH=CH₂ | -CH₂CH₂CH₃ |
| 139 | -CH₃ | -CH(CH₃)₂ |
| 140 | -CH₂CH₃ | -CH(CH₃)₂ |
| 141 | -CH₂CH₂CH₃ | -CH(CH₃)₂ |
| 142 | -CH(CH₃)₂ | -CH(CH₃)₂ |
| 143 | -CH₂CH₂CH₂CH₃ | -CH(CH₃)₂ |
| 144 | -CH(CH₃)CH₂CH₃ | -CH(CH₃)₂ |
| 145 | -CH₂CH(CH₃)₂ | -CH(CH₃)₂ |
| 146 | -C(CH₃)₃ | -CH(CH₃)₂ |
| 147 | -CH₂(CH₂)₃CH₃ | -CH(CH₃)₂ |
| 148 | -CH(CH₃)(CH₂)₂CH₃ | -CH(CH₃)₂ |
| 149 | -CH₂CH(CH₃)CH₂CH₃ | -CH(CH₃)₂ |
| 150 | -(CH₂)₂CH(CH₃)₂ | -CH(CH₃)₂ |
| 151 | -CH₂(CH₂)₄CH₃ | -CH(CH₃)₂ |
| 152 | -CH₂(CH₂)₅CH₃ | -CH(CH₃)₂ |
| 153 | -CH₂(CH₂)₆CH₃ | -CH(CH₃)₂ |
| 154 | -CH=CH₂ | -CH(CH₃)₂ |
| 155 | -CH=CHCH₃ | -CH(CH₃)₂ |
| 156 | -CH=C(CH₃)₂ | -CH(CH₃)₂ |
| 157 | -C(CH₃)=CH₂ | -CH(CH₃)₂ |
| 158 | -C(CH₃)=CHCH₃ | -CH(CH₃)₂ |
| 159 | -C(CH₃)=C(CH₃)₂ | -CH(CH₃)₂ |
| 160 | -CH=CH-CH₂CH₃ | -CH(CH₃)₂ |
| 161 | -CH₂CH=CH-CH₃ | -CH(CH₃)₂ |
| 162 | -(CH₂)₂CH=CH₂ | -CH(CH₃)₂ |
| 163 | -CH=CH-(CH₂)₂CH₃ | -CH(CH₃)₂ |
| 164 | -CH₂CH=CH-CH₂CH₃ | -CH(CH₃)₂ |
| 165 | -(CH₂)₂CH=CH-CH₃ | -CH(CH₃)₂ |
| 166 | -(CH₂)₃CH=CH₂ | -CH(CH₃)₂ |
| 167 | -CH=CH-(CH₂)₃CH₃ | -CH(CH₃)₂ |
| 168 | -CH₂CH=CH-(CH₂)₂CH₃ | -CH(CH₃)₂ |
| 169 | -(CH₂)₂CH=CH-CH₂CH₃ | -CH(CH₃)₂ |
| 170 | -(CH₂)₃CH=CH-CH₃ | -CH(CH₃)₂ |
| 171 | -(CH₂)₄CH=CH₂ | -CH(CH₃)₂ |
| 172 | -CH=CH-(CH₂)₃CH₃ | -CH(CH₃)₂ |
| 173 | -CH₂CH=CH-(CH₂)₃CH₃ | -CH(CH₃)₂ |
| 174 | -(CH₂)₂CH=CH-(CH₂)₂CH₃ | -CH(CH₃)₂ |
| 175 | -(CH₂)₃CH=CH-CH₂CH₃ | -CH(CH₃)₂ |
| 176 | -(CH₂)₄CH=CH-CH₃ | -CH(CH₃)₂ |
| 177 | -(CH₂)₅CH=CH₂ | -CH(CH₃)₂ |
| 178 | -CH=CH-(CH₂)₅CH₃ | -CH(CH₃)₂ |
| 179 | -CH₂CH=CH-(CH₂)₄CH₃ | -CH(CH₃)₂ |
| 180 | -(CH₂)₂CH=CH-(CH₂)₃CH₃ | -CH(CH₃)₂ |
| 181 | -(CH₂)₃CH=CH-(CH₂)₂CH₃ | -CH(CH₃)₂ |
| 182 | -(CH₂)₄CH=CH-CH₂CH₃ | -CH(CH₃)₂ |
| 183 | -(CH₂)₅CH=CH-CH₃ | -CH(CH₃)₂ |
| 184 | -(CH₂)₆CH=CH₂ | -CH(CH₃)₂ |
| 185 | -CH₃ | -CH₂CH₂CH₂CH₃ |
| 186 | -CH₂CH₃ | -CH₂CH₂CH₂CH₃ |
| 187 | -CH₂CH₂CH₃ | -CH₂CH₂CH₂CH₃ |
| 188 | -CH(CH₃)₂ | -CH₂CH₂CH₂CH₃ |
| 189 | -CH₂CH₂CH₂CH₃ | -CH₂CH₂CH₂CH₃ |
| 190 | -CH(CH₃)CH₂CH₃ | -CH₂CH₂CH₂CH₃ |
| 191 | -CH₂CH(CH₃)₂ | -CH₂CH₂CH₂CH₃ |
| 192 | -C(CH₃)₃ | -CH₂CH₂CH₂CH₃ |
| 193 | -CH₂(CH₂)₃CH₃ | -CH₂CH₂CH₂CH₃ |
| 194 | -CH(CH₃)(CH₂)₂CH₃ | -CH₂CH₂CH₂CH₃ |
| 195 | -CH₂CH(CH₃)CH₂CH₃ | -CH₂CH₂CH₂CH₃ |
| 196 | -(CH₂)₂CH(CH₃)₂ | -CH₂CH₂CH₂CH₃ |
| 197 | -CH₂(CH₂)₄CH₃ | -CH₂CH₂CH₂CH₃ |
| 198 | -CH₂(CH₂)₅CH₃ | -CH₂CH₂CH₂CH₃ |
| 199 | -CH₂(CH₂)₆CH₃ | -CH₂CH₂CH₂CH₃ |
| 200 | -CH=CH₂ | -CH₂CH₂CH₂CH₃ |
| 201 | -CH=CHCH₃ | -CH₂CH₂CH₂CH₃ |
| 202 | -CH=C(CH₃)₂ | -CH₂CH₂CH₂CH₃ |
| 203 | -C(CH₃)=CH₂ | -CH₂CH₂CH₂CH₃ |
| 204 | -C(CH₃)=CHCH₃ | -CH₂CH₂CH₂CH₃ |
| 205 | -C(CH₃)=C(CH₃)₂ | -CH₂CH₂CH₂CH₃ |
| 206 | -CH=CH-CH₂CH₃ | -CH₂CH₂CH₂CH₃ |
| 207 | -CH₂CH=CH-CH₃ | -CH₂CH₂CH₂CH₃ |
| 208 | -(CH₂)₂CH=CH₂ | -CH₂CH₂CH₂CH₃ |
| 209 | -CH=CH-(CH₂)₂CH₃ | -CH₂CH₂CH₂CH₃ |
| 210 | -CH₂CH=CH-CH₂CH₃ | -CH₂CH₂CH₂CH₃ |
| 211 | -(CH₂)₂CH=CH-CH₃ | -CH₂CH₂CH₂CH₃ |
| 212 | -(CH₂)₃CH=CH₂ | -CH₂CH₂CH₂CH₃ |
| 213 | -CH=CH-(CH₂)₃CH₃ | -CH₂CH₂CH₂CH₃ |
| 214 | -CH₂CH=CH-(CH₂)₂CH₃ | -CH₂CH₂CH₂CH₃ |
| 215 | -(CH₂)₂CH=CH-CH₂CH₃ | -CH₂CH₂CH₂CH₃ |
| 216 | -(CH₂)₃CH=CH-CH₃ | -CH₂CH₂CH₂CH₃ |
| 217 | -(CH₂)₄CH=CH₂ | -CH₂CH₂CH₂CH₃ |
| 218 | -CH=CH-(CH₂)₃CH₃ | -CH₂CH₂CH₂CH₃ |
| 219 | -CH₂CH=CH-(CH₂)₃CH₃ | -CH₂CH₂CH₂CH₃ |
| 220 | -(CH₂)₂CH=CH-(CH₂)₂CH₃ | -CH₂CH₂CH₂CH₃ |
| 221 | -(CH₂)₃CH=CH-CH₂CH₃ | -CH₂CH₂CH₂CH₃ |
| 222 | -(CH₂)₄CH=CH-CH₃ | -CH₂CH₂CH₂CH₃ |
| 223 | -(CH₂)₅CH=CH₂ | -CH₂CH₂CH₂CH₃ |
| 224 | -CH=CH-(CH₂)₅CH₃ | -CH₂CH₂CH₂CH₃ |
| 225 | -CH₂CH=CH-(CH₂)₄CH₃ | -CH₂CH₂CH₂CH₃ |
| 226 | -(CH₂)₂CH=CH-(CH₂)₃CH₃ | -CH₂CH₂CH₂CH₃ |
| 227 | -(CH₂)₃CH=CH-(CH₂)₂CH₃ | -CH₂CH₂CH₂CH₃ |
| 228 | -(CH₂)₄CH=CH-CH₂CH₃ | -CH₂CH₂CH₂CH₃ |
| 229 | -(CH₂)₅CH=CH-CH₃ | -CH₂CH₂CH₂CH₃ |
| 230 | -(CH₂)₆CH=CH₂ | -CH₂CH₂CH₂CH₃ |
| 231 | -CH₃ | -CH(CH₃)CH₂CH₃ |
| 232 | -CH₂CH₃ | -CH(CH₃)CH₂CH₃ |
| 233 | -CH₂CH₂CH₃ | -CH(CH₃)CH₂CH₃ |
| 234 | -CH(CH₃)₂ | -CH(CH₃)CH₂CH₃ |
| 235 | -CH₂CH₂CH₂CH₃ | -CH(CH₃)CH₂CH₃ |
| 236 | -CH(CH₃)CH₂CH₃ | -CH(CH₃)CH₂CH₃ |
| 237 | -CH₂CH(CH₃)₂ | -CH(CH₃)CH₂CH₃ |
| 238 | -C(CH₃)₃ | -CH(CH₃)CH₂CH₃ |
| 239 | -CH₂(CH₂)₃CH₃ | -CH(CH₃)CH₂CH₃ |
| 240 | -CH(CH₃)(CH₂)₂CH₃ | -CH(CH₃)CH₂CH₃ |
| 241 | -CH₂CH(CH₃)CH₂CH₃ | -CH(CH₃)CH₂CH₃ |
| 242 | -(CH₂)₂CH(CH₃)₂ | -CH(CH₃)CH₂CH₃ |
| 243 | -CH₂(CH₂)₄CH₃ | -CH(CH₃)CH₂CH₃ |
| 244 | -CH₂(CH₂)₅CH₃ | -CH(CH₃)CH₂CH₃ |
| 245 | -CH₂(CH₂)₆CH₃ | -CH(CH₃)CH₂CH₃ |
| 246 | -CH=CH₂ | -CH(CH₃)CH₂CH₃ |
| 247 | -CH=CHCH₃ | -CH(CH₃)CH₂CH₃ |
| 248 | -CH=C(CH₃)₂ | -CH(CH₃)CH₂CH₃ |
| 249 | -C(CH₃)=CH₂ | -CH(CH₃)CH₂CH₃ |
| 250 | -C(CH₃)=CHCH₃ | -CH(CH₃)CH₂CH₃ |
| 251 | -C(CH₃)=C(CH₃)₂ | -CH(CH₃)CH₂CH₃ |
| 252 | -CH=CH-CH₂CH₃ | -CH(CH₃)CH₂CH₃ |
| 253 | -CH₂CH=CH-CH₃ | -CH(CH₃)CH₂CH₃ |
| 254 | -(CH₂)₂CH=CH₂ | -CH(CH₃)CH₂CH₃ |
| 255 | -CH=CH-(CH₂)₂CH₃ | -CH(CH₃)CH₂CH₃ |
| 256 | -CH₂CH=CH-CH₂CH₃ | -CH(CH₃)CH₂CH₃ |
| 257 | -(CH₂)₂CH=CH-CH₃ | -CH(CH₃)CH₂CH₃ |
| 258 | -(CH₂)₃CH=CH₂ | -CH(CH₃)CH₂CH₃ |
| 259 | -CH=CH-(CH₂)₃CH₃ | -CH(CH₃)CH₂CH₃ |
| 260 | -CH₂CH=CH-(CH₂)₂CH₃ | -CH(CH₃)CH₂CH₃ |
| 261 | -(CH₂)₂CH=CH-CH₂CH₃ | -CH(CH₃)CH₂CH₃ |
| 262 | -(CH₂)₃CH=CH-CH₃ | -CH(CH₃)CH₂CH₃ |
| 263 | -(CH₂)₄CH=CH₂ | -CH(CH₃)CH₂CH₃ |
| 264 | -CH=CH-(CH₂)₃CH₃ | -CH(CH₃)CH₂CH₃ |
| 265 | -CH₂CH=CH-(CH₂)₃CH₃ | -CH(CH₃)CH₂CH₃ |
| 266 | -(CH₂)₂CH=CH-(CH₂)₂CH₃ | -CH(CH₃)CH₂CH₃ |
| 267 | -(CH₂)₃CH=CH-CH₂CH₃ | -CH(CH₃)CH₂CH₃ |
| 268 | -(CH₂)₄CH=CH-CH₃ | -CH(CH₃)CH₂CH₃ |
| 269 | -(CH₂)₅CH=CH₂ | -CH(CH₃)CH₂CH₃ |
| 270 | -CH=CH-(CH₂)₅CH₃ | -CH(CH₃)CH₂CH₃ |
| 271 | -CH₂CH=CH-(CH₂)₄CH₃ | -CH(CH₃)CH₂CH₃ |
| 272 | -(CH₂)₂CH=CH-(CH₂)₃CH₃ | -CH(CH₃)CH₂CH₃ |
| 273 | -(CH₂)₃CH=CH-(CH₂)₂CH₃ | -CH(CH₃)CH₂CH₃ |
| 274 | -(CH₂)₄CH=CH-CH₂CH₃ | -CH(CH₃)CH₂CH₃ |
| 275 | -(CH₂)₅CH=CH-CH₃ | -CH(CH₃)CH₂CH₃ |
| 276 | -(CH₂)₆CH=CH₂ | -CH(CH₃)CH₂CH₃ |
| 277 | -CH₃ | -CH₂CH(CH₃)₂ |
| 278 | -CH₂CH₃ | -CH₂CH(CH₃)₂ |
| 279 | -CH₂CH₂CH₃ | -CH₂CH(CH₃)₂ |
| 280 | -CH(CH₃)₂ | -CH₂CH(CH₃)₂ |
| 281 | -CH₂CH₂CH₂CH₃ | -CH₂CH(CH₃)₂ |
| 282 | -CH(CH₃)CH₂CH₃ | -CH₂CH(CH₃)₂ |
| 283 | -CH₂CH(CH₃)₂ | -CH₂CH(CH₃)₂ |
| 284 | -C(CH₃)₃ | -CH₂CH(CH₃)₂ |
| 285 | -CH₂(CH₂)₃CH₃ | -CH₂CH(CH₃)₂ |
| 286 | -CH(CH₃)(CH₂)₂CH₃ | -CH₂CH(CH₃)₂ |
| 287 | -CH₂CH(CH₃)CH₂CH₃ | -CH₂CH(CH₃)₂ |
| 288 | -(CH₂)₂CH(CH₃)₂ | -CH₂CH(CH₃)₂ |
| 289 | -CH₂(CH₂)₄CH₃ | -CH₂CH(CH₃)₂ |
| 290 | -CH₂(CH₂)₅CH₃ | -CH₂CH(CH₃)₂ |
| 291 | -CH₂(CH₂)₆CH₃ | -CH₂CH(CH₃)₂ |
| 292 | -CH=CH₂ | -CH₂CH(CH₃)₂ |
| 293 | -CH=CHCH₃ | -CH₂CH(CH₃)₂ |
| 294 | -CH=C(CH₃)₂ | -CH₂CH(CH₃)₂ |
| 295 | -C(CH₃)=CH₂ | -CH₂CH(CH₃)₂ |
| 296 | -C(CH₃)=CHCH₃ | -CH₂CH(CH₃)₂ |
| 297 | -C(CH₃)=C(CH₃)₂ | -CH₂CH(CH₃)₂ |
| 298 | -CH=CH-CH₂CH₃ | -CH₂CH(CH₃)₂ |
| 299 | -CH₂CH=CH-CH₃ | -CH₂CH(CH₃)₂ |
| 300 | -(CH₂)₂CH=CH₂ | -CH₂CH(CH₃)₂ |
| 301 | -CH=CH-(CH₂)₂CH₃ | -CH₂CH(CH₃)₂ |
| 302 | -CH₂CH=CH-CH₂CH₃ | -CH₂CH(CH₃)₂ |
| 303 | -(CH₂)₂CH=CH-CH₃ | -CH₂CH(CH₃)₂ |
| 304 | -(CH₂)₃CH=CH₂ | -CH₂CH(CH₃)₂ |
| 305 | -CH=CH-(CH₂)₃CH₃ | -CH₂CH(CH₃)₂ |
| 306 | -CH₂CH=CH-(CH₂)₂CH₃ | -CH₂CH(CH₃)₂ |
| 307 | -(CH₂)₂CH=CH-CH₂CH₃ | -CH₂CH(CH₃)₂ |
| 308 | -(CH₂)₃CH=CH-CH₃ | -CH₂CH(CH₃)₂ |
| 309 | -(CH₂)₄CH=CH₂ | -CH₂CH(CH₃)₂ |
| 310 | -CH=CH-(CH₂)₃CH₃ | -CH₂CH(CH₃)₂ |
| 311 | -CH₂CH=CH-(CH₂)₃CH₃ | -CH₂CH(CH₃)₂ |
| 312 | -(CH₂)₂CH=CH-(CH₂)₂CH₃ | -CH₂CH(CH₃)₂ |
| 313 | -(CH₂)₃CH=CH-CH₂CH₃ | -CH₂CH(CH₃)₂ |
| 314 | -(CH₂)₄CH=CH-CH₃ | -CH₂CH(CH₃)₂ |
| 315 | -(CH₂)₅CH=CH₂ | -CH₂CH(CH₃)₂ |
| 316 | -CH=CH-(CH₂)₅CH₃ | -CH₂CH(CH₃)₂ |
| 317 | -CH₂CH=CH-(CH₂)₄CH₃ | -CH₂CH(CH₃)₂ |
| 318 | -(CH₂)₂CH=CH-(CH₂)₃CH₃ | -CH₂CH(CH₃)₂ |
| 319 | -(CH₂)₃CH=CH-(CH₂)₂CH₃ | -CH₂CH(CH₃)₂ |
| 320 | -(CH₂)₄CH=CH-CH₂CH₃ | -CH₂CH(CH₃)₂ |
| 321 | -(CH₂)₅CH=CH-CH₃ | -CH₂CH(CH₃)₂ |
| 322 | -(CH₂)₆CH=CH₂ | -CH₂CH(CH₃)₂ |
| 323 | -CH₃ | -C(CH₃)₃ |
| 324 | -CH₂CH₃ | -C(CH₃)₃ |
| 325 | -CH₂CH₂CH₃ | -C(CH₃)₃ |
| 326 | -CH(CH₃)₂ | -C(CH₃)₃ |
| 327 | -CH₂CH₂CH₂CH₃ | -C(CH₃)₃ |
| 328 | -CH(CH₃)CH₂CH₃ | -C(CH₃)₃ |
| 329 | -CH₂CH(CH₃)₂ | -C(CH₃)₃ |
| 330 | -C(CH₃)₃ | -C(CH₃)₃ |
| 331 | -CH₂(CH₂)₃CH₃ | -C(CH₃)₃ |
| 332 | -CH(CH₃)(CH₂)₂CH₃ | -C(CH₃)₃ |
| 333 | -CH₂CH(CH₃)CH₂CH₃ | -C(CH₃)₃ |
| 334 | -(CH₂)₂CH(CH₃)₂ | -C(CH₃)₃ |
| 335 | -CH₂(CH₂)₄CH₃ | -C(CH₃)₃ |
| 336 | -CH₂(CH₂)₅CH₃ | -C(CH₃)₃ |
| 337 | -CH₂(CH₂)₆CH₃ | -C(CH₃)₃ |
| 338 | -CH=CH₂ | -C(CH₃)₃ |
| 339 | -CH=CHCH₃ | -C(CH₃)₃ |
| 340 | -CH=C(CH₃)₂ | -C(CH₃)₃ |
| 341 | -C(CH₃)=CH₂ | -C(CH₃)₃ |
| 342 | -C(CH₃)=CHCH₃ | -C(CH₃)₃ |
| 343 | -C(CH₃)=C(CH₃)₂ | -C(CH₃)₃ |
| 344 | -CH=CH-CH₂CH₃ | -C(CH₃)₃ |
| 345 | -CH₂CH=CH-CH₃ | -C(CH₃)₃ |
| 346 | -(CH₂)₂CH=CH₂ | -C(CH₃)₃ |
| 347 | -CH=CH-(CH₂)₂CH₃ | -C(CH₃)₃ |
| 348 | -CH₂CH=CH-CH₂CH₃ | -C(CH₃)₃ |
| 349 | -(CH₂)₂CH=CH-CH₃ | -C(CH₃)₃ |
| 350 | -(CH₂)₃CH=CH₂ | -C(CH₃)₃ |
| 351 | -CH=CH-(CH₂)₃CH₃ | -C(CH₃)₃ |
| 352 | -CH₂CH=CH-(CH₂)₂CH₃ | -C(CH₃)₃ |
| 353 | -(CH₂)₂CH=CH-CH₂CH₃ | -C(CH₃)₃ |
| 354 | -(CH₂)₃CH=CH-CH₃ | -C(CH₃)₃ |
| 355 | -(CH₂)₄CH=CH₂ | -C(CH₃)₃ |
| 356 | -CH=CH-(CH₂)₃CH₃ | -C(CH₃)₃ |
| 357 | -CH₂CH=CH-(CH₂)₃CH₃ | -C(CH₃)₃ |
| 358 | -(CH₂)₂CH=CH-(CH₂)₂CH₃ | -C(CH₃)₃ |
| 359 | -(CH₂)₃CH=CH-CH₂CH₃ | -C(CH₃)₃ |
| 360 | -(CH₂)₄CH=CH-CH₃ | -C(CH₃)₃ |
| 361 | -(CH₂)₅CH=CH₂ | -C(CH₃)₃ |
| 362 | -CH=CH-(CH₂)₅CH₃ | -C(CH₃)₃ |
| 363 | -CH₂CH=CH-(CH₂)₄CH₃ | -C(CH₃)₃ |
| 364 | -(CH₂)₂CH=CH-(CH₂)₃CH₃ | -C(CH₃)₃ |
| 365 | -(CH₂)₃CH=CH-(CH₂)₂CH₃ | -C(CH₃)₃ |
| 366 | -(CH₂)₄CH=CH-CH₂CH₃ | -C(CH₃)₃ |
| 367 | -(CH₂)₅CH=CH-CH₃ | -C(CH₃)₃ |
| 368 | -(CH₂)₆CH=CH₂ | -C(CH₃)₃ |
| 369 | -CH₃ | -CH₂(CH₂)₃CH₃ |
| 370 | -CH₂CH₃ | -CH₂(CH₂)₃CH₃ |
| 371 | -CH₂CH₂CH₃ | -CH₂(CH₂)₃CH₃ |
| 372 | -CH(CH₃)₂ | -CH₂(CH₂)₃CH₃ |
| 373 | -CH₂CH₂CH₂CH₃ | -CH₂(CH₂)₃CH₃ |
| 374 | -CH(CH₃)CH₂CH₃ | -CH₂(CH₂)₃CH₃ |
| 375 | -CH₂CH(CH₃)₂ | -CH₂(CH₂)₃CH₃ |
| 376 | -C(CH₃)₃ | -CH₂(CH₂)₃CH₃ |
| 377 | -CH₂(CH₂)₃CH₃ | -CH₂(CH₂)₃CH₃ |
| 378 | -CH(CH₃)(CH₂)₂CH₃ | -CH₂(CH₂)₃CH₃ |
| 379 | -CH₂CH(CH₃)CH₂CH₃ | -CH₂(CH₂)₃CH₃ |
| 380 | -(CH₂)₂CH(CH₃)₂ | -CH₂(CH₂)₃CH₃ |
| 381 | -CH₂(CH₂)₄CH₃ | -CH₂(CH₂)₃CH₃ |
| 382 | -CH₂(CH₂)₅CH₃ | -CH₂(CH₂)₃CH₃ |
| 383 | -CH₂(CH₂)₆CH₃ | -CH₂(CH₂)₃CH₃ |
| 384 | -CH=CH₂ | -CH₂(CH₂)₃CH₃ |
| 385 | -CH=CHCH₃ | -CH₂(CH₂)₃CH₃ |
| 386 | -CH=C(CH₃)₂ | -CH₂(CH₂)₃CH₃ |
| 387 | -C(CH₃)=CH₂ | -CH₂(CH₂)₃CH₃ |
| 388 | -C(CH₃)=CHCH₃ | -CH₂(CH₂)₃CH₃ |
| 389 | -C(CH₃)=C(CH₃)₂ | -CH₂(CH₂)₃CH₃ |
| 390 | -CH=CH-CH₂CH₃ | -CH₂(CH₂)₃CH₃ |
| 391 | -CH₂CH=CH-CH₃ | -CH₂(CH₂)₃CH₃ |
| 392 | -(CH₂)₂CH=CH₂ | -CH₂(CH₂)₃CH₃ |
| 393 | -CH=CH-(CH₂)₂CH₃ | -CH₂(CH₂)₃CH₃ |
| 394 | -CH₂CH=CH-CH₂CH₃ | -CH₂(CH₂)₃CH₃ |
| 395 | -(CH₂)₂CH=CH-CH₃ | -CH₂(CH₂)₃CH₃ |
| 396 | -(CH₂)₃CH=CH₂ | -CH₂(CH₂)₃CH₃ |
| 397 | -CH=CH-(CH₂)₃CH₃ | -CH₂(CH₂)₃CH₃ |
| 398 | -CH₂CH=CH-(CH₂)₂CH₃ | -CH₂(CH₂)₃CH₃ |
| 399 | -(CH₂)₂CH=CH-CH₂CH₃ | -CH₂(CH₂)₃CH₃ |
| 400 | -(CH₂)₃CH=CH-CH₃ | -CH₂(CH₂)₃CH₃ |
| 401 | -(CH₂)₄CH=CH₂ | -CH₂(CH₂)₃CH₃ |
| 402 | -CH=CH-(CH₂)₃CH₃ | -CH₂(CH₂)₃CH₃ |
| 403 | -CH₂CH=CH-(CH₂)₃CH₃ | -CH₂(CH₂)₃CH₃ |
| 404 | -(CH₂)₂CH=CH-(CH₂)₂CH₃ | -CH₂(CH₂)₃CH₃ |
| 405 | -(CH₂)₃CH=CH-CH₂CH₃ | -CH₂(CH₂)₃CH₃ |
| 406 | -(CH₂)₄CH=CH-CH₃ | -CH₂(CH₂)₃CH₃ |
| 407 | -(CH₂)₅CH=CH₂ | -CH₂(CH₂)₃CH₃ |
| 408 | -CH=CH-(CH₂)₅CH₃ | -CH₂(CH₂)₃CH₃ |
| 409 | -CH₂CH=CH-(CH₂)₄CH₃ | -CH₂(CH₂)₃CH₃ |
| 410 | -(CH₂)₂CH=CH-(CH₂)₃CH₃ | -CH₂(CH₂)₃CH₃ |
| 411 | -(CH₂)₃CH=CH-(CH₂)₂CH₃ | -CH₂(CH₂)₃CH₃ |
| 412 | -(CH₂)₄CH=CH-CH₂CH₃ | -CH₂(CH₂)₃CH₃ |
| 413 | -(CH₂)₅CH=CH-CH₃ | -CH₂(CH₂)₃CH₃ |
| 414 | -(CH₂)₆CH=CH₂ | -CH₂(CH₂)₃CH₃ |
| 415 | -CH₃ | -CH(CH₃)(CH₂)₂CH₃ |
| 416 | -CH₂CH₃ | -CH(CH₃)(CH₂)₂CH₃ |
| 417 | -CH₂CH₂CH₃ | -CH(CH₃)(CH₂)₂CH₃ |
| 418 | -CH(CH₃)₂ | -CH(CH₃)(CH₂)₂CH₃ |
| 419 | -CH₂CH₂CH₂CH₃ | -CH(CH₃)(CH₂)₂CH₃ |
| 420 | -CH(CH₃)CH₂CH₃ | -CH(CH₃)(CH₂)₂CH₃ |
| 421 | -CH₂CH(CH₃)₂ | -CH(CH₃)(CH₂)₂CH₃ |
| 422 | -C(CH₃)₃ | -CH(CH₃)(CH₂)₂CH₃ |
| 423 | -CH₂(CH₂)₃CH₃ | -CH(CH₃)(CH₂)₂CH₃ |
| 424 | -CH(CH₃)(CH₂)₂CH₃ | -CH(CH₃)(CH₂)₂CH₃ |
| 425 | -CH₂CH(CH₃)CH₂CH₃ | -CH(CH₃)(CH₂)₂CH₃ |
| 426 | -(CH₂)₂CH(CH₃)₂ | -CH(CH₃)(CH₂)₂CH₃ |
| 427 | -CH₂(CH₂)₄CH₃ | -CH(CH₃)(CH₂)₂CH₃ |
| 428 | -CH₂(CH₂)₅CH₃ | -CH(CH₃)(CH₂)₂CH₃ |
| 429 | -CH₂(CH₂)₆CH₃ | -CH(CH₃)(CH₂)₂CH₃ |
| 430 | -CH=CH₂ | -CH(CH₃)(CH₂)₂CH₃ |
| 431 | -CH=CHCH₃ | -CH(CH₃)(CH₂)₂CH₃ |
| 432 | -CH=C(CH₃)₂ | -CH(CH₃)(CH₂)₂CH₃ |
| 433 | -C(CH₃)=CH₂ | -CH(CH₃)(CH₂)₂CH₃ |
| 434 | -C(CH₃)=CHCH₃ | -CH(CH₃)(Ch₂)₂CH₃ |
| 435 | -C(CH₃)=C(CH₃)_{z} | -CH(CH₃)(CH₂)₂CH₃ |
| 436 | -CH=CH-CH₂CH₃ | -CH(CH₃)(CH₂)₂CH₃ |
| 437 | -CH₂CH=CH-CH₃ | -CH(CH₃)(CH₂)₂CH₃ |
| 438 | -(CH₂)₂CH=CH₂ | -CH(CH₃)(CH₂)₂CH₃ |
| 439 | -CH=CH-(CH₂)₂CH₃ | -CH(CH₃)(CH₂)₂CH₃ |
| 440 | -CH₂CH=CH-CH₂CH₃ | -CH(CH₃)(CH₂)₂CH₃ |
| 441 | -(CH₂)₂CH=CH-CH₃ | -CH(CH₃)(CH₂)₂CH₃ |
| 442 | -(CH₂)₃CH=CH₂ | -CH(CH₃)(CH₂)₂CH₃ |
| 443 | -CH=CH-(CH₂)₃CH₃ | -CH(CH₃)(CH₂)₂CH₃ |
| 444 | -CH₂CH=CH-(CH₂)₂CH₃ | -CH(CH₃)(CH₂)₂CH₃ |
| 445 | -(CH₂)₂CH=CH-CH₂CH₃ | -CH(CH₃)(CH₂)₂CH₃ |
| 446 | -(CH₂)₃CH=CH-CH₃ | -CH(CH₃)(CH₂)₂CH₃ |
| 447 | -(CH₂)₄CH=CH₂ | -CH(CH₃)(CH₂)₂CH₃ |
| 448 | -CH=CH-(CH₂)₃CH₃ | -CH(CH₃)(CH₂)₂CH₃ |
| 449 | -CH₂CH=CH-(CH₂)₃CH₃ | -CH(CH₃)(CH₂)₂CH₃ |
| 450 | -(CH₂)₂CH=CH-(CH₂)₂CH₃ | -CH(CH₃)(CH₂)₂CH₃ |
| 451 | -(CH₂)₃CH=CH-CH₂CH₃ | -CH(CH₃)(CH₂)₂CH₃ |
| 452 | -(CH₂)₄CH=CH-CH₃ | -CH(CH₃)(CH₂)₂CH₃ |
| 453 | -(CH₂)₅CH=CH₂ | -CH(CH₃)(CH₂)₂CH₃ |
| 454 | -CH=CH-(CH₂)₅CH₃ | -CH(CH₃)(CH₂)₂CH₃ |
| 455 | -CH₂CH=CH-(CH₂)₄CH₃ | -CH(CH₃)(CH₂)₂CH₃ |
| 456 | -(CH₂)₂CH=CH-(CH₂)₃CH₃ | -CH(CH₃)(CH₂)₂CH₃ |
| 457 | -(CH₂)₃CH=CH-(CH₂)₂CH₃ | -CH(CH₃)(CH₂)₂CH₃ |
| 458 | -(CH₂)₄CH=CH-CH₂CH₃ | -CH(CH₃)(CH₂)₂CH₃ |
| 459 | -(CH₂)₅CH=CH-CH₃ | -CH(CH₃)(CH₂)₂CH₃ |
| 460 | -(CH₂)₆CH=CH₂ | -CH(CH₃)(CH₂)₂CH₃ |
| 461 | -CH₃ | -CH₂CH(CH₃)CH₂CH₃ |
| 462 | -CH₂CH₃ | -CH₂CH(CH₃)CH₂CH₃ |
| 463 | -CH₂CH₂CH₃ | -CH₂CH(CH₃)CH₂CH₃ |
| 464 | -CH(CH₃)₂ | -CH₂CH(CH₃)CH₂CH₃ |
| 465 | -CH₂CH₂CH₂CH₃ | -CH₂CH(CH₃)CH₂CH₃ |
| 466 | -CH(CH₃)CH₂CH₃ | -CH₂CH(CH₃)CH₂CH₃ |
| 467 | -CH₂CH(CH₃)₂ | -CH₂CH(CH₃)CH₂CH₃ |
| 468 | -C(CH₃)₃ | -CH₂CH(CH₃)CH₂CH₃ |
| 469 | -CH₂(CH₂)₃CH₃ | -CH₂CH(CH₃)CH₂CH₃ |
| 470 | -CH(CH₃)(CH₂)₂CH₃ | -CH₂CH(CH₃)CH₂CH₃ |
| 471 | -CH₂CH(CH₃)CH₂CH₃ | -CH₂CH(CH₃)CH₂CH₃ |
| 472 | -(CH₂)₂CH(CH₃)₂ | -CH₂CH(CH₃)CH₂CH₃ |
| 473 | -CH₂(CH₂)₄CH₃ | -CH₂CH(CH₃)CH₂CH₃ |
| 474 | -CH₂(CH₂)₅CH₃ | -CH₂CH(CH₃)CH₂CH₃ |
| 475 | -CH₂(CH₂)₆CH₃ | -CH₂CH(CH₃)CH₂CH₃ |
| 476 | -CH=CH₂ | -CH₂CH(CH₃)CH₂CH₃ |
| 477 | -CH=CHCH₃ | -CH₂CH(CH₃)CH₂CH₃ |
| 478 | -CH=C(CH₃)₂ | -CH₂CH(CH₃)CH₂CH₃ |
| 479 | -C(CH₃)=CH₂ | -CH₂CH(CH₃)CH₂CH₃ |
| 480 | -C(CH₃)=CHCH₃ | -CH₂CH(CH₃)CH₂CH₃ |
| 481 | -C(CH₃)=C(CH₃)₂ | -CH₂CH(CH₃)CH₂CH₃ |
| 482 | -CH=CH-CH₂CH₃ | -CH₂CH(CH₃)CH₂CH₃ |
| 483 | -CH₂CH=CH-CH₃ | -CH₂CH(CH₃)CH₂CH₃ |
| 484 | -(CH₂)₂CH=CH₂ | -CH₂CH(CH₃)CH₂CH₃ |
| 485 | -CH=CH-(CH₂)₂CH₃ | -CH₂CH(CH₃)CH₂CH₃ |
| 486 | -CH₂CH=CH-CH₂CH₃ | -CH₂CH(CH₃)CH₂CH₃ |
| 487 | -(CH₂)₂CH=CH-CH₃ | -CH₂CH(CH₃)CH₂CH₃ |
| 488 | -(CH₂)₃CH=CH₂ | -CH₂CH(CH₃)CH₂CH₃ |
| 489 | -CH=CH-(CH₂)₃CH₃ | -CH₂CH(CH₃)CH₂CH₃ |
| 490 | -CH₂CH=CH-(CH₂)₂CH₃ | -CH₂CH(CH₃)CH₂CH₃ |
| 491 | -(CH₂)₂CH=CH-CH₂CH₃ | -CH₂CH(CH₃)CH₂CH₃ |
| 492 | -(CH₂)₃CH=CH-CH₃ | -CH₂CH(CH₃)CH₂CH₃ |
| 493 | -(CH₂)₄CH=CH₂ | -CH₂CH(CH₃)CH₂CH₃ |
| 494 | -CH=CH-(CH₂)₃CH₃ | -CH₂CH(CH₃)CH₂CH₃ |
| 495 | -CH₂CH=CH-(CH₂)₃CH₃ | -CH₂CH(CH₃)CH₂CH₃ |
| 496 | -(CH₂)₂CH=CH-(CH₂)₂CH₃ | -CH₂CH(CH₃)CH₂CH₃ |
| 497 | -(CH₂)₃CH=CH-CH₂CH₃ | -CH₂CH(CH₃)CH₂CH₃ |
| 498 | -(CH₂)₄CH=CH-CH₃ | -CH₂CH(CH₃)CH₂CH₃ |
| 499 | -(CH₂)₅CH=CH₂ | -CH₂CH(CH₃)CH₂CH₃ |
| 500 | -CH=CH-(CH₂)₅CH₃ | -CH₂CH(CH₃)CH₂CH₃ |
| 501 | -CH₂CH=CH-(CH₂)₄CH₃ | -CH₂CH(CH₃)CH₂CH₃ |
| 502 | -(CH₂)₂CH=CH-(CH₂)₃CH₃ | -CH₂CH(CH₃)CH₂CH₃ |
| 503 | -(CH₂)₃CH=CH-(CH₂)₂CH₃ | -CH₂CH(CH₃)CH₂CH₃ |
| 504 | -(CH₂)₄CH=CH-CH₂CH₃ | -CH₂CH(CH₃)CH₂CH₃ |
| 505 | -(CH₂)₅CH=CH-CH₃ | -CH₂CH(CH₃)CH₂CH₃ |
| 506 | -(CH₂)₆CH=CH₂ | -CH₂CH(CH₃)CH₂CH₃ |
| 507 | -CH₃ | -(CH₂)₂CH(CH₃)₂ |
| 508 | -CH₂CH₃ | -(CH₂)₂CH(CH₃)₂ |
| 509 | -CH₂CH₂CH₃ | -(CH₂)₂CH(CH₃)₂ |
| 510 | -CH(CH₃)₂ | -(CH₂)₂CH(CH₃)₂ |
| 511 | -CH₂CH₂CH₂CH₃ | -(CH₂)₂CH(CH₃)₂ |
| 512 | -CH(CH₃)CH₂CH₃ | -(CH₂)₂CH(CH₃)₂ |
| 513 | -CH₂CH(CH₃)₂ | -(CH₂)₂CH(CH₃)₂ |
| 514 | -C(CH₃)₃ | -(CH₂)₂CH(CH₃)₂ |
| 515 | -CH₂(CH₂)₃CH₃ | -(CH₂)₂CH(CH₃)₂ |
| 516 | -CH(CH₃)(CH₂)₂CH₃ | -(CH₂)₂CH(CH₃)₂ |
| 517 | -CH₂CH(CH₃)CH₂CH₃ | -(CH₂)₂CH(CH₃)₂ |
| 518 | -(CH₂)₂CH(CH₃)₂ | -(CH₂)₂CH(CH₃)₂ |
| 519 | -CH₂(CH₂)₄CH₃ | -(CH₂)₂CH(CH₃)₂ |
| 520 | -CH₂(CH₂)₅CH₃ | -(CH₂)₂CH(CH₃)₂ |
| 521 | -CH₂(CH₂)₆CH₃ | -(CH₂)₂CH(CH₃)₂ |
| 522 | -CH=CH₂ | -(CH₂)₂CH(CH₃)₂ |
| 523 | -CH=CHCH₃ | -(CH₂)₂CH(CH₃)₂ |
| 524 | -CH=C(CH₃)₂ | -(CH₂)₂CH(CH₃)₂ |
| 525 | -C(CH₃)=CH₂ | -(CH₂)₂CH(CH₃)₂ |
| 526 | -C(CH₃)=CHCH₃ | -(CH₂)₂CH(CH₃)₂ |
| 527 | -C(CH₃)=C(CH₃)₂ | -(CH₂)₂CH(CH₃)₂ |
| 528 | -CH=CH-CH₂CH₃ | -(CH₂)₂CH(CH₃)₂ |
| 529 | -CH₂CH=CH-CH₃ | -(CH₂)₂CH(CH₃)₂ |
| 530 | -(CH₂)₂CH=CH₂ | -(CH₂)₂CH(CH₃)₂ |
| 531 | -CH=CH-(CH₂)₂CH₃ | -(CH₂)₂CH(CH₃)₂ |
| 532 | -CH₂CH=CH-CH₂CH₃ | -(CH₂)₂CH(CH₃)₂ |
| 533 | -(CH₂)₂CH=CH-CH₃ | -(CH₂)₂CH(CH₃)₂ |
| 534 | -(CH₂)₃CH=CH₂ | -(CH₂)₂CH(CH₃)₂ |
| 535 | -CH=CH-(CH₂)₃CH₃ | -(CH₂)₂CH(CH₃)₂ |
| 536 | -CH₂CH=CH-(CH₂)₂CH₃ | -(CH₂)₂CH(CH₃)₂ |
| 537 | -(CH₂)₂CH=CH-CH₂CH₃ | -(CH₂)₂CH(CH₃)₂ |
| 538 | -(CH₂)₃CH=CH-CH₃ | -(CH₂)₂CH(CH₃)₂ |
| 539 | -(CH₂)₄CH=CH₂ | -(CH₂)₂CH(CH₃)₂ |
| 540 | -CH=CH-(CH₂)₃CH₃ | -(CH₂)₂CH(CH₃)₂ |
| 541 | -CH₂CH=CH-(CH₂)₃CH₃ | -(CH₂)₂CH(CH₃)₂ |
| 542 | -(CH₂)₂CH=CH-(CH₂)₂CH₃ | -(CH₂)₂CH(CH₃)₂ |
| 543 | -(CH₂)₃CH=CH-CH₂CH₃ | -(CH₂)₂CH(CH₃)₂ |
| 544 | -(CH₂)₄CH=CH-CH₃ | -(CH₂)₂CH(CH₃)₂ |
| 545 | -(CH₂)₅CH=CH₂ | -(CH₂)₂CH(CH₃)₂ |
| 546 | -CH=CH-(CH₂)₅CH₃ | -(CH₂)₂CH(CH₃)₂ |
| 547 | -CH₂CH=CH-(CH₂)₄CH₃ | -(CH₂)₂CH(CH₃)₂ |
| 548 | -(CH₂)₂CH=CH-(CH₂)₃CH₃ | -(CH₂)₂CH(CH₃)₂ |
| 549 | -(CH₂)₃CH=CH-(CH₂)₂CH₃ | -(CH₂)₂CH(CH₃)₂ |
| 550 | -(CH₂)₄CH=CH-CH₂CH₃ | -(CH₂)₂CH(CH₃)₂ |
| 551 | -(CH₂)₅CH=CH-CH₃ | -(CH₂)₂CH(CH₃)₂ |
| 552 | -(CH₂)₆CH=CH₂ | -(CH₂)₂CH(CH₃)₂ |
| 553 | -CH₃ | -CH₂(CH₂)₄CH₃ |
| 554 | -CH₂CH₃ | -CH₂(CH₂)₄CH₃ |
| 555 | -CH₂CH₂CH₃ | -CH₂(CH₂)₄CH₃ |
| 556 | -CH(CH₃)₂ | -CH₂(CH₂)₄CH₃ |
| 557 | -CH₂CH₂CH₂CH₃ | -CH₂(CH₂)₄CH₃ |
| 558 | -CH(CH₃)CH₂CH₃ | -CH₂(CH₂)₄CH₃ |
| 559 | -CH₂CH(CH₃)₂ | -CH₂(CH₂)₄CH₃ |
| 560 | -C(CH₃)₃ | -CH₂(CH₂)₄CH₃ |
| 561 | -CH₂(CH₂)₃CH₃ | -CH₂(CH₂)₄CH₃ |
| 562 | -CH(CH₃)(CH₂)₂CH₃ | -CH₂(CH₂)₄CH₃ |
| 563 | -CH₂CH(CH₃)CH₂CH₃ | -CH₂(CH₂)₄CH₃ |
| 564 | -(CH₂)₂CH(CH₃)₂ | -CH₂(CH₂)₄CH₃ |
| 565 | -CH₂(CH₂)₄CH₃ | -CH₂(CH₂)₄CH₃ |
| 566 | -CH₂(CH₂)₅CH₃ | -CH₂(CH₂)₄CH₃ |
| 567 | -CH₂(CH₂)₆CH₃ | -CH₂(CH₂)₄CH₃ |
| 568 | -CH=CH₂ | -CH₂(CH₂)₄CH₃ |
| 569 | -CH=CHCH₃ | -CH₂(CH₂)₄CH₃ |
| 570 | -CH=C(CH₃)₂ | -CH₂(CH₂)₄CH₃ |
| 571 | -C(CH₃)=CH₂ | -CH₂(CH₂)₄CH₃ |
| 572 | -C(CH₃)=CHCH₃ | -CH₂(CH₂)₄CH₃ |
| 573 | -C(CH₃)=C(CH₃)₂ | -CH₂(CH₂)₄CH₃ |
| 574 | -CH=CH-CH₂CH₃ | -CH₂(CH₂)₄CH₃ |
| 575 | -CH₂CH=CH-CH₃ | -CH₂(CH₂)₄CH₃ |
| 576 | -(CH₂)₂CH₌CH₂ | -CH₂(CH₂)₄CH₃ |
| 577 | -CH=CH-(CH₂)₂CH₃ | -CH₂(CH₂)₄CH₃ |
| 578 | -CH₂CH=CH-CH₂CH₃ | -CH₂(CH₂)₄CH₃ |
| 579 | -(CH₂)₂CH=CH-CH₃ | -CH₂(CH₂)₄CH₃ |
| 580 | -(CH₂)₃CH=CH₂ | -CH₂(CH₂)₄CH₃ |
| 581 | -CH=CH-(CH₂)₃CH₃ | -CH₂(CH₂)₄CH₃ |
| 582 | -CH₂CH=CH-(CH₂)₂CH₃ | -CH₂(CH₂)₄CH₃ |
| 583 | -(CH₂)₂CH=CH-CH₂CH₃ | -CH₂(CH₂)₄CH₃ |
| 584 | -(CH₂)₃CH=CH-CH₃ | -CH₂(CH₂)₄CH₃ |
| 585 | -(CH₂)₄CH=CH₂ | -CH₂(CH₂)₄CH₃ |
| 586 | -CH=CH-(CH₂)₃CH₃ | -CH₂(CH₂)₄CH₃ |
| 587 | -CH₂CH=CH-(CH₂)₃CH₃ | -CH₂(CH₂)₄CH₃ |
| 588 | -(CH₂)₂CH=CH-(CH₂)₂CH₃ | -CH₂(CH₂)₄CH₃ |
| 589 | -(CH₂)₃CH=CH-CH₂CH₃ | -CH₂(CH₂)₄CH₃ |
| 590 | -(CH₂)₄CH=CH-CH₃ | -CH₂(CH₂)₄CH₃ |
| 591 | -(CH₂)₅CH=CH₂ | -CH₂(CH₂)₄CH₃ |
| 592 | -CH=CH-(CH₂)₅CH₃ | -CH₂(CH₂)₄CH₃ |
| 593 | -CH₂CH=CH-(CH₂)₄CH₃ | -CH₂(CH₂)₄CH₃ |
| 594 | -(CH₂)₂CH=CH-(CH₂)₃CH₃ | -CH₂(CH₂)₄CH₃ |
| 595 | -(CH₂)₃CH=CH-(CH₂)₂CH₃ | -CH₂(CH₂)₄CH₃ |
| 596 | -(CH₂)₄CH=CH-CH₂CH₃ | -CH₂(CH₂)₄CH₃ |
| 597 | -(CH₂)₅CH=CH-CH₃ | -CH₂(CH₂)₄CH₃ |
| 598 | -(CH₂)₆CH=CH₂ | -CH₂(CH₂)₄CH₃ |
| 599 | -CH₃ | -CH₂(CH₂)₅CH₃ |
| 600 | -CH₂CH₃ | -CH₂(CH₂)₅CH₃ |
| 601 | -CH₂CH₂CH₃ | -CH₂(CH₂)₅CH₃ |
| 602 | -CH(CH₃)₂ | -CH₂(CH₂)₅CH₃ |
| 603 | -CH₂CH₂CH₂CH₃ | -CH₂(CH₂)₅CH₃ |
| 604 | -CH(CH₃)CH₂CH₃ | -CH₂(CH₂)₅CH₃ |
| 605 | -CH₂CH(CH₃)₂ | -CH₂(CH₂)₅CH₃ |
| 606 | -C(CH₃)₃ | -CH₂(CH₂)₅CH₃ |
| 607 | -CH₂(CH₂)₃CH₃ | -CH₂(CH₂)₅CH₃ |
| 608 | -CH(CH₃)(CH₂)₂CH₃ | -CH₂(CH₂)₅CH₃ |
| 609 | -CH₂CH(CH₃)CH₂CH₃ | -CH₂(CH₂)₅CH₃ |
| 610 | -(CH₂)₂CH(CH₃)₂ | -CH₂(CH₂)₅CH₃ |
| 611 | -CH₂(CH₂)₄CH₃ | -CH₂(CH₂)₅CH₃ |
| 612 | -CH₂(CH₂)₅CH₃ | -CH₂(CH₂)₅CH₃ |
| 613 | -CH₂(CH₂)₆CH₃ | -CH₂(CH₂)₅CH₃ |
| 614 | -CH=CH₂ | -CH₂(CH₂)₅CH₃ |
| 615 | -CH=CHCH₃ | -CH₂(CH₂)₅CH₃ |
| 616 | -CH=C(CH₃)₂ | -CH₂(CH₂)₅CH₃ |
| 617 | -C(CH₃)=CH₂ | -CH₂(CH₂)₅CH₃ |
| 618 | -C(CH₃)=CHCH₃ | -CH₂(CH₂)ₛCH₃ |
| 619 | -C(CH₃)=C(CH₃)₂ | -CH₂(CH₂)₅CH₃ |
| 620 | -CH=CH-CH₂CH₃ | -CH₂(CH₂)₅CH₃ |
| 621 | -CH₂CH=CH-CH₃ | -CH₂(CH₂)₅CH₃ |
| 622 | -(CH₂)₂CH=CH₂ | -CH₂(CH₂)₅CH₃ |
| 623 | -CH=CH-(CH₂)₂CH₃ | -CH₂(CH₂)₅CH₃ |
| 624 | -CH₂CH=CH-CH₂CH₃ | -CH₂(CH₂)₅CH₃ |
| 625 | -(CH₂)₂CH=CH-CH₃ | -CH₂(CH₂)₅CH₃ |
| 626 | -(CH₂)₃CH=CH₂ | -CH₂(CH₂)₅CH₃ |
| 627 | -CH=CH-(CH₂)₃CH₃ | -CH₂(CH₂)₅CH₃ |
| 628 | -CH₂CH=CH-(CH₂)₂CH₃ | -CH₂(CH₂)₅CH₃ |
| 629 | -(CH₂)₂CH=CH-CH₂CH₃ | -CH₂(CH₂)₅CH₃ |
| 630 | -(CH₂)₃CH=CH-CH₃ | -CH₂(CH₂)₅CH₃ |
| 631 | -(CH₂)₄CH=CH₂ | -CH₂(CH₂)₅CH₃ |
| 632 | -CH=CH-(CH₂)₃CH₃ | -CH₂(CH₂)₅CH₃ |
| 633 | -CH₂CH=CH-(CH₂)₃CH₃ | -CH₂(CH₂)₅CH₃ |
| 634 | -(CH₂)₂CH=CH-(CH₂)₂CH₃ | -CH₂(CH₂)₅CH₃ |
| 635 | -(CH₂)₃CH=CH-CH₂CH₃ | -CH₂(CH₂)₅CH₃ |
| 636 | -(CH₂)₄CH=CH-CH₃ | -CH₂(CH₂)₅CH₃ |
| 637 | -(CH₂)₅CH=CH₂ | -CH₂(CH₂)₅CH₃ |
| 638 | -CH=CH-(CH₂)₅CH₃ | -CH₂(CH₂)₅CH₃ |
| 639 | -CH₂CH=CH-(CH₂)₄CH₃ | -CH₂(CH₂)₅CH₃ |
| 640 | -(CH₂)₂CH=CH-(CH₂)₃CH₃ | -CH₂(CH₂)₅CH₃ |
| 641 | -(CH₂)₃CH=CH-(CH₂)₂CH₃ | -CH₂(CH₂)₅CH₃ |
| 642 | -(CH₂)₄CH=CH-CH₂CH₃ | -CH₂(CH₂)₅CH₃ |
| 643 | -(CH₂)₅CH=CH-CH₃ | -CH₂(CH₂)₅CH₃ |
| 644 | -(CH₂)₆CH=CH₂ | -CH₂(CH₂)₅CH₃ |
| 645 | -CH₃ | -CH₂(CH₂)₆CH₃ |
| 646 | -CH₂CH₃ | -CH₂(CH₂)₆CH₃ |
| 647 | -CH₂CH₂CH₃ | -CH₂(CH₂)₆CH₃ |
| 648 | -CH(CH₃)₂ | -CH₂(CH₂)₆CH₃ |
| 649 | -CH₂CH₂CH₂CH₃ | -CH₂(CH₂)₆CH₃ |
| 650 | -CH(CH₃)CH₂CH₃ | -CH₂(CH₂)₆CH₃ |
| 651 | -CH₂CH(CH₃)₂ | -CH₂(CH₂)₆CH₃ |
| 652 | -C(CH₃)₃ | -CH₂(CH₂)₆CH₃ |
| 653 | -CH₂(CH₂)₃CH₃ | -CH₂(CH₂)₆CH₃ |
| 654 | -CH(CH₃)(CH₂)₂CH₃ | -CH₂(CH₂)₆CH₃ |
| 655 | -CH₂CH(CH₃)CH₂CH₃ | -CH₂(CH₂)₆CH₃ |
| 656 | -(CH₂)₂CH(CH₃)₂ | -CH₂(CH₂)₆CH₃ |
| 657 | -CH₂(CH₂)₄CH₃ | -CH₂(CH₂)₆CH₃ |
| 658 | -CH₂(CH₂)₅CH₃ | -CH₂(CH₂)₆CH₃ |
| 59 | -CH₂(CH₂)₆CH₃ | -CH₂(CH₂)₆CH₃ |
| 660 | -CH=CH₂ | -CH₂(CH₂)₆CH₃ |
| 661 | -CH=CHCH₃ | -CH₂(CH₂)₆CH₃ |
| 662 | -CH=C(CH₃)₂ | -CH₂(CH₂)₆CH₃ |
| 663 | -C(CH₃)=CH₂ | -CH₂(CH₂)₆CH₃ |
| 664 | -C(CH₃)=CHCH₃ | -CH₂(CH₂)₆CH₃ |
| 665 | -C(CH₃)=C(CH₃)₂ | -CH₂(CH₂)₆CH₃ |
| 666 | -CH=CH-CH₂CH₃ | -CH₂(CH₂)₆CH₃ |
| 667 | -CH₂CH=CH-CH₃ | -CH₂(CH₂)₆CH₃ |
| 668 | -(CH₂)₂CH=CH₂ | -CH₂(CH₂)₆CH₃ |
| 669 | -CH=CH-(CH₂)₂CH₃ | -CH₂(CH₂)₆CH₃ |
| 670 | -CH₂CH=CH-CH₂CH₃ | -CH₂(CH₂)₆CH₃ |
| 671 | -(CH₂)₂CH=CH-CH₃ | -CH₂(CH₂)₆CH₃ |
| 672 | -(CH₂)₃CH=CH₂ | -CH₂(CH₂)₆CH₃ |
| 673 | -CH=CH-(CH₂)₃CH₃ | -CH₂(CH₂)₆CH₃ |
| 674 | -CH₂CH=CH-(CH₂)₂CH₃ | -CH₂(CH₂)₆CH₃ |
| 675 | -(CH₂)₂CH=CH-CH₂CH₃ | -CH₂(CH₂)₆CH₃ |
| 676 | -(CH₂)₃CH=CH-CH₃ | -CH₂(CH₂)₆CH₃ |
| 677 | -(CH₂)₄CH=CH₂ | -CH₂(CH₂)₆CH₃ |
| 678 | -CH=CH-(CH₂)₃CH₃ | -CH₂(CH₂)₆CH₃ |
| 679 | -CH₂CH=CH-(CH₂)₃CH₃ | -CH₂(CH₂)₆CH₃ |
| 680 | -(CH₂)₂CH=CH-(CH₂)₂CH₃ | -CH₂(CH₂)₆CH₃ |
| 681 | -(CH₂)₃CH=CH-CH₂CH₃ | -CH₂(CH₂)₆CH₃ |
| 682 | -(CH₂)₄CH=CH-CH₃ | -CH₂(CH₂)₆CH₃ |
| 683 | -(CH₂)₅CH=CH₂ | -CH₂(CH₂)₆CH₃ |
| 684 | -CH=CH-(CH₂)₅CH₃ | -CH₂(CH₂)₆CH₃ |
| 685 | -CH₂CH=CH-(CH₂)₄CH₃ | -CH₂(CH₂)₆CH₃ |
| 686 | -(CH₂)₂CH=CH-(CH₂)₃CH₃ | -CH₂(CH₂)₆CH₃ |
| 687 | -(CH₂)₃CH=CH-(CH₂)₂CH₃ | -CH₂(CH₂)₆CH₃ |
| 688 | -(CH₂)₄CH=CH-CH₂CH₃ | -CH₂(CH₂)₆CH₃ |
| 689 | -(CH₂)₅CH=CH-CH₃ | -CH₂(CH₂)₆CH₃ |
| 690 | -(CH₂)₆CH=CH₂ | -CH₂(CH₂)₆CH₃ |
| 691 | -CH₃ | -CH=CH₂ |
| 692 | -CH₂CH₃ | -CH=CH₂ |
| 693 | -CH₂CH₂CH₃ | -CH=CH₂ |
| 694 | -CH(CH₃)₂ | -CH=CH₂ |
| 695 | -CH₂CH₂CH₂CH₃ | -CH=CH₂ |
| 696 | -CH(CH₃)CH₂CH₃ | -CH=CH₂ |
| 697 | -CH₂CH(CH₃)₂ | -CH=CH₂ |
| 698 | -C(CH₃)₃ | -CH=CH₂ |
| 699 | -CH₂(CH₂)₃CH₃ | -CH=CH₂ |
| 700 | -CH(CH₃)(CH₂)₂CH₃ | -CH=CH₂ |
| 701 | -CH₂CH(CH₃)CH₂CH₃ | -CH=CH₂ |
| 702 | -(CH₂)₂CH(CH₃)₂ | -CH=CH₂ |
| 703 | -CH₂(CH₂)₄CH₃ | -CH=CH₂ |
| 704 | -CH₂(CH₂)₅CH₃ | -CH=CH₂ |
| 705 | -CH₂(CH₂)₆CH₃ | -CH=CH₂ |
| 706 | -CH=CH₂ | -CH=CH₂ |
| 707 | -CH=CHCH₃ | -CH=CH₂ |
| 708 | -CH=C(CH₃)₂ | -CH=CH₂ |
| 709 | -C(CH₃)=CH₂ | -CH=CH₂ |
| 710 | -C(CH₃)=CHCH₃ | -CH=CH₂ |
| 711 | -C(CH₃)=C(CH₃)₂ | -CH=CH₂ |
| 712 | -CH=CH-CH₂CH₃ | -CH=CH₂ |
| 713 | -CH₂CH=CH-CH₃ | -CH=CH₂ |
| 714 | -(CH₂)₂CH=CH₂ | -CH=CH₂ |
| 715 | -CH=CH-(CH₂)₂CH₃ | -CH=CH₂ |
| 716 | -CH₂CH=CH-CH₂CH₃ | -CH=CH₂ |
| 717 | -(CH₂)₂CH=CH-CH₃ | -CH=CH₂ |
| 718 | -(CH₂)₃CH=CH₂ | -CH=CH₂ |
| 719 | -CH=CH-(CH₂)₃CH₃ | -CH=CH₂ |
| 720 | -CH₂CH=CH-(CH₂)₂CH₃ | -CH=CH₂ |
| 721 | -(CH₂)₂CH=CH-CH₂CH₃ | -CH=CH₂ |
| 722 | -(CH₂)₃CH=CH-CH₃ | -CH=CH₂ |
| 723 | -(CH₂)₄CH=CH₂ | -CH=CH₂ |
| 724 | -CH=CH-(CH₂)₃CH₃ | -CH=CH₂ |
| 725 | -CH₂CH=CH-(CH₂)₃CH₃ | -CH=CH₂ |
| 726 | -(CH₂)₂CH=CH-(CH₂)₂CH₃ | -CH=CH₂ |
| 727 | -(CH₂)₃CH=CH-CH₂CH₃ | -CH=CH₂ |
| 728 | -(CH₂)₄CH=CH-CH₃ | -CH=CH₂ |
| 729 | -(CH₂)₅CH=CH₂ | -CH=CH₂ |
| 730 | -CH=CH-(CH₂)₅CH₃ | -CH=CH₂ |
| 731 | -CH₂CH=CH-(CH₂)₄CH₃ | -CH=CH₂ |
| 732 | -(CH₂)₂CH=CH-(CH₂)₃CH₃ | -CH=CH₂ |
| 733 | -(CH₂)₃CH=CH-(CH₂)₂CH₃ | -CH=CH₂ |
| 734 | -(CH₂)₄CH=CH-CH₂CH₃ | -CH=CH₂ |
| 735 | -(CH₂)₅CH=CH-CH₃ | -CH=CH₂ |
| 736 | -(CH₂)₆CH=CH₂ | -CH=CH₂ |
| 737 | -CH₃ | -C(CH₃)=CH₂ |
| 738 | -CH₂CH₃ | -C(CH₃)=CH₂ |
| 739 | -CH₂CH₂CH₃ | -C(CH₃)=CH₂ |
| 740 | -CH(CH₃)₂ | -C(CH₃)=CH₂ |
| 741 | -CH₂CH₂CH₂CH₃ | -C(CH₃)=CH₂ |
| 742 | -CH(CH₃)CH₂CH₃ | -C(CH₃)=CH₂ |
| 743 | -CH₂CH(CH₃)₂ | -C(CH₃)=CH₂ |
| 744 | -C(CH₃)₃ | -C(CH₃)=CH₂ |
| 745 | -CH₂(CH₂)₃CH₃ | -C(CH₃)=CH₂ |
| 746 | -CH(CH₃)(CH₂)₂CH₃ | -C(CH₃)=CH₂ |
| 747 | -CH₂CH(CH₃)CH₂CH₃ | -C(CH₃)=CH₂ |
| 748 | -(CH₂)₂CH(CH₃)₂ | -C(CH₃)=CH₂ |
| 749 | -CH₂(CH₂)₄CH₃ | -C(CH₃)=CH₂ |
| 750 | -CH₂(CH₂)₅CH₃ | -C(CH₃)=CH₂ |
| 751 | -CH₂(CH₂)₆CH₃ | -C(CH₃)=CH₂ |
| 752 | -CH=CH₂ | -C(CH₃)=CH₂ |
| 753 | -CH=CHCH₃ | -C(CH₃)=CH₂ |
| 754 | -CH=C(CH₃)₂ | -C(CH₃)=CH₂ |
| 755 | -C(CH₃)=CH₂ | -C(CH₃)=CH₂ |
| 756 | -C(CH₃)=CHCH₃ | -C(CH₃)=CH₂ |
| 757 | -C(CH₃)=C(CH₃)₂ | -C(CH₃)=CH₂ |
| 758 | -CH=CH-CH₂CH₃ | -C(CH₃)=CH₂ |
| 759 | -CH₂CH=CH-CH₃ | -C(CH₃)=CH₂ |
| 760 | -(CH₂)₂CH=CH₂ | -C(CH₃)=CH₂ |
| 761 | -CH=CH-(CH₂)₂CH₃ | -C(CH₃)=CH₂ |
| 762 | -CH₂CH=CH-CH₂CH₃ | -C(CH₃)=CH₂ |
| 763 | -(CH₂)₂CH=CH-CH₃ | -C(CH₃)=CH₂ |
| 764 | -(CH₂)₃CH=CH₂ | -C(CH₃)=CH₂ |
| 765 | -CH=CH-(CH₂)₃CH₃ | -C(CH₃)=CH₂ |
| 766 | -CH₂CH=CH-(CH₂)₂CH₃ | -C(CH₃)=CH₂ |
| 767 | -(CH₂)₂CH=CH-CH₂CH₃ | -C(CH₃)=CH₂ |
| 768 | -(CH₂)₃CH=CH-CH₃ | -C(CH₃)=CH₂ |
| 769 | -(CH₂)₄CH=CH₂ | -C(CH₃)=CH₂ |
| 770 | -CH=CH-(CH₂)₃CH₃ | -C(CH₃)=CH₂ |
| 771 | -CH₂CH=CH-(CH₂)₃CH₃ | -C(CH₃)=CH₂ |
| 772 | -(CH₂)₂CH=CH-(CH₂)₂CH₃ | -C(CH₃)=CH₂ |
| 773 | -(CH₂)₃CH=CH-CH₂CH₃ | -C(CH₃)=CH₂ |
| 774 | -(CH₂)₄CH=CH-CH₃ | -C(CH₃)=CH₂ |
| 775 | -(CH₂)₅CH=CH₂ | -C(CH₃)=CH₂ |
| 776 | -CH=CH-(CH₂)₅CH₃ | -C(CH₃)=CH₂ |
| 777 | -CH₂CH=CH-(CH₂)₄CH₃ | -C(CH₃)=CH₂ |
| 778 | -(CH₂)₂CH=CH-(CH₂)₃CH₃ | -C(CH₃)=CH₂ |
| 779 | -(CH₂)₃CH=CH-(CH₂)₂CH₃ | -C(CH₃)=CH₂ |
| 780 | -(CH₂)₄CH=CH-CH₂CH₃ | -C(CH₃)=CH₂ |
| 781 | -(CH₂)₅CH=CH-CH₃ | -C(CH₃)=CH₂ |
| 782 | -(CH₂)₆CH=CH₂ | -C(CH₃)=CH₂ |
| 783 | -CH₃ | -O-CH₃ |
| 784 | -CH₂CH₃ | -O-CH₃ |
| 785 | -CH₂CH₂CH₃ | -O-CH₃ |
| 786 | -CH(CH₃)₂ | -O-CH₃ |
| 787 | -CH₂CH₂CH₂CH₃ | -O-CH₃ |
| 788 | -CH(CH₃)CH₂CH₃ | -O-CH₃ |
| 789 | -CH₂CH(CH₃)₂ | -O-CH₃ |
| 790 | -C(CH₃)₃ | -O-CH₃ |
| 791 | -CH₂(CH₂)₃CH₃ | -O-CH₃ |
| 792 | -CH(CH₃)(CH₂)₂CH₃ | -O-CH₃ |
| 793 | -CH₂CH(CH₃)CH₂CH₃ | -O-CH₃ |
| 794 | -(CH₂)₂CH(CH₃)₂ | -O-CH₃ |
| 795 | -CH₂(CH₂)₄CH₃ | -O-CH₃ |
| 796 | -CH₂(CH₂)₅CH₃ | -O-CH₃ |
| 797 | -CH₂(CH₂)₆CH₃ | -O-CH₃ |
| 798 | -CH=CH₂ | -O-CH₃ |
| 799 | -CH=CHCH₃ | -O-CH₃ |
| 800 | -CH=C(CH₃)₂ | -O-CH₃ |
| 801 | -C(CH₃)=CH₂ | -O-CH₃ |
| 802 | -C(CH₃)=CHCH₃ | -O-CH₃ |
| 803 | -C(CH₃)=C(CH₃)₂ | -O-CH₃ |
| 804 | -CH=CH-CH₂CH₃ | -O-CH₃ |
| 805 | -CH₂CH=CH-CH₃ | -O-CH₃ |
| 806 | -(CH₂)₂CH=CH₂ | -O-CH₃ |
| 807 | -CH=CH-(CH₂)₂CH₃ | -O-CH₃ |
| 808 | -CH₂CH=CH-CH₂CH₃ | -O-CH₃ |
| 809 | -(CH₂)₂CH=CH-CH₃ | -O-CH₃ |
| 810 | -(CH₂)₃CH=CH₂ | -O-CH₃ |
| 811 | -CH=CH-(CH₂)₃CH₃ | -O-CH₃ |
| 812 | -CH₂CH=CH-(CH₂)₂CH₃ | -O-CH₃ |
| 813 | -(CH₂)₂CH=CH-CH₂CH₃ | -O-CH₃ |
| 814 | -(CH₂)₃CH=CH-CH₃ | -O-CH₃ |
| 815 | -(CH₂)₄CH=CH₂ | -O-CH₃ |
| 816 | -CH=CH-(CH₂)₃CH₃ | -O-CH₃ |
| 817 | -CH₂CH=CH-(CH₂)₃CH₃ | -O-CH₃ |
| 818 | -(CH₂)₂CH=CH-(CH₂)₂CH₃ | -O-CH₃ |
| 819 | -(CH₂)₃CH=CH-CH₂CH₃ | -O-CH₃ |
| 820 | -(CH₂)₄CH=CH-CH₃ | -O-CH₃ |
| 821 | -(CH₂)₅CH=CH₂ | -O-CH₃ |
| 822 | -CH=CH-(CH₂)₅CH₃ | -O-CH₃ |
| 823 | -CH₂CH=CH-(CH₂)₄CH₃ | -O-CH₃ |
| 824 | -(CH₂)₂CH=CH-(CH₂)₃CH₃ | -O-CH₃ |
| 825 | -(CH₂)₃CH=CH-(CH₂)₂CH₃ | -O-CH₃ |
| 826 | -(CH₂)₄CH=CH-CH₂CH₃ | -O-CH₃ |
| 827 | -(CH₂)₅CH=CH-CH₃ | -O-CH₃ |
| 828 | -(CH₂)₆CH=CH₂ | -O-CH₃ |
| 829 | -CH₃ | -O-CH₂CH₃ |
| 830 | -CH₂CH₃ | -O-CH₂CH₃ |
| 831 | -CH₂CH₂CH₃ | -O-CH₂CH₃ |
| 832 | -CH(CH₃)₂ | -O-CH₂CH₃ |
| 833 | -CH₂CH₂CH₂CH₃ | -O-CH₂CH₃ |
| 834 | -CH(CH₃)CH₂CH₃ | -O-CH₂CH₃ |
| 835 | -CH₂CH(CH₃)₂ | -O-CH₂CH₃ |
| 836 | -C(CH₃)₃ | -O-CH₂CH₃ |
| 837 | -CH₂(CH₂)₃CH₃ | -O-CH₂CH₃ |
| 838 | -CH(CH₃)(CH₂)₂CH₃ | -O-CH₂CH₃ |
| 839 | -CH₂CH(CH₃)CH₂CH₃ | -O-CH₂CH₃ |
| 840 | -(CH₂)₂CH(CH₃)₂ | -O-CH₂CH₃ |
| 841 | -CH₂(CH₂)₄CH₃ | -O-CH₂CH₃ |
| 842 | -CH₂(CH₂)₅CH₃ | -O-CH₂CH₃ |
| 843 | -CH₂(CH₂)₆CH₃ | -O-CH₂CH₃ |
| 844 | -CH=CH₂ | -O-CH₂CH₃ |
| 845 | -CH=CHCH₃ | -O-CH₂CH₃ |
| 846 | -CH=C(CH₃)₂ | -O-CH₂CH₃ |
| 847 | -C(CH₃)=CH₂ | -O-CH₂CH₃ |
| 848 | -C(CH₃)=CHCH₃ | -O-CH₂CH₃ |
| 849 | -C(CH₃)=C(CH₃)₂ | -O-CH₂CH₃ |
| 850 | -CH=CH-CH₂CH₃ | -O-CH₂CH₃ |
| 851 | -CH₂CH=CH-CH₃ | -O-CH₂CH₃ |
| 852 | -(CH₂)₂CH=CH₂ | -O-CH₂CH₃ |
| 853 | -CH=CH-(CH₂)₂CH₃ | -O-CH₂CH₃ |
| 854 | -CH₂CH=CH-CH₂CH₃ | -O-CH₂CH₃ |
| 855 | -(CH₂)₂CH=CH-CH₃ | -O-CH₂CH₃ |
| 856 | -(CH₂)₃CH=CH₂ | -O-CH₂CH₃ |
| 857 | -CH=CH-(CH₂)₃CH₃ | -O-CH₂CH₃ |
| 858 | -CH₂CH=CH-(CH₂)₂CH₃ | -O-CH₂CH₃ |
| 859 | -(CH₂)₂CH=CH-CH₂CH₃ | -O-CH₂CH₃ |
| 860 | -(CH₂)₃CH=CH-CH₃ | -O-CH₂CH₃ |
| 861 | -(CH₂)₄CH=CH₂ | -O-CH₂CH₃ |
| 862 | -CH=CH-(CH₂)₃CH₃ | -O-CH₂CH₃ |
| 863 | -CH₂CH=CH-(CH₂)₃CH₃ | -O-CH₂CH₃ |
| 864 | -(CH₂)₂CH=CH-(CH₂)₂CH₃ | -O-CH₂CH₃ |
| 865 | -(CH₂)₃CH=CH-CH₂CH₃ | -O-CH₂CH₃ |
| 866 | -(CH₂)₄CH=CH-CH₃ | -O-CH₂CH₃ |
| 867 | -(CH₂)₅CH=CH₂ | -O-CH₂CH₃ |
| 868 | -CH=CH-(CH₂)₅CH₃ | -O-CH₂CH₃ |
| 869 | -CH₂CH=CH-(CH₂)₄CH₃ | -O-CH₂CH₃ |
| 870 | -(CH₂)₂CH=CH-(CH₂)₃CH₃ | -O-CH₂CH₃ |
| 871 | -(CH₂)₃CH=CH-(CH₂)₂CH₃ | -O-CH₂CH₃ |
| 872 | -(CH₂)₄CH=CH-CH₂CH₃ | -O-CH₂CH₃ |
| 873 | -(CH₂)₅CH=CH-CH₃ | -O-CH₂CH₃ |
| 874 | -(CH₂)₆CH=CH₂ | -O-CH₂CH₃ |
| 875 | -CH₃ | -O-CH₂CH₂CH₃ |
| 876 | -CH₂CH₃ | -O-CH₂CH₂CH₃ |
| 877 | -CH₂CH₂CH₃ | -O-CH₂CH₂CH₃ |
| 878 | -CH(CH₃)₂ | -O-CH₂CH₂CH₃ |
| 879 | -CH₂CH₂CH₂CH₃ | -O-CH₂CH₂CH₃ |
| 880 | -CH(CH₃)CH₂CH₃ | -O-CH₂CH₂CH₃ |
| 881 | -CH₂CH(CH₃)₂ | -O-CH₂CH₂CH₃ |
| 882 | -C(CH₃)₃ | -O-CH₂CH₂CH₃ |
| 883 | -CH₂(CH₂)₃CH₃ | -O-CH₂CH₂CH₃ |
| 884 | -CH(CH₃)(CH₂)₂CH₃ | -O-CH₂CH₂CH₃ |
| 885 | -CH₂CH(CH₃)CH₂CH₃ | -O-CH₂CH₂CH₃ |
| 886 | -(CH₂)₂CH(CH₃)₂ | -O-CH₂CH₂CH₃ |
| 887 | -CH₂(CH₂)₄CH₃ | -O-CH₂CH₂CH₃ |
| 888 | -CH₂(CH₂)₅CH₃ | -O-CH₂CH₂CH₃ |
| 889 | -CH₂(CH₂)₆CH₃ | -O-CH₂CH₂CH₃ |
| 890 | -CH=CH₂ | -O-CH₂CH₂CH₃ |
| 891 | -CH=CHCH₃ | -O-CH₂CH₂CH₃ |
| 892 | -CH=C(CH₃)₂ | -O-CH₂CH₂CH₃ |
| 893 | -C(CH₃)=CH₂ | -O-CH₂CH₂CH₃ |
| 894 | -C(CH₃)=CHCH₃ | -O-CH₂CH₂CH₃ |
| 895 | -C(CH₃)=C(CH₃)₂ | -O-CH₂CH₂CH₃ |
| 896 | -CH=CH-CH₂CH₃ | -O-CH₂CH₂CH₃ |
| 897 | -CH₂CH=CH-CH₃ | -O-CH₂CH₂CH₃ |
| 898 | -(CH₂)₂CH=CH₂ | -O-CH₂CH₂CH₃ |
| 899 | -CH=CH-(CH₂)₂CH₃ | -O-CH₂CH₂CH₃ |
| 900 | -CH₂CH=CH-CH₂CH₃ | -O-CH₂CH₂CH₃ |
| 901 | -(CH₂)₂CH=CH-CH₃ | -O-CH₂CH₂CH₃ |
| 902 | -(CH₂)₃CH=CH₂ | -O-CH₂CH₂CH₃ |
| 903 | -CH=CH-(CH₂)₃CH₃ | -O-CH₂CH₂CH₃ |
| 904 | -CH₂CH=CH-(CH₂)₂CH₃ | -O-CH₂CH₂CH₃ |
| 905 | -(CH₂)₂CH=CH-CH₂CH₃ | -O-CH₂CH₂CH₃ |
| 906 | -(CH₂)₃CH=CH-CH₃ | -O-CH₂CH₂CH₃ |
| 907 | -(CH₂)₄CH=CH₂ | -O-CH₂CH₂CH₃ |
| 908 | -CH=CH-(CH₂)₃CH₃ | -O-CH₂CH₂CH₃ |
| 909 | -CH₂CH=CH-(CH₂)₃CH₃ | -O-CH₂CH₂CH₃ |
| 910 | -(CH₂)₂CH=CH-(CH₂)₂CH₃ | -O-CH₂CH₂CH₃ |
| 911 | -(CH₂)₃CH=CH-CH₂CH₃ | -O-CH₂CH₂CH₃ |
| 912 | -(CH₂)₄CH=CH-CH₃ | -O-CH₂CH₂CH₃ |
| 913 | -(CH₂)₅CH=CH₂ | -O-CH₂CH₂CH₃ |
| 914 | -CH=CH-(CH₂)₅CH₃ | -O-CH₂CH₂CH₃ |
| 915 | -CH₂CH=CH-(CH₂)₄CH₃ | -O-CH₂CH₂CH₃ |
| 916 | -(CH₂)₂CH=CH-(CH₂)₃CH₃ | -O-CH₂CH₂CH₃ |
| 917 | -(CH₂)₃CH=CH-(CH₂)₂CH₃ | -O-CH₂CH₂CH₃ |
| 918 | -(CH₂)₄CH=CH-CH₂CH₃ | -O-CH₂CH₂CH₃ |
| 919 | -(CH₂)₅CH=CH-CH₃ | -O-CH₂CH₂CH₃ |
| 920 | -(CH₂)₆CH=CH₂ | -O-CH₂CH₂CH₃ |
| 921 | -CH₃ | -O-CH(CH₃)₂ |
| 922 | -CH₂CH₃ | -O-CH(CH₃)₂ |
| 923 | -CH₂CH₂CH₃ | -O-CH(CH₃)₂ |
| 924 | -CH(CH₃)₂ | -O-CH(CH₃)₂ |
| 925 | -CH₂CH₂CH₂CH₃ | -O-CH(CH₃)₂ |
| 926 | -CH(CH₃)CH₂CH₃ | -O-CH(CH₃)₂ |
| 927 | -CH₂CH(CH₃)₂ | -O-CH(CH₃)₂ |
| 928 | -C(CH₃)₃ | -O-CH(CH₃)₂ |
| 929 | -CH₂(CH₂)₃CH₃ | -O-CH(CH₃)₂ |
| 930 | -CH(CH₃)(CH₂)₂CH₃ | -0-CH(CH₃)₂ |
| 931 | -CH₂CH(CH₃)CH₂CH₃ | -O-cH(CH₃)₂ |
| 932 | -(CH₂)₂CH(CH₃)₂ | -O-CH(CH₃)₂ |
| 933 | -CH₂(CH₂)₄CH₃ | -O-CH(CH₃)₂ |
| 934 | -CH₂(CH₂)₅CH₃ | -O-CH(CH₃)₂ |
| 935 | -CH₂(CH₂)₆CH₃ | -O-CH(CH₃)₂ |
| 936 | -CH=CH₂ | -O-CH(CH₃)₂ |
| 937 | -CH=CHCH₃ | -O-CH(CH₃)₂ |
| 938 | -CH=C(CH₃)₂ | -O-CH(CH₃)₂ |
| 939 | -C(CH₃)=CH₂ | -O-CH(CH₃)₂ |
| 940 | -C(CH₃)=CHCH₃ | -O-CH(CH₃)₂ |
| 941 | -C(CH₃)=C(CH₃)₂ | -O-CH(CH₃)₂ |
| 942 | -CH=CH-CH₂CH₃ | -O-CH(CH₃)₂ |
| 943 | -CH₂CH=CH-CH₃ | -O-CH(CH₃)₂ |
| 944 | -(CH₂)₂CH=CH₂ | -O-CH(CH₃)₂ |
| 945 | -CH=CH-(CH₂)₂CH₃ | -O-CH(CH₃)₂ |
| 946 | -CH₂CH=CH-CH₂CH₃ | -O-CH(CH₃)₂ |
| 947 | -(CH₂)₂CH=CH-CH₃ | -O-CH(CH₃)₂ |
| 948 | -(CH₂)₃CH=CH₂ | -O-CH(CH₃)₂ |
| 949 | -CH=CH-(CH₂)₃CH₃ | -O-CH(CH₃)₂ |
| 950 | -CH₂CH=CH-(CH₂)₂CH₃ | -O-CH(CH₃)₂ |
| 951 | -(CH₂)₂CH=CH-CH₂CH₃ | -O-CH(CH₃)₂ |
| 952 | -(CH₂)₃CH=CH-CH₃ | -O-CH(CH₃)₂ |
| 953 | -(CH₂)₄CH=CH₂ | -O-CH(CH₃)₂ |
| 954 | -CH=CH-(CH₂)₃CH₃ | -O-CH(CH₃)₂ |
| 955 | -CH₂CH=CH-(CH₂)₃CH₃ | -O-CH(CH₃)₂ |
| 956 | -(CH₂)₂CH=CH-(CH₂)₂CH₃ | -O-CH(CH₃)₂ |
| 957 | -(CH₂)₃CH=CH-CH₂CH₃ | -O-CH(CH₃)₂ |
| 958 | -(CH₂)₄CH=CH-CH₃ | -O-CH(CH₃)₂ |
| 959 | -(CH₂)₅CH=CH₂ | -O-CH(CH₃)₂ |
| 960 | -CH=CH-(CH₂)₅CH₃ | -O-CH(CH₃)₂ |
| 961 | -CH₂CH=CH-(CH₂)₄CH₃ | -O-CH(CH₃)₂ |
| 962 | -(CH₂)₂CH=CH-(CH₂)₃CH₃ | -O-CH(CH₃)₂ |
| 963 | -(CH₂)₃CH=CH-(CH₂)₂CH₃ | -O-CH(CH₃)₂ |
| 964 | -(CH₂)₄CH=CH-CH₂CH₃ | -O-CH(CH₃)₂ |
| 965 | -(CH₂)₅CH=CH-CH₃ | -O-CH(CH₃)₂ |
| 966 | -(CH₂)₆CH=CH₂ | -O-CH(CH₃)₂ |

Als weiterhin besonders geeignet haben sich im Rahmen der vorliegenden Erfindung Carvonacetale der Formel 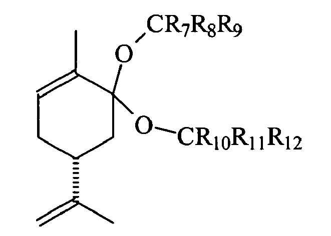 erwiesen, in der R7 bis R12 unabhängig voneinander ausgewählt sind aus -H, -CH₃, -CH₂CH₃, -CH₂CH₂CH₃, -CH(CH₃)₂, -CH₂ CH₂CH₂CH₃, -CH₂CH(CH₃)₂, -CH(CH₃)CH₂CH₃. C(CH₃)₃ oder R9 und R10 zusammen eine chemische Bindung oder eine Gruppe -(CR13R14)ₓ bedeuten, worin x für die Werte 1 oder 2 steht und R13 und R14 unabhängig voneinander ausgewählt sind aus -H, - CH₃, -CH₂CH₃, -CH₂CH₂CH₃, -CH(CH₃)₂, -CH₂ CH₂CH₂CH₃, -CH₂CH(CH₃)₂, -CH(CH₃)CH₂CH₃. - C(CH₃)₃

Einzelne Spezies, die der vorstehend genannten Formel genügen, sind hinsichtlich ihrer Reste R7 bis R12 in der nachstehenden Tabelle 3 aufgeführt. Diese sind erfindungsgemäß besonders bevorzugt.

**Tabelle 3: Bevorzugte Carvonacetale**

| **Nr.** | **R7** | **R8** | **R9** | **R10** | **R11** | **R12** |
|---|---|---|---|---|---|---|
| 1 | -H | -H | -H | -H | -H | -H |
| 2 | -CH₃ | -H | -H | -H | -H | -H |
| 3 | -CH₂CH₃ | -H | -H | -H | -H | -H |
| 4 | -CH₂CH₂CH₃ | -H | -H | -H | -H | -H |
| 5 | -CH(CH₃)₂ | -H | -H | -H | -H | -H |
| 6 | -CH₂CH₂CH₂CH₃ | -H | -H | -H | -H | -H |
| 7 | -CH(CH₃)CH₂CH₃ | -H | -H | -H | -H | -H |
| 8 | -CH₂CH(CH₃)₂ | -H | -H | -H | -H | -H |
| 9 | -C(CH₃)₃ | -H | -H | -H | -H | -H |
| 10 | -H | -CH₃ | -H | -H | -H | -H |
| 11 | -CH₃ | -CH₃ | -H | -H | -H | -H |
| 12 | -CH₂CH₃ | -CH₃ | -H | -H | -H | -H |
| 13 | -CH₂CH₂CH₃ | -CH₃ | -H | -H | -H | -H |
| 14 | -CH(CH₃)₂ | -CH₃ | -H | -H | -H | -H |
| 15 | -CH₂CH₂CH₂CH₃ | -CH₃ | -H | -H | -H | -H |
| 16 | -CH(CH₃)CH₂CH₃ | -CH₃ | -H | -H | -H | -H |
| 17 | -CH₂CH(CH₃)₂ | -CH₃ | -H | -H | -H | -H |
| 18 | -C(CH₃)₃ | -CH₃ | -H | -H | -H | -H |
| 19 | -H | -CH₃ | -CH₃ | -H | -H | -H |
| 20 | -CH₃ | -CH₃ | -CH₃ | -H | -H | -H |
| 21 | -CH₂CH₃ | -CH₃ | -CH₃ | -H | -H | -H |
| 22 | -CH₂CH₂CH₃ | -CH₃ | -CH₃ | -H | -H | -H |
| 23 | -CH(CH₃)₂ | -CH₃ | -CH₃ | -H | -H | -H |
| 24 | -CH₂CH₂CH₂CH₃ | -CH₃ | -CH₃ | -H | -H | -H |
| 25 | -CH(CH₃)CH₂CH₃ | -CH₃ | -CH₃ | -H | -H | -H |
| 26 | -CH₂CH(CH₃)₂ | -CH₃ | -CH₃ | -H | -H | -H |
| 27 | -C(CH₃)₃ | -CH₃ | -CH₃ | -H | -H | -H |
| 28 | -H | -CH₂CH₃ | -H | -H | -H | -H |
| 29 | -CH₃ | -CH₂CH₃ | -H | -H | -H | -H |
| 30 | -CH₂CH₃ | -CH₂CH₃ | -H | -H | -H | -H |
| 31 | -CH₂CH₂CH₃ | -CH₂CH₃ | -H | -H | -H | -H |
| 32 | -CH(CH₃)₂ | -CH₂CH₃ | -H | -H | -H | -H |
| 33 | -CH₂CH₂CH₂CH₃ | -CH₂CH₃ | -H | -H | -H | -H |
| 34 | -CH(CH₃)CH₂CH₃ | -CH₂CH₃ | -H | -H | -H | -H |
| 35 | -CH₂CH(CH₃)₂ | -CH₂CH₃ | -H | -H | -H | -H |
| 36 | -C(CH₃)₃ | -CH₂CH₃ | -H | -H | -H | -H |
| 37 | -H | -CH₂CH₃ | -CH₃ | -H | -H | -H |
| 38 | -CH₃ | -CH₂CH₃ | -CH₃ | -H | -H | -H |
| 39 | -CH₂CH₃ | -CH₂CH₃ | -CH₃ | -H | -H | -H |
| 40 | -CH₂CH₂CH₃ | -CH₂CH₃ | -CH₃ | -H | -H | -H |
| 41 | -CH(CH₃)₂ | -CH₂CH₃ | -CH₃ | -H | -H | -H |
| 42 | -CH₂CH₂CH₂CH₃ | -CH₂CH₃ | -CH₃ | -H | -H | -H |
| 43 | -CH(CH₃)CH₂CH₃ | -CH₂CH₃ | -CH₃ | -H | -H | -H |
| 44 | -CH₂CH(CH₃)₂ | -CH₂CH₃ | -CH₃ | -H | -H | -H |
| 45 | -C(CH₃)₃ | -CH₂CH₃ | -CH₃ | -H | -H | -H |
| 46 | -H | -CH₂CH₃ | -CH₂CH₃ | -H | -H | -H |
| 47 | -CH₃ | -CH₂CH₃ | -CH₂CH₃ | -H | -H | -H |
| 48 | -CH₂CH₃ | -CH₂CH₃ | -CH₂CH₃ | -H | -H | -H |
| 49 | -CH₂CH₂CH₃ | -CH₂CH₃ | -CH₂CH₃ | -H | -H | -H |
| 50 | -CH(CH₃)₂ | -CH₂CH₃ | -CH₂CH₃ | -H | -H | -H |
| 51 | -CH₂CH₂CH₂CH₃ | -CH₂CH₃ | -CH₂CH₃ | -H | -H | -H |
| 52 | -CH(CH₃)CH₂CH₃ | -CH₂CH₃ | -CH₂CH₃ | -H | -H | -H |
| 53 | -CH₂CH(CH₃)₂ | -CH₂CH₃ | -CH₂CH₃ | -H | -H | -H |
| 54 | -C(CH₃)₃ | -CH₂CH₃ | -CH₂CH₃ | -H | -H | -H |
| 55 | -H | -CH₂CH₂CH₃ | -H | -H | -H | -H |
| 56 | -CH₃ | -CH₂CH₂CH₃ | -H | -H | -H | -H |
| 57 | -CH₂CH₃ | -CH₂CH₂CH₃ | -H | -H | -H | -H |
| 58 | -CH₂CH₂CH₃ | -CH₂CH₂CH₃ | -H | -H | -H | -H |
| 59 | -CH(CH₃)₂ | -CH₂CH₂CH₃ | -H | -H | -H | -H |
| 60 | -CH₂CH₂CH₂CH₃ | -CH₂CH₂CH₃ | -H | -H | -H | -H |
| 61 | -CH(CH₃)CH₂CH₃ | -CH₂CH₂CH₃ | -H | -H | -H | -H |
| 62 | -CH₂CH(CH₃)₂ | -CH₂CH₂CH₃ | -H | -H | -H | -H |
| 63 | -C(CH₃)₃ | -CH₂CH₂CH₃ | -H | -H | -H | -H |
| 64 | -H | -CH₂CH₂CH₃ | -CH₃ | -H | -H | -H |
| 65 | -CH₃ | -CH₂CH₂CH₃ | -CH₃ | -H | -H | -H |
| 66 | -CH₂CH₃ | -CH₂CH₂CH₃ | -CH₃ | -H | -H | -H |
| 67 | -CH₂CH₂CH₃ | -CH₂CH₂CH₃ | -CH₃ | -H | -H | -H |
| 68 | -CH(CH₃)₂ | -CH₂CH₂CH₃ | -CH₃ | -H | -H | -H |
| 69 | -CH₂CH₂CH₂CH₃ | -CH₂CH₂CH₃ | -CH₃ | -H | -H | -H |
| 70 | -CH(CH₃)CH₂CH₃ | -CH₂CH₂CH₃ | -CH₃ | -H | -H | -H |
| 71 | -CH₂CH(CH₃)₂ | -CH₂CH₂CH₃ | -CH₃ | -H | -H | -H |
| 72 | -C(CH₃)₃ | -CH₂CH₂CH₃ | -CH₃ | -H | -H | -H |
| 73 | -H | -CH₂CH₂CH₃ | -CH₂CH₃ | -H | -H | -H |
| 74 | -CH₃ | -CH₂CH₂CH₃ | -CH₂CH₃ | -H | -H | -H |
| 75 | -CH₂CH₃ | -CH₂CH₂CH₃ | -CH₂CH₃ | -H | -H | -H |
| 76 | -CH₂CH₂CH₃ | -CH₂CH₂CH₃ | -CH₂CH₃ | -H | -H | -H |
| 77 | -CH(CH₃)₂ | -CH₂CH₂CH₃ | -CH₂CH₃ | -H | -H | -H |
| 78 | -CH₂CH₂CH₂CH₃ | -CH₂CH₂CH₃ | -CH₂CH₃ | -H | -H | -H |
| 79 | -CH(CH₃)CH₂CH₃ | -CH₂CH₂CH₃ | -CH₂CH₃ | -H | -H | -H |
| 80 | -CH₂CH(CH₃)₂ | -CH₂CH₂CH₃ | -CH₂CH₃ | -H | -H | -H |
| 81 | -C(CH₃)₃ | -CH₂CH₂CH₃ | -CH₂CH₃ | -H | -H | -H |
| 82 | -H | -CH₂CH₂CH₃ | -CH₂CH₂CH₃ | -H | -H | -H |
| 83 | -CH₃ | -CH₂CH₂CH₃ | -CH₂CH₂CH₃ | -H | -H | -H |
| 84 | -CH₂CH₃ | -CH₂CH₂CH₃ | -CH₂CH₂CH₃ | -H | -H | -H |
| 85 | -CH₂CH₂CH₃ | -CH₂CH₂CH₃ | -CH₂CH₂CH₃ | -H | -H | -H |
| 86 | -CH(CH₃)₂ | -CH₂CH₂CH₃ | -CH₂CH₂CH₃ | -H | -H | -H |
| 87 | -CH₂CH₂CH₂CH₃ | -CH₂CH₂CH₃ | -CH₂CH₂CH₃ | -H | -H | -H |
| 88 | -CH(CH₃)CH₂CH₃ | -CH₂CH₂CH₃ | -CH₂CH₂CH₃ | -H | -H | -H |
| 89 | -CH₂CH(CH₃)₂ | -CH₂CH₂CH₃ | -CH₂CH₂CH₃ | -H | -H | -H |
| 90 | -C(CH₃)₃ | -CH₂CH₂CH₃ | -CH₂CH₂CH₃ | -H | -H | -H |
| 91 | -H | -CH(CH₃)₂ | -H | -H | -H | -H |
| 92 | -CH₃ | -CH(CH₃)₂ | -H | -H | -H | -H |
| 93 | -CH₂CH₃ | -CH(CH₃)₂ | -H | -H | -H | -H |
| 94 | -CH₂CH₂CH₃ | -CH(CH₃)₂ | -H | -H | -H | -H |
| 95 | -CH(CH₃)₂ | -CH(CH₃)₂ | -H | -H | -H | -H |
| 96 | -CH₂CH₂CH₂CH₃ | -CH(CH₃)₂ | -H | -H | -H | -H |
| 97 | -CH(CH₃)CH₂CH₃ | -CH(CH₃)₂ | -H | -H | -H | -H |
| 98 | -CH₂CH(CH₃)₂ | -CH(CH₃)₂ | -H | -H | -H | -H |
| 99 | -C(CH₃)₃ | -CH(CH₃)₂ | -H | -H | -H | -H |
| 100 | -H | -CH(CH₃)₂ | -CH₃ | -H | -H | -H |
| 101 | -CH₃ | -CH(CH₃)₂ | -CH₃ | -H | -H | -H |
| 102 | -CH₂CH₃ | -CH(CH₃)₂ | -CH₃ | -H | -H | -H |
| 103 | -CH₂CH₂CH₃ | -CH(CH₃)₂ | -CH₃ | -H | -H | -H |
| 104 | -CH(CH₃)₂ | -CH(CH₃)₂ | -CH₃ | -H | -H | -H |
| 105 | -CH₂CH₂CH₂CH₃ | -CH(CH₃)₂ | -CH₃ | -H | -H | -H |
| 106 | -CH(CH₃)CH₂CH₃ | -CH(CH₃)₂ | -CH₃ | -H | -H | -H |
| 107 | -CH₂CH(CH₃)₂ | -CH(CH₃)₂ | -CH₃ | -H | -H | -H |
| 108 | -C(CH₃)₃ | -CH(CH₃)₂ | -CH₃ | -H | -H | -H |
| 109 | -H | -CH(CH₃)₂ | -CH₂CH₃ | -H | -H | -H |
| 110 | -CH₃ | -CH(CH₃)₂ | -CH₂CH₃ | -H | -H | -H |
| 111 | -CH₂CH₃ | -CH(CH₃)₂ | -CH₂CH₃ | -H | -H | -H |
| 112 | -CH₂CH₂CH₃ | -CH(CH₃)₂ | -CH₂CH₃ | -H | -H | -H |
| 113 | -CH(CH₃)₂ | -CH(CH₃)₂ | -CH₂CH₃ | -H | -H | -H |
| 114 | -CH₂CH₂CH₂CH₃ | -CH(CH₃)₂ | -CH₂CH₃ | -H | -H | -H |
| 115 | -CH(CH₃)CH₂CH₃ | -CH(CH₃)₂ | -CH₂CH₃ | -H | -H | -H |
| 116 | -CH₂CH(CH₃)₂ | -CH(CH₃)₂ | -CH₂CH₃ | -H | -H | -H |
| 117 | -C(CH₃)₃ | -CH(CH₃)₂ | -CH₂CH₃ | -H | -H | -H |
| 118 | -H | -CH(CH₃)₂ | -CH₂CH₂CH₃ | -H | -H | -H |
| 119 | -CH₃ | -CH(CH₃)₂ | -CH₂CH₂CH₃ | -H | -H | -H |
| 120 | -CH₂CH₃ | -CH(CH₃)₂ | -CH₂CH₂CH₃ | -H | -H | -H |
| 121 | -CH₂CH₂CH₃ | -CH(CH₃)₂ | -CH₂CH₂CH₃ | -H | -H | -H |
| 122 | -CH(CH₃)₂ | -CH(CH₃)₂ | -CH₂CH₂CH₃ | -H | -H | -H |
| 123 | -CH₂CH₂CH₂CH₃ | -CH(CH₃)₂ | -CH₂CH₂CH₃ | -H | -H | -H |
| 124 | -CH(CH₃)CH₂CH₃ | -CH(CH₃)₂ | -CH₂CH₂CH₃ | -H | -H | -H |
| 125 | -CH₂CH(CH₃)₂ | -CH(CH₃)₂ | -CH₂CH₂CH₃ | -H | -H | -H |
| 126 | -C(CH₃)₃ | -CH(CH₃)₂ | -CH₂CH₂CH₃ | -H | -H | -H |
| 127 | -H | -CH(CH₃)₂ | -CH(CH₃)₂ | -H | -H | -H |
| 128 | -CH₃ | -CH(CH₃)₂ | -CH(CH₃)₂ | -H | -H | -H |
| 129 | -CH₂CH₃ | -CH(CH₃)₂ | -CH(CH₃)₂ | -H | -H | -H |
| 130 | -CH₂CH₂CH₃ | -CH(CH₃)₂ | -CH(CH₃)₂ | -H | -H | -H |
| 131 | -CH(CH₃)₂ | -CH(CH₃)₂ | -CH(CH₃)₂ | -H | -H | -H |
| 132 | -CH₂CH₂CH₂CH₃ | -CH(CH₃)₂ | -CH(CH₃)₂ | -H | -H | -H |
| 133 | -CH(CH₃)CH₂CH₃ | -CH(CH₃)₂ | -CH(CH₃)₂ | -H | -H | -H |
| 134 | -CH₂CH(CH₃)₂ | -CH(CH₃)₂ | -CH(CH₃)₂ | -H | -H | -H |
| 135 | -C(CH₃)₃ | -CH(CH₃)₂ | -CH(CH₃)₂ | -H | -H | -H |
| | | | | | | |

| **Nr.** | **R10** | **R11** | **R12** | **R7** | **R8** | **R9** |
|---|---|---|---|---|---|---|
| 136 | -H | -H | -H | -H | -H | -H |
| 137 | -CH₃ | -H | -H | -H | -H | -H |
| 138 | -CH₂CH₃ | -H | -H | -H | -H | -H |
| 139 | -CH₂CH₂CH₃ | -H | -H | -H | -H | -H |
| 140 | -CH(CH₃)₂ | -H | -H | -H | -H | -H |
| 141 | -CH₂CH₂CH₂CH₃ | -H | -H | -H | -H | -H |
| 142 | -CH(CH₃)CH₂CH₃ | -H | -H | -H | -H | -H |
| 143 | -CH₂CH(CH₃)₂ | -H | -H | -H | -H | -H |
| 144 | -C(CH₃)₃ | -H | -H | -H | -H | -H |
| 145 | -H | -CH₃ | -H | -H | -H | -H |
| 146 | -CH₃ | -CH₃ | -H | -H | -H | -H |
| 147 | -CH₂CH₃ | -CH₃ | -H | -H | -H | -H |
| 148 | -CH₂CH₂CH₃ | -CH₃ | -H | -H | -H | -H |
| 149 | -CH(CH₃)₂ | -CH₃ | -H | -H | -H | -H |
| 150 | -CH₂CH₂CH₂CH₃ | -CH₃ | -H | -H | -H | -H |
| 151 | -CH(CH₃)CH₂CH₃ | -CH₃ | -H | -H | -H | -H |
| 152 | -CH₂CH(CH₃)₂ | -CH₃ | -H | -H | -H | -H |
| 153 | -C(CH₃)₃ | -CH₃ | -H | -H | -H | -H |
| 154 | -H | -CH₃ | -CH₃ | -H | -H | -H |
| 155 | -CH₃ | -CH₃ | -CH₃ | -H | -H | -H |
| 156 | -CH₂CH₃ | -CH₃ | -CH₃ | -H | -H | -H |
| 157 | -CH₂CH₂CH₃ | -CH₃ | -CH₃ | -H | -H | -H |
| 158 | -CH(CH₃)₂ | -CH₃ | -CH₃ | -H | -H | -H |
| 159 | -CH₂CH₂CH₂CH₃ | -CH₃ | -CH₃ | -H | -H | -H |
| 160 | -CH(CH₃)CH₂CH₃ | -CH₃ | -CH₃ | -H | -H | -H |
| 161 | -CH₂CH(CH₃)₂ | -CH₃ | -CH₃ | -H | -H | -H |
| 162 | -C(CH₃)₃ | -CH₃ | -CH₃ | -H | -H | -H |
| 163 | -H | -CH₂CH₃ | -H | -H | -H | -H |
| 164 | -CH₃ | -CH₂CH₃ | -H | -H | -H | -H |
| 165 | -CH₂CH₃ | -CH₂CH₃ | -H | -H | -H | -H |
| 166 | -CH₂CH₂CH₃ | -CH₂CH₃ | -H | -H | -H | -H |
| 167 | -CH(CH₃)₂ | -CH₂CH₃ | -H | -H | -H | -H |
| 168 | -CH₂CH₂CH₂CH₃ | -CH₂CH₃ | -H | -H | -H | -H |
| 169 | -CH(CH₃)CH₂CH₃ | -CH₂CH₃ | -H | -H | -H | -H |
| 170 | -CH₂CH(CH₃)₂ | -CH₂CH₃ | -H | -H | -H | -H |
| 171 | -C(CH₃)₃ | -CH₂CH₃ | -H | -H | -H | -H |
| 172 | -H | -CH₂CH₃ | -CH₃ | -H | -H | -H |
| 173 | -CH₃ | -CH₂CH₃ | -CH₃ | -H | -H | -H |
| 174 | -CH₂CH₃ | -CH₂CH₃ | -CH₃ | -H | -H | -H |
| 175 | -CH₂CH₂CH₃ | -CH₂CH₃ | -CH₃ | -H | -H | -H |
| 176 | -CH(CH₃)₂ | -CH₂CH₃ | -CH₃ | -H | -H | -H |
| 177 | -CH₂CH₂CH₂CH₃ | -CH₂CH₃ | -CH₃ | -H | -H | -H |
| 178 | -CH(CH₃)CH₂CH₃ | -CH₂CH₃ | -CH₃ | -H | -H | -H |
| 179 | -CH₂CH(CH₃)₂ | -CH₂CH₃ | -CH₃ | -H | -H | -H |
| 180 | -C(CH₃)₃ | -CH₂CH₃ | -CH₃ | -H | -H | -H |
| 181 | -H | -CH₂CH₃ | -CH₂CH₃ | -H | -H | -H |
| 182 | -CH₃ | -CH₂CH₃ | -CH₂CH₃ | -H | -H | -H |
| 183 | -CH₂CH₃ | -CH₂CH₃ | -CH₂CH₃ | -H | -H | -H |
| 184 | -CH₂CH₂CH₃ | -CH₂CH₃ | -CH₂CH₃ | -H | -H | -H |
| 185 | -CH(CH₃)₂ | -CH₂CH₃ | -CH₂CH₃ | -H | -H | -H |
| 186 | -CH₂CH₂CH₂CH₃ | -CH₂CH₃ | -CH₂CH₃ | -H | -H | -H |
| 187 | -CH(CH₃)CH₂CH₃ | -CH₂CH₃ | -CH₂CH₃ | -H | -H | -H |
| 188 | -CH₂CH(CH₃)₂ | -CH₂CH₃ | -CH₂CH₃ | -H | -H | -H |
| 189 | -C(CH₃)₃ | -CH₂CH₃ | -CH₂CH₃ | -H | -H | -H |
| 190 | -H | -CH₂CH₂CH₃ | -H | -H | -H | -H |
| 191 | -CH₃ | -CH₂CH₂CH₃ | -H | -H | -H | -H |
| 192 | -CH₂CH₃ | -CH₂CH₂CH₃ | -H | -H | -H | -H |
| 193 | -CH₂CH₂CH₃ | -CH₂CH₂CH₃ | -H | -H | -H | -H |
| 194 | -CH(CH₃)₂ | -CH₂CH₂CH₃ | -H | -H | -H | -H |
| 195 | -CH₂CH₂CH₂CH₃ | -CH₂CH₂CH₃ | -H | -H | -H | -H |
| 196 | -CH(CH₃)CH₂CH₃ | -CH₂CH₂CH₃ | -H | -H | -H | -H |
| 197 | -CH₂CH(CH₃)₂ | -CH₂CH₂CH₃ | -H | -H | -H | -H |
| 198 | -C(CH₃)₃ | -CH₂CH₂CH₃ | -H | -H | -H | -H |
| 199 | -H | -CH₂CH₂CH₃ | -CH₃ | -H | -H | -H |
| 200 | -CH₃ | -CH₂CH₂CH₃ | -CH₃ | -H | -H | -H |
| 201 | -CH₂CH₃ | -CH₂CH₂CH₃ | -CH₃ | -H | -H | -H |
| 202 | -CH₂CH₂CH₃ | -CH₂CH₂CH₃ | -CH₃ | -H | -H | -H |
| 203 | -CH(CH₃)₂ | -CH₂CH₂CH₃ | -CH₃ | -H | -H | -H |
| 204 | -CH₂CH₂CH₂CH₃ | -CH₂CH₂CH₃ | -CH₃ | -H | -H | -H |
| 205 | -CH(CH₃)CH₂CH₃ | -CH₂CH₂CH₃ | -CH₃ | -H | -H | -H |
| 206 | -CH₂CH(CH₃)₂ | -CH₂CH₂CH₃ | -CH₃ | -H | -H | -H |
| 207 | -C(CH₃)₃ | -CH₂CH₂CH₃ | -CH₃ | -H | -H | -H |
| 208 | -H | -CH₂CH₂CH₃ | -CH₂CH₃ | -H | -H | -H |
| 209 | -CH₃ | -CH₂CH₂CH₃ | -CH₂CH₃ | -H | -H | -H |
| 210 | -CH₂CH₃ | -CH₂CH₂CH₃ | -CH₂CH₃ | -H | -H | -H |
| 211 | -CH₂CH₂CH₃ | -CH₂CH₂CH₃ | -CH₂CH₃ | -H | -H | -H |
| 212 | -CH(CH₃)₂ | -CH₂CH₂CH₃ | -CH₂CH₃ | -H | -H | -H |
| 213 | -CH₂CH₂CH₂CH₃ | -CH₂CH₂CH₃ | -CH₂CH₃ | -H | -H | -H |
| 214 | -CH(CH₃)CH₂CH₃ | -CH₂CH₂CH₃ | -CH₂CH₃ | -H | -H | -H |
| 215 | -CH₂CH(CH₃)₂ | -CH₂CH₂CH₃ | -CH₂CH₃ | -H | -H | -H |
| 216 | -C(CH₃)₃ | -CH₂CH₂CH₃ | -CH₂CH₃ | -H | -H | -H |
| 217 | -H | -CH₂CH₂CH₃ | -CH₂CH₂CH₃ | -H | -H | -H |
| 218 | -CH₃ | -CH₂CH₂CH₃ | -CH₂CH₂CH₃ | -H | -H | -H |
| 219 | -CH₂CH₃ | -CH₂CH₂CH₃ | -CH₂CH₂CH₃ | -H | -H | -H |
| 220 | -CH₂CH₂CH₃ | -CH₂CH₂CH₃ | -CH₂CH₂CH₃ | -H | -H | -H |
| 221 | -CH(CH₃)₂ | -CH₂CH₂CH₃ | -CH₂CH₂CH₃ | -H | -H | -H |
| 222 | -CH₂CH₂CH₂CH₃ | -CH₂CH₂CH₃ | -CH₂CH₂CH₃ | -H | -H | -H |
| 223 | -CH(CH₃)CH₂CH₃ | -CH₂CH₂CH₃ | -CH₂CH₂CH₃ | -H | -H | -H |
| 224 | -CH₂CH(CH₃)₂ | -CH₂CH₂CH₃ | -CH₂CH₂CH₃ | -H | -H | -H |
| 225 | -C(CH₃)₃ | -CH₂CH₂CH₃ | -CH₂CH₂CH₃ | -H | -H | -H |
| 226 | -H | -CH(CH₃)₂ | -H | -H | -H | -H |
| 227 | -CH₃ | -CH(CH₃)₂ | -H | -H | -H | -H |
| 228 | -CH₂CH₃ | -CH(CH₃)₂ | -H | -H | -H | -H |
| 229 | -CH₂CH₂CH₃ | -CH(CH₃)₂ | -H | -H | -H | -H |
| 230 | -CH(CH₃)₂ | -CH(CH₃)₂ | -H | -H | -H | -H |
| 231 | -CH₂CH₂CH₂CH₃ | -CH(CH₃)₂ | -H | -H | -H | -H |
| 232 | -CH(CH₃)CH₂CH₃ | -CH(CH₃)₂ | -H | -H | -H | -H |
| 233 | -CH₂CH(CH₃)₂ | -CH(CH₃)₂ | -H | -H | -H | -H |
| 234 | -C(CH₃)₃ | -CH(CH₃)₂ | -H | -H | -H | -H |
| 235 | -H | -CH(CH₃)₂ | -CH₃ | -H | -H | -H |
| 236 | -CH₃ | -CH(CH₃)₂ | -CH₃ | -H | -H | -H |
| 237 | -CH₂CH₃ | -CH(CH₃)₂ | -CH₃ | -H | -H | -H |
| 238 | -CH₂CH₂CH₃ | -CH(CH₃)₂ | -CH₃ | -H | -H | -H |
| 239 | -CH(CH₃)₂ | -CH(CH₃)₂ | -CH₃ | -H | -H | -H |
| 240 | -CH₂CH₂CH₂CH₃ | -CH(CH₃)₂ | -CH₃ | -H | -H | -H |
| 241 | -CH(CH₃)CH₂CH₃ | -CH(CH₃)₂ | -CH₃ | -H | -H | -H |
| 242 | -CH₂CH(CH₃)₂ | -CH(CH₃)₂ | -CH₃ | -H | -H | -H |
| 243 | -C(CH₃)₃ | -CH(CH₃)₂ | -CH₃ | -H | -H | -H |
| 244 | -H | -CH(CH₃)₂ | -CH₂CH₃ | -H | -H | -H |
| 245 | -CH₃ | -CH(CH₃)₂ | -CH₂CH₃ | -H | -H | -H |
| 246 | -CH₂CH₃ | -CH(CH₃)₂ | -CH₂CH₃ | -H | -H | -H |
| 247 | -CH₂CH₂CH₃ | -CH(CH₃)₂ | -CH₂CH₃ | -H | -H | -H |
| 248 | -CH(CH₃)₂ | -CH(CH₃)₂ | -CH₂CH₃ | -H | -H | -H |
| 249 | -CH₂CH₂CH₂CH₃ | -CH(CH₃)₂ | -CH₂CH₃ | -H | -H | -H |
| 250 | -CH(CH₃)CH₂CH₃ | -CH(CH₃)₂ | -CH₂CH₃ | -H | -H | -H |
| 251 | -CH₂CH(CH₃)₂ | -CH(CH₃)₂ | -CH₂CH₃ | -H | -H | -H |
| 252 | -C(CH₃)₃ | -CH(CH₃)₂ | -CH₂CH₃ | -H | -H | -H |
| 253 | -H | -CH(CH₃)₂ | -CH₂CH₂CH₃ | -H | -H | -H |
| 254 | -CH₃ | -CH(CH₃)₂ | -CH₂CH₂CH₃ | -H | -H | -H |
| 255 | -CH₂CH₃ | -CH(CH₃)₂ | -CH₂CH₂CH₃ | -H | -H | -H |
| 256 | -CH₂CH₂CH₃ | -CH(CH₃)₂ | -CH₂CH₂CH₃ | -H | -H | -H |
| 257 | -CH(CH₃)₂ | -CH(CH₃)₂ | -CH₂CH₂CH₃ | -H | -H | -H |
| 258 | -CH₂CH₂CH₂CH₃ | -CH(CH₃)₂ | -CH₂CH₂CH₃ | -H | -H | -H |
| 259 | -CH(CH₃)CH₂CH₃ | -CH(CH₃)₂ | -CH₂CH₂CH₃ | -H | -H | -H |
| 260 | -CH₂CH(CH₃)₂ | -CH(CH₃)₂ | -CH₂CH₂CH₃ | -H | -H | -H |
| 261 | -C(CH₃)₃ | -CH(CH₃)₂ | -CH₂CH₂CH₃ | -H | -H | -H |
| 262 | -H | -CH(CH₃)₂ | -CH(CH₃)₂ | -H | -H | -H |
| 263 | -CH₃ | -CH(CH₃)₂ | -CH(CH₃)₂ | -H | -H | -H |
| 264 | -CH₂CH₃ | -CH(CH₃)₂ | -CH(CH₃)₂ | -H | -H | -H |
| 265 | -CH₂CH₂CH₃ | -CH(CH₃)₂ | -CH(CH₃)₂ | -H | -H | -H |
| 266 | -CH(CH₃)₂ | -CH(CH₃)₂ | -CH(CH₃)₂ | -H | -H | -H |
| 267 | -CH₂CH₂CH₂CH₃ | -CH(CH₃)₂ | -CH(CH₃)₂ | -H | -H | -H |
| 268 | -CH(CH₃)CH₂CH₃ | -CH(CH₃)₂ | -CH(CH₃)₂ | -H | -H | -H |
| 269 | -CH₂CH(CH₃)₂ | -CH(CH₃)₂ | -CH(CH₃)₂ | -H | -H | -H |
| 270 | -C(CH₃)₃ | -CH(CH₃)₂ | -CH(CH₃)₂ | -H | -H | -H |
| | | | | | | |

| **Nr.** | **R7** | **R8** | **R9** | **R10** | **R11** | **R12** |
|---|---|---|---|---|---|---|
| 271 | -H | -H | -H | -H | -H | -H |
| 272 | -CH₃ | -H | -H | -CH₃ | -H | -H |
| 273 | -CH₂CH₃ | -H | -H | -CH₂CH₃ | -H | -H |
| 274 | -CH₂CH₂CH₃ | -H | -H | -CH₂CH₂CH₃ | -H | -H |
| 275 | -CH(CH₃)₂ | -H | -H | -CH(CH₃)₂ | -H | -H |
| 276 | -CH₂CH₂CH₂CH₃ | -H | -H | -CH₂CH₂CH₂CH₃ | -H | -H |
| 277 | -CH(CH₃)CH₂CH₃ | -H | -H | -CH(CH₃)CH₂CH₃ | -H | -H |
| 278 | -CH₂CH(CH₃)₂ | -H | -H | -CH₂CH(CH₃)₂ | -H | -H |
| 279 | -C(CH₃)₃ | -H | -H | -C(CH₃)₃ | -H | -H |
| 280 | -H | -CH₃ | -H | -H | -CH₃ | -H |
| 281 | -CH₃ | -CH₃ | -H | -CH₃ | -CH₃ | -H |
| 282 | -CH₂CH₃ | -CH₃ | -H | -CH₂CH₃ | -CH₃ | -H |
| 283 | -CH₂CH₂CH₃ | -CH₃ | -H | -CH₂CH₂CH₃ | -CH₃ | -H |
| 284 | -CH(CH₃)₂ | -CH₃ | -H | -CH(CH₃)₂ | -CH₃ | -H |
| 285 | -CH₂CH₂CH₂CH₃ | -CH₃ | -H | -CH₂CH₂CH₂CH₃ | -CH₃ | -H |
| 286 | -CH(CH₃)CH₂CH₃ | -CH₃ | -H | -CH(CH₃)CH₂CH₃ | -CH₃ | -H |
| 287 | -CH₂CH(CH₃)₂ | -CH₃ | -H | -CH₂CH(CH₃)₂ | -CH₃ | -H |
| 288 | -C(CH₃)₃ | -CH₃ | -H | -C(CH₃)₃ | -CH₃ | -H |
| 289 | -H | -CH₃ | -CH₃ | -H | -CH₃ | -CH₃ |
| 290 | -CH₃ | -CH₃ | -CH₃ | -CH₃ | -CH₃ | -CH₃ |
| 291 | -CH₂CH₃ | -CH₃ | -CH₃ | -CH₂CH₃ | -CH₃ | -CH₃ |
| 292 | -CH₂CH₂CH₃ | -CH₃ | -CH₃ | -CH₂CH₂CH₃ | -CH₃ | -CH₃ |
| 293 | -CH(CH₃)₂ | -CH₃ | -CH₃ | -CH(CH₃)₂ | -CH₃ | -CH₃ |
| 294 | -CH₂CH₂CH₂CH₃ | -CH₃ | -CH₃ | -CH₂CH₂CH₂CH₃ | -CH₃ | -CH₃ |
| 295 | -CH(CH₃)CH₂CH₃ | -CH₃ | -CH₃ | -CH(CH₃)CH₂CH₃ | -CH₃ | -CH₃ |
| 296 | -CH₂CH(CH₃)₂ | -CH₃ | -CH₃ | -CH₂CH(CH₃)₂ | -CH₃ | -CH₃ |
| 297 | -C(CH₃)₃ | -CH₃ | -CH₃ | -C(CH₃)₃ | -CH₃ | -CH₃ |
| 298 | -H | -CH₂CH₃ | -H | -H | -CH₂CH₃ | -H |
| 299 | -CH₃ | -CH₂CH₃ | -H | -CH₃ | -CH₂CH₃ | -H |
| 300 | -CH₂CH₃ | -CH₂CH₃ | -H | -CH₂CH₃ | -CH₂CH₃ | -H |
| 301 | -CH₂CH₂CH₃ | -CH₂CH₃ | -H | -CH₂CH₂CH₃ | -CH₂CH₃ | -H |
| 302 | -CH(CH₃)₂ | -CH₂CH₃ | -H | -CH(CH₃)₂ | -CH₂CH₃ | -H |
| 303 | -CH₂CH₂CH₂CH₃ | -CH₂CH₃ | -H | -CH₂CH₂CH₂CH₃ | -CH₂CH₃ | -H |
| 304 | -CH(CH₃)CH₂CH₃ | -CH₂CH₃ | -H | -CH(CH₃)CH₂CH₃ | -CH₂CH₃ | -H |
| 305 | -CH₂CH(CH₃)₂ | -CH₂CH₃ | -H | -CH₂CH(CH₃)₂ | -CH₂CH₃ | -H |
| 306 | -C(CH₃)₃ | -CH₂CH₃ | -H | -C(CH₃)₃ | -CH₂CH₃ | -H |
| 307 | -H | -CH₂CH₃ | -CH₃ | -H | -CH₂CH₃ | -CH₃ |
| 308 | -CH₃ | -CH₂CH₃ | -CH₃ | -CH₃ | -CH₂CH₃ | -CH₃ |
| 309 | -CH₂CH₃ | -CH₂CH₃ | -CH₃ | -CH₂CH₃ | -CH₂CH₃ | -CH₃ |
| 310 | -CH₂CH₂CH₃ | -CH₂CH₃ | -CH₃ | -CH₂CH₂CH₃ | -CH₂CH₃ | -CH₃ |
| 311 | -CH(CH₃)₂ | -CH₂CH₃ | -CH₃ | -CH(CH₃)₂ | -CH₂CH₃ | -CH₃ |
| 312 | -CH₂CH₂CH₂CH₃ | -CH₂CH₃ | -CH₃ | -CH₂CH₂CH₂CH₃ | -CH₂CH₃ | -CH₃ |
| 313 | -CH(CH₃)CH₂CH₃ | -CH₂CH₃ | -CH₃ | -CH(CH₃)CH₂CH₃ | -CH₂CH₃ | -CH₃ |
| 314 | -CH₂CH(CH₃)₂ | -CH₂CH₃ | -CH₃ | -CH₂CH(CH₃)₂ | -CH₂CH₃ | -CH₃ |
| 315 | -C(CH₃)₃ | -CH₂CH₃ | -CH₃ | -C(CH₃)₃ | -CH₂CH₃ | -CH₃ |
| 316 | -H | -CH₂CH₃ | -CH₂CH₃ | -H | -CH₂CH₃ | -CH₂CH₃ |
| 317 | -CH₃ | -CH₂CH₃ | -CH₂CH₃ | -CH₃ | -CH₂CH₃ | -CH₂CH₃ |
| 318 | -CH₂CH₃ | -CH₂CH₃ | -CH₂CH₃ | -CH₂CH₃ | -CH₂CH₃ | -CH₂CH₃ |
| 319 | -CH₂CH₂CH₃ | -CH₂CH₃ | -CH₂CH₃ | -CH₂CH₂CH₃ | -CH₂CH₃ | -CH₂CH₃ |
| 320 | -CH(CH₃)₂ | -CH₂CH₃ | -CH₂CH₃ | -CH(CH₃)₂ | -CH₂CH₃ | -CH₂CH₃ |
| 321 | -CH₂CH₂CH₂CH₃ | -CH₂CH₃ | -CH₂CH₃ | -CH₂CH₂CH₂CH₃ | -CH₂CH₃ | -CH₂CH₃ |
| 322 | -CH(CH₃)CH₂CH₃ | -CH₂CH₃ | -CH₂CH₃ | -CH(CH₃)CH₂CH₃ | -CH₂CH₃ | -CH₂CH₃ |
| 323 | -CH₂CH(CH₃)₂ | -CH₂CH₃ | -CH₂CH₃ | -CH₂CH(CH₃)₂ | -CH₂CH₃ | -CH₂CH₃ |
| 324 | -C(CH₃)₃ | -CH₂CH₃ | -CH₂CH₃ | -C(CH₃)₃ | -CH₂CH₃ | -CH₂CH₃ |
| 325 | -H | -CH₂CH₂CH₃ | -H | -H | -CH₂CH₂CH₃ | -H |
| 326 | -CH₃ | -CH₂CH₂CH₃ | -H | -CH₃ | -CH₂CH₂CH₃ | -H |
| 327 | -CH₂CH₃ | -CH₂CH₂CH₃ | -H | -CH₂CH₃ | -CH₂CH₂CH₃ | -H |
| 328 | -CH₂CH₂CH₃ | -CH₂CH₂CH₃ | -H | -CH₂CH₂CH₃ | -CH₂CH₂CH₃ | -H |
| 329 | -CH(CH₃)₂ | -CH₂CH₂CH₃ | -H | -CH(CH₃)₂ | -CH₂CH₂CH₃ | -H |
| 330 | -CH₂CH₂CH₂CH₃ | -CH₂CH₂CH₃ | -H | -CH₂CH₂CH₂CH₃ | -CH₂CH₂CH₃ | -H |
| 331 | -CH(CH₃)CH₂CH₃ | -CH₂CH₂CH₃ | -H | -CH(CH₃)CH₂CH₃ | -CH₂CH₂CH₃ | -H |
| 332 | -CH₂CH(CH₃)₂ | -CH₂CH₂CH₃ | -H | -CH₂CH(CH₃)₂ | -CH₂CH₂CH₃ | -H |
| 333 | -C(CH₃)₃ | -CH₂CH₂CH₃ | -H | -C(CH₃)₃ | -CH₂CH₂CH₃ | -H |
| 334 | -H | -CH₂CH₂CH₃ | -CH₃ | -H | -CH₂CH₂CH₃ | -CH₃ |
| 335 | -CH₃ | -CH₂CH₂CH₃ | -CH₃ | -CH₃ | -CH₂CH₂CH₃ | -CH₃ |
| 336 | -CH₂CH₃ | -CH₂CH₂CH₃ | -CH₃ | -CH₂CH₃ | -CH₂CH₂CH₃ | -CH₃ |
| 337 | -CH₂CH₂CH₃ | -CH₂CH₂CH₃ | -CH₃ | -CH₂CH₂CH₃ | -CH₂CH₂CH₃ | -CH₃ |
| 338 | -CH(CH₃)₂ | -CH₂CH₂CH₃ | -CH₃ | -CH(CH₃)₂ | -CH₂CH₂CH₃ | -CH₃ |
| 339 | -CH₂CH₂CH₂CH₃ | -CH₂CH₂CH₃ | -CH₃ | -CH₂CH₂CH₂CH₃ | -CH₂CH₂CH₃ | -CH₃ |
| 340 | -CH(CH₃)CH₂CH₃ | -CH₂CH₂CH₃ | -CH₃ | -CH(CH₃)CH₂CH₃ | -CH₂CH₂CH₃ | -CH₃ |
| 341 | -CH₂CH(CH₃)₂ | -CH₂CH₂CH₃ | -CH₃ | -CH₂CH(CH₃)₂ | -CH₂CH₂CH₃ | -CH₃ |
| 342 | -C(CH₃)₃ | -CH₂CH₂CH₃ | -CH₃ | -C(CH₃)₃ | -CH₂CH₂CH₃ | -CH₃ |
| 343 | -H | -CH₂CH₂CH₃ | -CH₂CH₃ | -H | -CH₂CH₂CH₃ | -CH₂CH₃ |
| 344 | -CH₃ | -CH₂CH₂CH₃ | -CH₂CH₃ | -CH₃ | -CH₂CH₂CH₃ | -CH₂CH₃ |
| 345 | -CH₂CH₃ | -CH₂CH₂CH₃ | -CH₂CH₃ | -CH₂CH₃ | -CH₂CH₂CH₃ | -CH₂CH₃ |
| 346 | -CH₂CH₂CH₃ | -CH₂CH₂CH₃ | -CH₂CH₃ | -CH₂CH₂CH₃ | -CH₂CH₂CH₃ | -CH₂CH₃ |
| 347 | -CH(CH₃)₂ | -CH₂CH₂CH₃ | -CH₂CH₃ | -CH(CH₃)₂ | -CH₂CH₂CH₃ | -CH₂CH₃ |
| 348 | -CH₂CH₂CH₂CH₃ | -CH₂CH₂CH₃ | -CH₂CH₃ | -CH₂CH₂CH₂CH₃ | -CH₂CH₂CH₃ | -CH₂CH₃ |
| 349 | -CH(CH₃)CH₂CH₃ | -CH₂CH₂CH₃ | -CH₂CH₃ | -CH(CH₃)CH₂CH₃ | -CH₂CH₂CH₃ | -CH₂CH₃ |
| 350 | -CH₂CH(CH₃)₂ | -CH₂CH₂CH₃ | -CH₂CH₃ | -CH₂CH(CH₃)₂ | -CH₂CH₂CH₃ | -CH₂CH₃ |
| 351 | -C(CH₃)₃ | -CH₂CH₂CH₃ | -CH₂CH₃ | -C(CH₃)₃ | -CH₂CH₂CH₃ | -CH₂CH₃ |
| 352 | -H | -CH₂CH₂CH₃ | -CH₂CH₂CH₃ | -H | -CH₂CH₂CH₃ | -CH₂CH₂CH₃ |
| 353 | -CH₃ | -CH₂CH₂CH₃ | -CH₂CH₂CH₃ | -CH₃ | -CH₂CH₂CH₃ | -CH₂CH₂CH₃ |
| 354 | -CH₂CH₃ | -CH₂CH₂CH₃ | -CH₂CH₂CH₃ | -CH₂CH₃ | -CH₂CH₂CH₃ | -CH₂CH₂CH₃ |
| 355 | -CH₂CH₂CH₃ | -CH₂CH₂CH₃ | -CH₂CH₂CH₃ | -CH₂CH₂CH₃ | -CH₂CH₂CH₃ | -CH₂CH₂CH₃ |
| 356 | -CH(CH₃)₂ | -CH₂CH₂CH₃ | -CH₂CH₂CH₃ | -CH(CH₃)₂ | -CH₂CH₂CH₃ | -CH₂CH₂CH₃ |
| 357 | -CH₂CH₂CH₂CH₃ | -CH₂CH₂CH₃ | -CH₂CH₂CH₃ | -CH₂CH₂CH₂CH₃ | -CH₂CH₂CH₃ | -CH₂CH₂CH₃ |
| 358 | -CH(CH₃)CH₂CH₃ | -CH₂CH₂CH₃ | -CH₂CH₂CH₃ | -CH(CH₃)CH₂CH₃ | -CH₂CH₂CH₃ | -CH₂CH₂CH₃ |
| 359 | -CH₂CH(CH₃)₂ | -CH₂CH₂CH₃ | -CH₂CH₂CH₃ | -CH₂CH(CH₃)₂ | -CH₂CH₂CH₃ | -CH₂CH₂CH₃ |
| 360 | -C(CH₃)₃ | -CH₂CH₂CH₃ | -CH₂CH₂CH₃ | -C(CH₃)₃ | -CH₂CH₂CH₃ | -CH₂CH₂CH₃ |
| 361 | -H | -CH(CH₃)₂ | -H | -H | -CH(CH₃)₂ | -H |
| 362 | -CH₃ | -CH(CH₃)₂ | -H | -CH₃ | -CH(CH₃)₂ | -H |
| 363 | -CH₂CH₃ | -CH(CH₃)₂ | -H | -CH₂CH₃ | -CH(CH₃)₂ | -H |
| 364 | -CH₂CH₂CH₃ | -CH(CH₃)₂ | -H | -CH₂CH₂CH₃ | -CH(CH₃)₂ | -H |
| 365 | -CH(CH₃)₂ | -CH(CH₃)₂ | -H | -CH(CH₃)₂ | -CH(CH₃)₂ | -H |
| 366 | -CH₂CH₂CH₂CH₃ | -CH(CH₃)₂ | -H | -CH₂CH₂CH₂CH₃ | -CH(CH₃)₂ | -H |
| 367 | -CH(CH₃)CH₂CH₃ | -CH(CH₃)₂ | -H | -CH(CH₃)CH₂CH₃ | -CH(CH₃)₂ | -H |
| 368 | -CH₂CH(CH₃)₂ | -CH(CH₃)₂ | -H | -CH₂CH(CH₃)₂ | -CH(CH₃)₂ | -H |
| 369 | -C(CH₃)₃ | -CH(CH₃)₂ | -H | -C(CH₃)₃ | -CH(CH₃)₂ | -H |
| 370 | -H | -CH(CH₃)₂ | -CH₃ | -H | -CH(CH₃)₂ | -CH₃ |
| 371 | -CH₃ | -CH(CH₃)₂ | -CH₃ | -CH₃ | -CH(CH₃)₂ | -CH₃ |
| 372 | -CH₂CH₃ | -CH(CH₃)₂ | -CH₃ | -CH₂CH₃ | -CH(CH₃)₂ | -CH₃ |
| 373 | -CH₂CH₂CH₃ | -CH(CH₃)₂ | -CH₃ | -CH₂CH₂CH₃ | -CH(CH₃)₂ | -CH₃ |
| 374 | -CH(CH₃)₂ | -CH(CH₃)₂ | -CH₃ | -CH(CH₃)₂ | -CH(CH₃)₂ | -CH₃ |
| 375 | -CH₂CH₂CH₂CH₃ | -CH(CH₃)₂ | -CH₃ | -CH₂CH₂CH₂CH₃ | -CH(CH₃)₂ | -CH₃ |
| 376 | -CH(CH₃)CH₂CH₃ | -CH(CH₃)₂ | -CH₃ | -CH(CH₃)CH₂CH₃ | -CH(CH₃)₂ | -CH₃ |
| 377 | -CH₂CH(CH₃)₂ | -CH(CH₃)₂ | -CH₃ | -CH₂CH(CH₃)₂ | -CH(CH₃)₂ | -CH₃ |
| 378 | -C(CH₃)₃ | -CH(CH₃)₂ | -CH₃ | -C(CH₃)₃ | -CH(CH₃)₂ | -CH₃ |
| 379 | -H | -CH(CH₃)₂ | -CH₂CH₃ | -H | -CH(CH₃)₂ | -CH₂CH₃ |
| 380 | -CH₃ | -CH(CH₃)₂ | -CH₂CH₃ | -CH₃ | -CH(CH₃)₂ | -CH₂CH₃ |
| 381 | -CH₂CH₃ | -CH(CH₃)₂ | -CH₂CH₃ | -CH₂CH₃ | -CH(CH₃)₂ | -CH₂CH₃ |
| 382 | -CH₂CH₂CH₃ | -CH(CH₃)₂ | -CH₂CH₃ | -CH₂CH₂CH₃ | -CH(CH₃)₂ | -CH₂CH₃ |
| 383 | -CH(CH₃)₂ | -CH(CH₃)₂ | -CH₂CH₃ | -CH(CH₃)₂ | -CH(CH₃)₂ | -CH₂CH₃ |
| 384 | -CH₂CH₂CH₂CH₃ | -CH(CH₃)₂ | -CH₂CH₃ | -CH₂CH₂CH₂CH₃ | -CH(CH₃)₂ | -CH₂CH₃ |
| 385 | -CH(CH₃)CH₂CH₃ | -CH(CH₃)₂ | -CH₂CH₃ | -CH(CH₃)CH₂CH₃ | -CH(CH₃)₂ | -CH₂CH₃ |
| 386 | -CH₂CH(CH₃)₂ | -CH(CH₃)₂ | -CH₂CH₃ | -CH₂CH(CH₃)₂ | -CH(CH₃)₂ | -CH₂CH₃ |
| 387 | -C(CH₃)₃ | -CH(CH₃)₂ | -CH₂CH₃ | -C(CH₃)₃ | -CH(CH₃)₂ | -CH₂CH₃ |
| 388 | -H | -CH(CH₃)₂ | -CH₂CH₂CH₃ | -H | -CH(CH₃)₂ | -CH₂CH₂CH₃ |
| 389 | -CH₃ | -CH(CH₃)₂ | -CH₂CH₂CH₃ | -CH₃ | -CH(CH₃)₂ | -CH₂CH₂CH₃ |
| 390 | -CH₂CH₃ | -CH(CH₃)₂ | -CH₂CH₂CH₃ | -CH₂CH₃ | -CH(CH₃)₂ | -CH₂CH₂CH₃ |
| 391 | -CH₂CH₂CH₃ | -CH(CH₃)₂ | -CH₂CH₂CH₃ | -CH₂CH₂CH₃ | -CH(CH₃)₂ | -CH₂CH₂CH₃ |
| 392 | -CH(CH₃)₂ | -CH(CH₃)₂ | -CH₂CH₂CH₃ | -CH(CH₃)₂ | -CH(CH₃)₂ | -CH₂CH₂CH₃ |
| 393 | -CH₂CH₂CH₂CH₃ | -CH(CH₃)₂ | -CH₂CH₂CH₃ | -CH₂CH₂CH₂CH₃ | -CH(CH₃)₂ | -CH₂CH₂CH₃ |
| 394 | -CH(CH₃)CH₂CH₃ | -CH(CH₃)₂ | -CH₂CH₂CH₃ - | CH(CH₃)CH₂CH₃ | -CH(CH₃)₂ | -CH₂CH₂CH₃ |
| 395 | -CH₂CH(CH₃)₂ | -CH(CH₃)₂ | -CH₂CH₂CH₃ | -CH₂CH(CH₃)₂ | -CH(CH₃)₂ | -CH₂CH₂CH₃ |
| 396 | -C(CH₃)₃ | -CH(CH₃)₂ | -CH₂CH₂CH₃ | -C(CH₃)₃ | -CH(CH₃)₂ | -CH₂CH₂CH₃ |
| 397 | -H | -CH(CH₃)₂ | -CH(CH₃)₂ | -H | -CH(CH₃)₂ | -CH(CH₃)₂ |
| 398 | -CH₃ | -CH(CH₃)₂ | -CH(CH₃)₂ | -CH₃ | -CH(CH₃)₂ | -CH(CH₃)₂ |
| 399 | -CH₂CH₃ | -CH(CH₃)₂ | -CH(CH₃)₂ | -CH₂CH₃ | -CH(CH₃)₂ | -CH(CH₃)₂ |
| 400 | -CH₂CH₂CH₃ | -CH(CH₃)₂ | -CH(CH₃)₂ | -CH₂CH₂CH₃ | -CH(CH₃)₂ | -CH(CH₃)₂ |
| 401 | -CH(CH₃)₂ | -CH(CH₃)₂ | -CH(CH₃)₂ | -CH(CH₃)₂ | -CH(CH₃)₂ | -CH(CH₃)₂ |
| 402 | -CH₂CH₂CH₂CH₃ | -CH(CH₃)₂ | -CH(CH₃)₂ | -CH₂CH₂CH₂CH₃ | -CH(CH₃)₂ | -CH(CH₃)₂ |
| 403 | -CH(CH₃)CH₂CH₃ | -CH(CH₃)₂ | -CH(CH₃)₂ | -CH(CH₃)CH₂CH₃ | -CH(CH₃)₂ | -CH(CH₃)₂ |
| 404 | -CH₂CH(CH₃)₂ | -CH(CH₃)₂ | -CH(CH₃)₂ | -CH₂CH(CH₃)₂ | -CH(CH₃)₂ | -CH(CH₃)₂ |
| 405 | -C(CH₃)₃ | -CH(CH₃)₂ | -CH(CH₃)₂ | -C(CH₃)₃ | -CH(CH₃)₂ | -CH(CH₃)₂ |

Zusammenfassend sind erfindungsgemäße Mundwasserzusammensetzungen bevorzugt, die mindestens einen Scharfstoff aus den Gruppen der
- alkylsubstituierten Dioxane der Formel 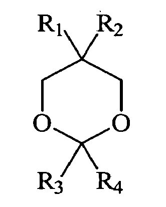 in der R1 und R2 unabhängig voneinander ausgewählt sind aus -H, -CH₃, - CH₂CH₃ und R3 und R4 unabhängig voneinander ausgewählt sind aus -H, -CH₃, - CH₂CH₃, -CH₂CH₂CH₃, -CH(CH₃)₂
   und/oder
- Phenylestern der Formel 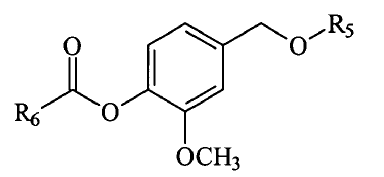 in der R5 für -CH₃ oder einen geradkettigen oder verzweigten Alkyl- oder Alkenylrest mit 2 bis 8 Kohlenstoffatomen und R6 für -CH₃ oder einen geradkettigen oder verzweigten Alkyl- oder Alkenylrest mit 2 bis 8 Kohlenstoffatomen oder eine Alkoxygruppe mit 1 bis 3 Kohlenstoffatomen steht
   und/oder
- Carvonacetalen der Formel 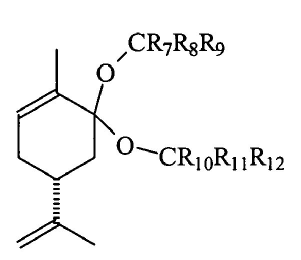 in der R7 bis R12 unabhängig voneinander ausgewählt sind aus -H, -CH₃, -CH₂CH₃, -CH₂CH₂CH₃, -CH(CH₃)₂, -CH₂ CH₂CH₂CH₃, -CH₂CH(CH₃)₂, -CH(CH₃)CH₂CH₃, C(CH₃)₃ oder R9 und R10 zusammen eine chemiche Bindung oder eine Gruppe - (CR13R14)ₓ bedeuten, worin x für die Werte 1 oder 2 steht und R13 und R14 unabhängig voneinander ausgewählt sind aus -H, -CH₃, -CH₂CH₃, -CH₂CH₂CH₃, - CH(CH₃)₂, -CH₂ CH₂CH₂CH₃, -CH₂CH(CH₃)₂, -CH(CH₃)CH₂CH₃, C(CH₃)₃
enthalten.

Die erfindungsgemäßen Mundwasserzusammensetzungen enthalten zusätzlich zu den genannten Inhaltsstoffen weitere Bestandteile, die beispielsweise aus der Gruppe weiterer Wirk- und/oder Hilfsstoffe stammen. Ein weiterer Inhaltsstoff, der vorzugsweise enthalten ist, ist Wasser, welches in den erfindungsgemäßen Mitteln in Mengen bis zu 89,9999 Gew.-% enthalten sein kann. Bevorzugte erfindungsgemäße Zusammensetzungen enthalten 10 bis 80 Gew.-% Wasser, weiter bevorzugt 20 bis 75 Gew.-%, besonders bevorzugt 30 bis 70 Gew.-% und insbesondere 40 bis 60 Gew.-% Wasser, jeweils bezogen auf die gesamte Zusammensetzung.

Das in den erfindungsgemäßen Mundwasserzusammensetzungen enthaltene Wasser kann Leistungswasser sein, dessen Härtegrad je nach Herstellungsort bzw. Quelle des Wassers variieren kann. Möglich und bevorzugt ist es jedoch, Wasser mit Härtegraden zwischen 0 und 20°dH, vorzugsweise zwischen 1 und 16°dH einzusetzen. Besonders bevorzugt ist der Einsatz von technisch vollentsalztem Wasser ("Wasser VE"), das mit Hilfe von lonenaustauschern weitgehend von Salzen befreit wurde.

Die erfindungsgemäßen Mundwasserzusammensetzungen können zusätzlich zu den vorstehend genannten wesentlichen Inhaltsstoffen und Wasser weitere Inhaltsstoffe von Mundreinigungsmitteln, Mundpflegemittein, Zahnreinigungsmitteln und/oder Zahnpflegemitteln enthalten. Die bevorzugten weiteren Inhaltsstoffe werden nachstehend beschrieben.

Eine bevorzugte Klasse von Inhaltsstoffen, die in den erfindungsgemäßen Mundwasserzusammensetzungen enthalten sein kann, stellen die Tenside dar.

Unter dem Begriff Tenside werden grenzflächenaktive Substanzen, die an Ober- und Grenzflächen Adsorptionsschichten bilden oder in Volumenphasen zu Mizellkolloiden oder lyotropen Mesophasen aggregieren können, verstanden. Man unterscheidet Aniontenside bestehend aus einem hydrophoben Rest und einer negativ geladenen hydrophilen Kopfgruppe, amphotere Tenside, welche sowohl eine negative als auch eine kompensierende positive Ladung tragen, kationische Tenside, welche neben einem hydrophoben Rest eine positiv geladene hydrophile Gruppe aufweisen, und nichtionische Tenside, welche keine Ladungen sondern starke Dipolmomente aufweisen und in wäßriger Lösung stark hydratisiert sind.

Als anionische Tenside (E1) eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel R-O(CH₂-CH₂O)ₓ-OSO₃H, in der R eine bevorzugt lineare Alkylgruppe mit 8 bis 30 C-Atomen und x = 0 oder 1 bis 12 ist,
- sulfatierte Hydroxyalkylpolyethylen- und/oder Hydroxyalkylenpropylenglykolether
- Sulfonate ungesättigter Fettsäuren mit 8 bis 24 C-Atomen und 1 bis 6 Doppelbindungen
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel (E1-I),

   R¹(OCH₂CH₂)ₙ-O-P(O)(OX)-OR² (E1-I)

   in der R¹ bevorzugt für einen aliphatischen Kohlenwasserstoffrest mit 8 bis 30 Kohlenstoffatomen, R² für Wasserstoff, einen Rest (CH₂CH₂O)ₙR² oder X, n für Zahlen von 1 bis 10 und X für Wasserstoff, ein Alkali- oder Erdalkalimetall oder NR³R⁴R⁵R⁶, mit R³ bis R⁶ unabhängig voneinander stehend für Wasserstoff oder einen C₁ bis C₄ - Kohlenwasserstoffrest, steht,
- sulfatierte Fettsäurealkylenglykolester der Formel (E1-II)

   R⁷CO(AlkO)ₙSO₃M (E1-II)

   in der R⁷CO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, n für Zahlen von 0,5 bis 5 und M für ein Kation steht,
- Monoglyceridsulfate und Monoglyceridethersulfate der Formel (E1-III) 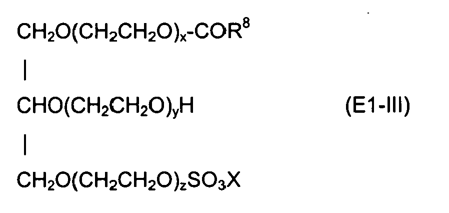 in der R⁸CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate der Formel (E1-III) eingesetzt, in der R⁸CO für einen linearen Acylrest mit 8 bis 18 Kohlenstoffatomen steht, ,
- Amidethercarbonsäuren,
- Kondensationsprodukte aus C₈ - C₃₀ - Fettalkoholen mit Proteinhydrolysaten und/oder Aminosäuren und deren Derivaten,welche dem Fachmann als Eiweissfettsäurekondensate bekannt sind, wie beispielsweise die Lamepon^{®} - Typen, Gluadin^{®} - Typen, Hostapon^{®} KCG oder die Amisoft^{®} - Typen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül, Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen, Monoglycerdisulfate, Alkyl- und Alkenyletherphosphate sowie Eiweissfettsäurekondensate.

Als zwitterionische Tenside (E2) werden solche oberflächenaktiven Verbindungen bezeichnet, die im Molekül mindestens eine quartäre Ammoniumgruppe und mindestens eine -COO⁽⁻⁾ - oder - SO₃⁽⁻⁾ -Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCl-Bezeichnung Cocamidopropyl Betaine bekannte Fettsäureamid-Derivat.

Unter ampholytischen Tensiden (E3) werden solche oberflächenaktiven Verbindungen verstanden, die außer einer C₈ - C₂₄ - Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das C₁₂ - C₁₈ - Acylsarcosin.

Nichtionische Tenside (E4) enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂ - C₆ - Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen^{®} HSP (Cognis) oder Sovermol - Typen (Cognis),
- alkoxilierte Triglyceride,
- alkoxilierte Fettsäurealkylester der Formel (E4-I)

   R¹CO-(OCH₂CHR²)_{w}OR³ (E4-I)

   in der R¹CO für einen linearen oder verzweigten, gesättigten und/oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und w für Zahlen von 1 bis 20 steht,
- Aminoxide,
- Hydroxymischether,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside gemäß Formel (E4-II),

   R⁴O-[G]ₚ (E4-II)

   in der R⁴ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Die Alkyl- und Alkenyloligoglykoside können sich von Aldosen bzw. Ketosen mit 5 oder 6 Kohlenstoffatomen, vorzugsweise von Glucose, ableiten. Die bevorzugten Alkyl- und/oder Alkenyloligoglykoside sind somit Alkyl- und/oder Alkenyloligoglucoside. Die Indexzahl p in der allgemeinen Formel (E4-II) gibt den Oligomerisierungsgrad (DP), d. h. die Verteilung von Mono- und Oligoglykosiden an und steht für eine Zahl zwischen 1 und 10. Während p im einzelnen Molekül stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl-und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R⁴ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R¹⁵ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.
- Zuckertenside vom Typ der Fettsäure-N-alkylpolyhydroxyalkylamide, ein nichtionisches Tensid der Formel (E4-III),

   R⁵CO-NR⁶-[Z] (E4-III)

   in der R⁵CO für einen aliphatischen Acylrest mit 6 bis 22 Kohlenstoffatomen, R⁶ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht. Bei den Fettsäure-N-alkylpolyhydroxyalkylamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden. Vorzugsweise leiten sich die Fettsäure-N-alkylpolyhydroxyalkylamide von reduzierenden Zuckern mit 5 oder 6 Kohlenstoffatomen, insbesondere von der Glucose ab. Die bevorzugten Fettsäure-N-alkylpolyhydroxyalkylamide stellen daher Fettsäure-N-alkylglucamide dar, wie sie durch die Formel (E4-IV) wiedergegeben werden:

   R⁷CO-NR⁸-CH₂(CHOH)₄CH₂OH (E4-IV)

   Vorzugsweise werden als Fettsäure-N-alkylpolyhydroxyalkylamide Glucamide der Formel (E4-IV) eingesetzt, in der R⁸ für Wasserstoff oder eine Alkylgruppe steht und R⁷CO für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. derer technischer Mischungen steht. Besonders bevorzugt sind Fettsäure-N-alkylglucamide der Formel (E4-IV), die durch reduktive Aminierung von Glucose mit Methylamin und anschließende Acylierung mit Laurinsäure oder C12/14-Kokosfettsäure bzw. einem entsprechenden Derivat erhalten werden. Weiterhin können sich die Polyhydroxyalkylamide auch von Maltose und Palatinose ableiten.

Als bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Diese Verbindungen sind durch die folgenden Parameter gekennzeichnet. Der Alkylrest R enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Weiterhin sind ganz besonders bevorzugte nichtionische Tenside die Zuckertenside. Diese können in den erfindungsgemäß verwendeten Mitteln bevorzugt in Mengen von 0,1 - 20 Gew.-%, bezogen auf das gesamte Mittel, enthalten sein. Mengen von 0,5 - 15 Gew.-% sind bevorzugt, und ganz besonders bevorzugt sind Mengen von 0,5 - 7,5 Gew.%.

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder - alkoholate als Katalysatoren verwendet werden. Die Verwendung von Produkten mit eingeengter Homologenverteilung kann bevorzugt sein.

Erfindungsgemäß einsetzbar sind kationische Tenside vom Typ der quarternären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bevorzugt einsetzbar sind erfindungsgemäß QAV mit Behenylresten, insbesondere die unter der Bezeichnung Behentrimoniumchlorid bzw. -bromid (Docosanyltrimethylammonium Chlorid bzw. - Bromid) bekannten Substanzen. Andere bevorzugte QAV weisen mindestens zwei Behenylreste auf, wobei QAV, welche zwei Behenylreste an einem Imidazoliniumrückgrat besonders bevorzugt sind. Kommerziell erhältlich sind diese Substanzen beispielsweise unter den Bezeichnungen Genamin^{®} KDMP (Clariant) und Crodazosoft^{®} DBQ (Crodauza).

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid, sowie Dehyquart^{®} F-75, Dehyquart^{®} C-4046, Dehyquart^{®} L80 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyl-dimethylamin dar.

Das bzw. die Tensid(e) werden in den erfindungsgemäßen Mundwasserzusammensetzungen vorzugsweise in Mengen von 0,01 bis 10 Gew.-% eingesetzt, wobei geringere Mengen, beispielsweise von 0,05 bis 5 Gew.-% oder 0,1 bis 2,5 Gew.-%, jeweils bezogen auf das gesamte Mittel, bevorzugt sind. Mit besonderem Vorzug werden nichtionische Tenside eingesetzt, wobei unter diesen die ethoxylierten Niotenside und die Zuckertenside besonders bevorzugt sind. Bevorzugte erfindungsgemäße Mundwasserzusammensetzungen enthalten Zuckertenside vom Typ der Alkyl- und Alkenyloligoglykoside der Formel R⁴O-[G]ₚ, in der R⁴ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht, wobei bevorzugte Zuckertenside aus der Gruppe der Alkylpolyglucoside stammen, die einen Oligomerisierungsgrad p von 1 bis 3, vorzugsweise von 1,1 bis 2 und insbesondere von 1,2 bis 1,5 aufweisen und als Alkylrest R⁴ einen Rest mit 8 bis 14 Kohlenstoffatomen, vorzugsweise mit 10 bis 12 Kohlenstoffatomen, tragen.

Weitere erfindungsgemäß bevorzugte Mundwasserzusammensetzungen sind dadurch gekennzeichnet, daß sie zusätzlich 0,05 bis 5 Gew.-%, vorzugsweise 0,1 bis 2,5 Gew.-% und insbesondere 0,15 bis 1,2 Gew.-% nichtionische(s) Tensid(e), vorzugsweise ethoxylierte(s) nichtionische(s) Tensid(e) und insbesondere ethoxylierte(s) nichtionische(s) Tensid(e) mit 40 bis 100, vorzugsweise 50 bis 90 und insbesondere 60 bis 80 EO-Gruppen, enthalten.

Die erfindungsgemäßen Mundwasserzusammensetzungen können z. B. auch antimikrobielle Stoffe als Konservierungsmittel oder als Antiplaque-Wirkstoffe enthalten. Derartige Stoffe können z. B. ausgewählt sein aus p-Hydroxybenzoesäuremethyl-, -ethyl- oder propylester, Natriumsorbat, Natriumbenzoat, Bromchlorophen, Triclosan, Phenyl- Salicylsäureester, Biguanide z. B. Chlorhexidin, Thymol usw.. Erfindungsgemäß bevorzugte Mundwasserzusammensetzungen sind dadurch gekennzeichnet, daß sie zusätzlich Antiplaque-Wirkstoffe, vorzugsweise p-Hydroxybenzoesäuremethyl-, -ethyl- oder propylester, Natriumsorbat, Natriumbenzoat, Bromchlorophen, Triclosan, Phenyl- Salicylsäureester, Biguanide z. B. Chlorhexidin, Thymol, vorzugsweise in Mengen von 0,01 bis 5 Gew.%, vorzugsweise von 0,02 bis 2,5 Gew.% und insbesondere von 0,03 bis 1,5 Gew.%, jeweils bezogen auf das gesamte Mittel, enthalten.

Besonders bevorzugt ist erfindungsgemäß der Einsatz von Biguanidinverbindungen. Diese antimikrobiell wirksamen Biguanidverbindungen sind bevorzugt in sehr niedriger Konzentration von 0,0025 bis 0,04 Gew.- % enthalten. Als antimikrobiell wirksame Biguanidverbindung wird bevorzugt das 1,1in-Hexamethylen-bis-(4-chlorphenyl)- biguanid ("Chlorhexidin") in Form eines wasserlöslichen, physiologisch verträglichen Salzes, z. B. in Form des Acetats oder als Gluconat eingesetzt. Andere erfindungsgemäß geeignete antimikrobielle Biguanidverbindungen sind z. B. die Polyhexamethylenbiguanidverbindungen des Typs Vantocil (R) IB (ICI) oder das 1,6-Bis-(4-chlorbenzylbiguanido)-hexan (Fluorhexidin).

Erfindungsgemäß bevorzugte Mundwasserzusammensetzungen sind dadurch gekennzeichnet, daß sie zusätzlich Antiplaque-Wirkstoffe, vorzugsweise p-Hydroxybenzoesäuremethyl-, -ethyl- oder propylester, Natriumsorbat, Natriumbenzoat, Bromchlorophen, Triclosan, Phenyl-Salicylsäureester, Biguanide z. B. Chlorhexidin, Thymol, vorzugsweise in Mengen von 0,01 bis 2,5 Gew.%, vorzugsweise von 0,05 bis 1 Gew.% und insbesondere von 0,1 bis 0,5 Gew.%, jeweils bezogen auf das gesamte Mittel, enthalten.

Eine weitere bevorzugte Gruppe von Inhaltsstoffen, die in den erfindungsgemäßen Mitteln enthalten sein kann, sind die Antikaries-Wirkstoffe. Diese können beispielsweise aus organischen oder anorganischen Fluoriden ausgewählt sein, z. B. aus Natriumfluorid, Kaliumfluorid, Natriummonofluorphosphat und Natriumfluorosilikat. Auch Zinkfluorid, Zinn-(II)-fluorid sind bevorzugt. Bevorzugt sollte eine Menge von 0,01 - 0,2 Gew.-% Fluor in Form der genannten Verbindungen enthalten sein.

Erfindungsgemäße Mundwasserzusammensetzung die zusätzlich Antikaries-Wirkstoffe, vorzugsweise Fluorverbindung(en), insbesondere Natriumfluorid, Kaliumfluorid, Natriummonofluorphosphat, Zinkfluorid, Zinnfluorid und Natriumfluorosilikat, vorzugsweise in Mengen von 0,01 bis 2,5 Gew.%, vorzugsweise von 0,02 bis 1 Gew.% und insbesondere von 0,03 bis 0,5 Gew.%, jeweils bezogen auf das gesamte Mittel, sind erfindungsgemäß bevorzugt.

Die Mund- und Zahnpflege- und reinigungsmittel, insbesondere die Zahnpasten, können z.B. auch Substanzen enthalten, die gegen Zahnstein wirksam sind. Derartige Substanzen können beispielsweise Chelatbildner sein wie z. B. Ethylendiamintetraessigsäure und deren Natriumsalze, Pyrophosphat- Salze wie die wasserlöslichen Dialkali- oder Tetraalkalimetallpyrophosphat- Salze, z. B. Na₄P₂O₇, K₄P₂O₇, Na₂K₂P₂O₇, Na₂H₂P₂O₇ und K₂H₂P₂O₇ oder Polyphosphat-Salze, die z. B. aus wasserlöslichen Alkalimethalltripolyphosphaten wie Natriumtripolyphosphat und Kaliumtripolyphosphat ausgewählt sein können.

Erfindungsgemäß bevorzugte Mundwasserzusammensetzungen sind dadurch gekennzeichnet, daß sie zusätzlich Phosphat(e), vorzugsweise Alkalimetallphosphat(e) und insbesondere Natriumtripolyphosphat, vorzugsweise in Mengen von 0,1 bis 5 Gew.-%, besonders bevorzugt von 0,2 bis 2,5 Gew.-% und insbesondere von 0,3 bis 1 Gew.-%, jeweils bezogen auf das gesamte Mittel, enthalten.

Die erfindungsgemäßen Mundwasserzusammensetzungen können mehrwertige Alkohole, insbesondere Xylit bzw. Sorbit bzw. Glycerin bzw. 1,2-Propylenglycol enthalten. Xylitol, auch "Xylit" genannt, ist ein zu den Pentiten (5-wertige Alkohole) gehörendes Polyol (*xylo*-1,2,3,4,5-Pentanpentol), das durch katalytische ydrierung von Xylose gewonnen wird. Erfindungsgemäß bevorzugte Mundwasserzusammensetzungen enthalten bezogen auf ihr Gewicht 0,5 bis 5 Gew.-% Xylitol, vorzugsweise 0,75 bis 4 Gew.-%, besonders bevorzugt 1 bis 3,75 Gew.-% und insbesondere 1,25 bis 2,75 Gew.-%.

Die erfindungsgemäßen Mundwasserzusammensetzungen können auch Glycerin enthalten. Glycerin wird nach IUPAC als 1,2,3-Propantriol bezeichnet, die INCI-Bezeichnung ist: Glycerin, andere Namen sind beispielsweise 1,2,3-Trihydroxypropan, Glycerol, Ölsüß, oder E 422.

Erfindungsgemäß bevorzugte Mundwasserzusammensetzungen enthalten bezogen auf ihr Gewicht 1 bis 15 Gew.-% Glycerin, wobei erfindungsgemäße Mundwasserzusammensetzungen bevorzugt sind, die bezogen auf ihr Gewicht 2 bis 12 Gew.-%, vorzugsweise 2,5 bis 11 Gew.-% und insbesondere 3 bis 10 Gew.-% Glycerin enthalten.

Zusätzlich zu Glycerin und Xylitol können die erfindungsgemäßen Mundwasserzusammensetzungen weitere Polyhydroxyverbindungen, vorzugsweise Alkohole mit mindestens 2 OH-Gruppen, besonders bevorzugt Mannit, Xylitol, Polyethylenglycol, Polypropylenglycol und deren Mischungen, enthalten.

Unter den Polyhydroxyverbindungen sind diejenigen mit 2 bis 12 OH-Gruppen und insbesondere diejenigen mit 2, 3, 4, 5, 6 oder 10 OH-Gruppen bevorzugt.

Polyhydroxyverbindungen mit 2 OH-Gruppen sind beispielsweise Glycol (CH₂(OH)CH₂OH) und andere 1,2-Diole wie H-(CH₂)ₙ-CH(OH)CH₂OH mit n = 2, 3, 4, 5, 6, 7, 8, 9, 10, 11, 12, 13, 14, 15, 16, 17, 18, 19, 20. Auch 1,3-Diole wie H-(CH₂)ₙ-CH(OH) CH₂CH₂OH mit n = 1, 2, 3, 4, 5, 6, 7, 8, 9, 10, 11, 12, 13, 14, 15, 16, 17, 18, 19, 20 sind erfindungsgemäß einsetzbar. Die (n,n+1)- bzw. (n,n+2)-Diole mit nicht endständigen OH-Gruppen können ebenfalls eingesetzt werden.

Wichtige Vertreter von Polyhydroxyverbindungen mit 2 OH-Gruppen sind auch die Polyethylen- und Polypropylenglycole.

Besonders bevorzugt sind erfindungsgemäße Mundwasserzusammensetzungen, die zusätzlich Sorbitol, vorzugsweise in Mengen von 1 bis 20 Gew.-%, besonders bevorzugt von 2 bis 15 Gew.-% und insbesondere von 3 bis 10 Gew.-%, jeweils bezogen auf das gesamte Mittel, enthalten.

Besonders bevorzugt ist weiterhin, daß die Gesamtmenge an Polyhydroxyverbindungen in den erfindungsgemäßen Mundwasserzusammensetzungen 1,5 bis 35 Gew.-%, vorzugsweise 2,5 bis 32,5 Gew.-%, besonders bevorzugt 5 bis 30 Gew.-% und insbesondere 7 bis 24 Gew.-%, jeweils bezogen auf das gesamte Mittel, beträgt.

Die Mundwasserzusammensetzungen können auch die Unempfindlichkeit der Zähne steigernde Substanzen enthalten, beispielsweise Kaliumsalze wie z. B. Kaliumnitrat, Kaliumcitrat, Kaliumchlorid, Kaliumbicarbonat und Kaliumoxalat. Erfindungsgemäß bevorzugte Mundwasserzusammensetzungen sind dadurch gekennzeichnet, daß sie die Unempfindlichkeit der Zähne steigernde Substanzen, vorzugsweise Kaliumsalze, besonders bevorzugt Kaliumnitrat und/oder Kaliumcitrat und/oder Kaliumchlorid und/oder Kaliumbicarbonat und/oder Kaliumoxalat, vorzugsweise in Mengen von 0,05 bis 5 Gew.%, besonders bevorzugt von 0,1 bis 2,5 Gew.%, weiter bevorzugt von 0,2 bis 1 Gew.-% und insbesondere von 0,25 bis 0,75 Gew.%, jeweils bezogen auf das gesamte Mittel, enthalten.

Die erfindungsgemäßen Mittel können auch zusätzlich weitere wundheilende und entzündungshemmende Stoffe, z. B. Wirkstoffe gegen Zahnfleischentzündungen, enthalten. Derartige Stoffe können z. B. ausgewählt sein aus Allantoin, Azulen, Kamillenextrakten, Tocopherol, Panthenol, Bisabolol, Salbeiextrakten.

Als nicht-kationische, bakterizide Komponente eignen sich z.B. Phenole, Resorcine, Bisphenole, Salicylanilide und deren halogenierte Derivate, halogenierte Carbanilide und p-Hydroxybenzoesäureester. Besonders bevorzugte antimikrobielle Komponenten sind halogenierte Diphenylether, z.B. 2,4-Dichlor-2`-hydroxydiphenylether, 4,4'-Dichlor-2'-hydroxydiphenylether, 2,4,4'-Tribrom-2'-hydroxydiphenylether und 2,4,4'-Trichlor-2'-hydroxydiphenylether (Triclosan). Sie werden bevorzugt in Mengen von 0,01 - 1 Gew.-% in die erfindungsgemäßen Mundwasserzusammensetzungen eingesetzt. Besonders bevorzugt wird Triclosan in einer Menge von 0,01 - 0,3 Gew.-% eingesetzt.

D-Panthenol D - (+) - 2,4-Dihydroxy-N-(3-hydroxypropyl)-3,3-dimethyl-butyramid zeigt eine der Pantothensäure entsprechende biologische Aktivität. Die Pantothensäure (R - (+) - N - (2,4-Dihydroxy-3,3-dimethylbutyryl-β-alanin) ist eine Vorstufe in der Biosynthese des Coenzyms A und wird zum Vitamin-B-Komplex (B3) gezählt. Diese Stoffe sind dafür bekannt, daß sie die Wundheilung fördern und eine günstige Wirkung auf die Haut haben. Sie sind daher auch gelegentlich in Zahnpasten beschrieben worden. Die erfindungsgemäßen Mundwasserzusammensetzungen enthalten bevorzugt 0,05 - 5 Gew.-% Panthenol oder ein Salz der Pantothensäure.

Retinol (3,7-Dimethyl-9-(2,6,6-trimethyl-1-cyclohexenyl)-2,4,6,8-nonatetraen-1-ol ist der internationale Freiname für Vitamin A1. Anstelle des Retinols kann auch eines seiner Derivate mit ähnlicher biologischer Wirkung, z.B. ein Ester oder die Retinoesäure (Tretinoin), eines ihrer Salze oder ihre Ester verwendet werden. Bevorzugt wird ein Retinol-Ester, insbesondere ein Fettsäureester einer Fettsäure mit 12 - 22 C-Atomen verwendet. Besonders bevorzugt ist Retinol-Palmitat geeignet. Bei Verwendung eines Retinol-Esters, z.B. Retinol-Palmitat mit einer Aktivität von 1,7 10⁶ I.E. pro g ist eine Menge von 0,001 bis 0,1 Gew.-% bevorzugt. Bei Verwendung anderer Retinol-Derivate empfiehlt sich eine Einsatzmenge, die einer Konzentration von 10³ bis 10⁶ I.E. (Internationale Einheiten) pro 100 g entspricht. Bevorzugte Mundwasserzusammensetzungen gemäß der vorliegenden Erfindung enthalten neben den vorstehend genannten zwingnden Inhaltstoffen bevorzugt
0,01 -1 Gew.-% eines halogenierten Diphenylethers
0,05 - 5 Gew.-% Panthenol oder ein Salz der Pantothensäure und
0,01 - 0,1 Gew.-% eines Retinol-Esters, bevorzugt Retinol-Palmitat.

Als wasserunlösliche Aromaöle kommen alle für Mund- und Zahnpflegemittel gebräuchlichen natürlichen und synthetischen Aromen infrage. Natürliche Aromen können sowohl in Form der aus den Drogen isolierten etherischen le als auch der aus diesen isolierten Einzelkomponenten verwendet werden. Bevorzugt sollte wenigstens ein Aromaöl aus der Gruppe Pfefferminzöl, Krauseminzöl, Anisöl, Sternanisöl, Kümmelöl, Eukalyptusöl, Fenchelöl, Zimtöl, Nelkenöl, Geraniumöl, Salbeiöl, Pimentöl, Thymianöl, Majoranöl, Basilikumöl, Citrusöl, Gaultheriaöl oder eine oder mehrere darausisolierte oder synthetisch erzeugte Komponenten dieser le enthalten sein. Die wichtigsten Komponenten der genannten le sind z. B. Menthol, Carvon, Anethol, Cineol, Eugenol, Zimtaldehyd, Caryophyllen, Geraniol, Citronellol, Linalool, Salven, Thymol, Terpinen, Terpinol, Methylchavicol und Methylsalicylat. Weitere geeignete Aromen sind z. B. Menthylacetat, Vanillin, Jonone, Linalylacetat, Rhodinol und Piperiton.

Lösungsvermittler aus der Gruppe der oxethylierten Fettsäureglyceride umfassen vor allem Anlagerungsprodukte von 20-60 Mol Ethylenoxid an Mono- und Diglyceride von linearen Fettsäuren mit 12-18 C- Atomen oder an Triglyceride von Hydroxyfettsäuren wie Oxystearinsäure oder Ricinolsäure. Weitere geeignete Lösungsvermittler sind oxethylierte Fettsäuresorbitanpartialester; das sind bevorzugt Anlagerungsprodukte von 20-60 Mol Ethylenoxid an Sorbitanmonoester und Sorbitandiester von Fettsäuren mit 12-18 C-Atomen. Ebenfalls geeignete Lösungsvermittler sind Fettsäurepartialester von Glycerin- oder Sorbitan- Oxethylaten; das sind bevorzugt Mono- und Diester von C12-18-Fettsäuren und Anlagerungsprodukten von 20-60 Mol Ethylenoxid an 1 Mol Glycerin oder an 1 Mol Sorbit.

Die erfindungsgemäßen Zubereitungen enthalten bevorzugt als Lösungsvermittler Anlagerungsprodukte von 20-60 Mol Ethylenoxid an gehärtetes oder ungehärtetes Ricinusöl (d. h. an Oxystearinsäure- oder Ricinolsäure-triglycerid), an Glycerin-mono- und/oder distearat oder an Sorbitanmono- und/oder distearat.

Neben den genannten obligatorischen Komponenten können die erfindungsgemäßen Zubereitungen noch weitere, an sich bekannte und in antimikrobiellen Zubereitungen für den Bereich der Körperhygiene übliche Komponenten zur Verbesserung des Aussehens, des Geschmacks oder der Handhabung enthalten. Zur Verbesserung des Aussehens können z. B. physiologisch unbedenklicheFarbstoffe, Trübungs- oder Perlglanzmittel zugegeben werden. Zur Verbesserung des Geschmackes können natürliche oder synthetische, bevorzugt von kariogenen Kohlehydraten freie Süssungsmittel zugegeben werden. Hierzu gehören z.B. Saccharin-Natrium, Cyclamate, Acesulfam- Kalium, Aspartame^{(R)} (L-Aspartyl-L- phenylalaninmethylester). Weitere geeignete Süßungsmittel sind z.B. Sucrose, Lactose, Meltose, Fructose.

Die erfindungsgemäßen Mundwasserzusammensetzung weisen vorzugsweise Viskositäten (gemesen bei 20°C mit Brookfield-Viskosimeter DV III, Spindel 4, 4 U/min) unterhalb von 1000 mPas auf. Besonders bevorzugt sind Viskositäten unterhalb von 500 mPas, weiter bevorzugt unterhalb von 250 mPas und insbesondere von unterhalb 100 mPas.

Sollten in Einzelfällen höhere Viskositäten gewünscht sein, können die erfindungsmäßen Mundwasserzusammensetzungen als Konsistenzregler z. B. natürliche und/oder synthetische wasserlösliche Polymere wie Alginate, Carragheenate, Traganth, Stärke und Stärkeether, Celluloseether wie z. B. Carboxymethylcellulose (Na-Salz), Hydroxyethylcellulose, Methylhydroxypropylcellulose, Guar, Akaziengum, Agar-Agar, Xanthan- Gum, Succinoglycan-Gum, Johannisbrotmehl, Pectine, wasserlösliche Carboxyvinylpolymere (z. B. Carbopol®-Typen), Polyvinylalkohol, Polyvinylpyrrolidon, Polyethylenglycole, insbesondere solche mit Molekulargewichten von 1 500-1 000 000, enthalten.

Weitere Stoffe, die sich zur Viskositätskontrolle eignen, sind z. B. Schichtsilikate wie z. B. Montmorillonit-Tone, kolloidale Verdickungskieselsäuren wie z. B. Aerogel-Kieselsäuren, pyrogene Kieselsäuren oder feinstvermahlene Fällungskieselsäuren. Es können auch viskositätsstabilisierende Zusätze aus der Gruppe der kationischen, zwitterionischen oder ampholytischen stickstoffhaltigen Tenside, der hydroxypropylsubstituierten Hydrocolloide oder der Polyethylenglycol/Polypropylenglycol- Copolymere mit einem mittleren Molgewicht von 1000 bis 5000 oder eine Kombination der genannten Verbindungen verwendet werden.

Ein weiterer Gegenstand der vorliegenden Erfindung ist ein Verfahren zur Reinigung der Mundhöhle, bei dem eine erfindungsgemäße gebrauchsfertige Mundwasserzusammensetzung unverdünnt in die Mundhöhle eingebracht und nach einer Einwirkzeit wieder aus ihr entfernt wird.

In bevorzugten erfindungsgemäßen Verfahren beträgt die Einwirkzeit 10 Sekunden bis fünf Minuten, vorzugsweise 20 bis 240 Sekunden, besonders bevorzugt 30 bis 180 Sekunden und insbesondere 60 bis 120 Sekunden. In weiter bevorzugten erfindungsgemäßen Verfahren wird die gebrauchsfertige Lösung innerhalb der Einwirkzeit im Mund bewegt, was der Anwender durch Spül- oder Gurgelbewegungen unterstützen kann.

Bevorzugt ist das Einbringen der erfindungsgemäßen Mundwasserzusammensetzung in die Mundhöhle in Mengen von 10 bis 200 ml, vorzugsweise von 50 bis 150 ml, besonders bevorzugt von 60 bis 120 ml und insbesondere von 70 bis 100 ml pro Anwendung.

Besonders bevorzugt läßt sich die Anwendung des erfindungsgemäßen Verfahrens durch den verbraucher sicherstellen, wenn die erfindungsgemäßen Mundwasserzusammensetzungen mit einer entsprechenden Gebrauchsanweisung versehen werden. Solche Mundwasserzusammensetzungen stellen quasi ein "Kit" aus der verpackten Mundwasserzusammensetzung und der Gebrauchsanweisung dar.

Solche erfindungsgemäßen Kits umfassen mindestens eine Verpackung. Diese Verpackung dient bei den nicht selbst formstabilen erfindungsgemäßen Mundwasserzusammensetzungen zu ihrer Aufbewahrung und erleichtert ihre Dosierung und Anwendung. Erfindungsgemäß bevorzugte Verpackungen sind beispielsweise Tuben, Spender, Flaschen, Dosen, Tiegel, Töpfe, Spraydosen, Boxen, Eimer, Kisten, Kübel usw.. Sie können aus den unterschiedlichsten Materialien gefertigt sein, beispielsweise Kunststoff, Metall, Glas, Verbundwerkstoffen usw.. Verpackungen für kosmetische Produkte im allgemeinen und für speziell Mundwässer sind im Stand der Technik breit beschrieben, und je nach zu verpackendem kosmetischem Mittel existiert eine breite Vielfalt marktüblicher Verpackungen in den unterschiedlichsten Formen und Größen.

Die erfindungsgemäßen verpackten Mundwasserzusammensetzungen können in einem Kit mit einer Umverpackung versehen werden - aus Kostengründen wird dies aber nur bei höherwertigen und höherpresigen erfindungsgemäßen Mitteln praktiziert werden.

Die Umverpackung kann wie die Verpackung aus den unterschiedlichsten Materialien gefertigt werden und die unterschiedlichsten Formen annehmen. Für höherwertige Produkte kann sich eine Weißblechdose mit abnehmbaren Deckel empfehlen. Auch Kunststoffboxen sind mögliche Ausführungsformen. Diese Umverpackungen haben den Vorteil, entsprechend dekoriert und nach dem Verbrauch als Aufbewahrungsbehälter genutzt werden zu können. Für Produkte, deren Umverpackung nach dem Gebrauch entsorgt werden soll, empfiehlt sich eine Kartonage. Kartons besitzen den Vorteil der geringen Kosten und der leichten Bedruckbarkeit. Farbliche und formliche Ausgestaltungen können bei diesen Umverpackungen breit variiert werden.

Erfindungsgemäße Kits, die mindestens eine die Mundwasserzusammensetzung direkt umgebende Verpackung und eine diese Verpackung enthaltende Umverpackung aufweisen, die vorzugsweise ausgewählt ist aus Kartonagen, sind erfindungsgemäß bevorzugt.

Als weiteren Bestandteil umfaßt das oben genannte erfindungsgemäße Kit eine Gebrauchsanweisung, die dem Verbraucher Anwendungshinweise zu der Mundwasserzusammensetzung gibt. "Gebrauchsanweisung" im Sinne der vorliegenden Anmeldung können nicht nur schriftliche Ausführungen in einer beliebigen Sprache sein, sondern auch bildliche Darstellungen wie beispielsweise Piktogramme.

Die Gebrauchsanweisung kann fest mit der Verpackung verbunden oder auch auf ihr angebracht sein. So kann die Gebrauchsanweisung in einer Ausführungsform der vorliegenden Erfindung außen auf die Verpackung aufgebracht werden. Die Gebrauchsanweisung kann in bevorzugten Ausführungsformen der vorliegenden Erfindung auch auf die Innenseite der Verpackung aufgebracht werden. Diese Ausführungsform empfiehlt sich insbesondere bei erfindungsgemäßen Kits, welche eine Umverpackung aufweisen.

"Aufgebracht" ist eine Gebrauchsanweisung im Sinne der vorliegenden Erfindung auf eine Verpackung dann, wenn sie mit der Verpackung haftfest verbunden ist. Im einfachsten Fall kann die Gebrauchsanweisung direkt auf die Außen- und/oder Innenseite der Verpackung gedruckt werden. Es ist aber auch möglich und bevorzugt, separat vorgefertigte Gebrauchsanweisungen, die duftend ausgestaltet sein können, haftfest mit der Verpackung zu verbinden. Möglichkeiten hierzu bestehen unter anderem im Aufkleben von Etiketten, Aufschrumpfen von Folien, der mechanischen Befestigung von Etiketten, Papieren, Kartons, Kärtchen usw. mittels Rast-, Schnapp- oder Klemmverbindungen. Der Gestaltungsspielraum für Verpackungsentwickler ist hier nahezu grenzenlos.

Selbstverständlich ist es auch möglich, die Gebrauchsanweisung als separates Beiblatt auszugestalten, welches in die Verpackung gegeben wird. Diese Ausführungsform empfiehlt sich wiederum bei erfindungsgemäßen Kits, welche eine zusätzliche Umverpackung aufweisen. Die separate Gebrauchsanweisung kann auf Papier, Karton, Kunstoffen usw. in Form von Bögen, Blättern, Karten, Kärtchen, Flyern, Leporellos usw. gefertigt werden. Die separate Gebrauchsanweisung hat den Vorteil, daß der Verbraucher diese an exponierter Stelle befestigen und bei der Anwendung des Mittels wiederholt lesen kann. Auch kann eine solche separate Gebrauchsanweisung aus der Verpackung genommen und mehrfach an wechselnden Orten gelesen werden, ohne daß immer das komplette Kit mitgeführt werden muß.

Erfindungsgemäße Kits, bei denen die Gebrauchsanweisung als separate Beilage ausgestaltet ist, sind eine weitere bevorzugte Ausführungsform der vorliegenden Erfindung.

Vorzugsweise leitet die Gebrauchsanweisung den Verbraucher an, das erfindungsgemäße Verfahren anzuwenden.

Bezüglich bevorzugter erfindungsgemäßer Verfahren gilt mutatis mutandis das zu den erfindungsgemäßen Mundwasserzusammensetzungen Gesagte.

Die erfindungsgemäßen Zusammensetzungen führen bei ihrer Anwendung im Rahmen des erfindungsgemäßen Verfahrens zu eine Reihe von Verbrauchervorteilen, wobei die nachfolgende Liste nicht abschließend ist. Insbesondere bevorzugt ist die Verwendung von erfindungsgemäßen gebrauchsfertigen Mundwasserzusammensetzungen
- zur Reinigung der Mundhöhle und/oder
- zur Reduzierung von Plaque und/oder
- zur Vorbeugung gegen und/oder Reduzierung von Mundgeruch und/oder
- zur prophylaktischen Vorbeugung vor Karies und/oder
- zur prophylaktischen Vorbeugung vor Gingivitis und/oder
- zur prophylaktischen Vorbeugung vor Periodontitis und/oder
- zur prophylaktischen Vorbeugung vor Aphthen und/oder
- zur prophylaktischen Vorbeugung vor Gebißreizungen und/oder
- zur prophylaktischen Vorbeugung vor Mucosainfektionen und/oder
- zur prophylaktischen Vorbeugung vor Herpes stomatitis.

Auch bezüglich bevorzugter erfindungsgemäßer Verwendungen gilt mutatis mutandis das zu den erfindungsgemäßen Mundwasserzusammensetzungen Gesagte.

## Patentansprüche

1. Gebrauchsfertige Mundwasserzusammensetzung, enthaltend bezogen auf ihr Gewicht
c) 10 bis 40 Gew.-% Ethanol;
d) mindestens eine scharf schmeckende und/oder ein Gefühl von Wärme erzeugende Substanz.

2. Mundwasserzusammensetzung nach Anspruch 1, **dadurch gekennzeichnet, daß** sie bezogen auf ihr Gewicht 12,5 bis 35 Gew.-%, vorzugsweise 15 bis 30 Gew.-% und insbesondere 17,5 bis 25 Gew.-% Ethanol enthält.

3. Mundwasserzusammensetzung nach einem der Ansprüche 1 oder 2, **dadurch gekennzeichnet, daß** sie die scharf schmeckende(n) und/oder ein Gefühl von Wärme erzeugende(n) Substanz(en) in Mengen von 0,00001 bis 5 Gew.-%, vorzugsweise von 0,0005 bis 2,5 Gew.-%, weiter bevorzugt von 0,001 bis 1 Gew.-%, besonders bevorzugt von 0,005 bis 0,75 Gew.-% und insbesondere von 0,01 bis 0,5 Gew.-%, jeweils bezogen auf das Gewichts des gesamten Mittels, enthält.

4. Mundwasserzusammensetzung nach einem der Ansprüche 1 bis 3, **dadurch gekennzeichnet, daß** sie mindestens einen Scharfstoff aus der Gruppe der N-Alkyl-substituierte Amide von ungesättigten Carbonsäuren, vorzugsweise
- 2E,6Z,8E-Decatriensäure-N-isobutylamid (Spilanthol) und/oder
- 2E,4E,8Z-Decatriensäure-N-isobutylamid und/oder
- 2E,7Z,9E-Undecatriensäure-N-isobutylamid und/oder
- 2E,4Z-Decadiensäure-N-isobutylamid (cis-Pellitorin) und/oder
- 2E,4E-Decadiensäure-N-isobutylamid (trans-Pellitorin) und/oder
- Ferulasäure-N-Vanillylamid und/oder
- N-[2-(4-Hydroxy-3-methoxyphenyl)ethyl]-3-(4-hydroxy-3-methoxyphenyl)- (2*E*)-propensäureamid (trans-Feruloylmethoxytyramin) und/oder
- N-[2- (4-Hydroxy-3-methoxyphenyl)ethyl]-3-(4-hydroxy-3-methoxyphenyl)-(2 Z)-propensäureamid (cis-Feruloylmethoxytyramin) und/oder
- N-[2-(4- Hydroxy-3-methoxyphenyl)ethyl]-3-(4-hydroxy-3-methoxyphenyl)- propansäureamid (Dihydroferuloylmethoxytyramin) und/oder
- *N*-[2-(3,4- Dihydroxyphenyl)ethyl]-3-(4-hydroxy-3-methoxy-phenyl)-(2*E*)-propensäureamid (trans-Feruloyldopamin) und/oder
- N-[2-(3,4-Dihydroxyphenyl) ethyl]-3-(4-hydroxy-3-methoxy-phenyl)-(2Z)-propensäureamid (cis- Feruloyldopamin) und/oder
- N-[2-(4-Hydroxyphenyl)ethyl]-3-(3,4- dihydroxyphenyl)-(2*E*)-propensäureamid (trans-Caffeoyltyramin) und/oder
- N-[2-(4-Hydroxyphenyl)ethyl]-3-(3,4-dihydroxyphenyl)-(2*Z*)-propensäureamid (cis-Caffeoyltyramin) und/oder
- N-[2-(3,4-Dimethoxyphenyl) ethyl]-3-(3,4-dimethoxyphenyl)-(2*E*)-propensäureamid (trans- Rubenamin) und/oder
- N-[2-(3,4-dimethoxyphenyl)ethyl]-3-(3,4- dimethoxy-phenyl)-(2*Z*)-propensäureamid (cis-Rubenamin)
enthält.

5. Mundwasserzusammensetzung nach einem der Ansprüche 1 bis 4, **dadurch gekennzeichnet, daß** sie mindestens einen Scharfstoff aus den Gruppen der
- alkylsubstituierten Dioxane der Formel 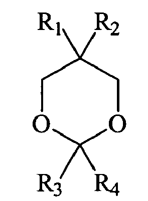 in der R1 und R2 unabhängig voneinander ausgewählt sind aus -H, -CH₃, - CH₂CH₃ und R3 und R4 unabhängig voneinander ausgewählt sind aus -H, -CH₃, - CH₂CH₃, -CH₂CH₂CH₃, -CH(CH₃)₂
und/oder
- Phenylestern der Formel 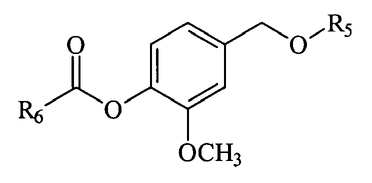 in der R5 für -CH₃ oder einen geradkettigen oder verzweigten Alkyl- oder Alkenylrest mit 2 bis 8 Kohlenstoffatomen und R6 für -CH₃ oder einen geradkettigen oder verzweigten Alkyl- oder Alkenylrest mit 2 bis 8 Kohlenstoffatomen oder eine Alkoxygruppe mit 1 bis 3 Kohlenstoffatomen steht
und/oder
- Carvonacetalen der Formel 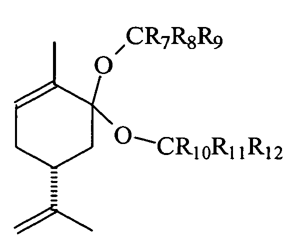 in der R7 bis R12 unabhängig voneinander ausgewählt sind aus -H, -CH₃, -CH₂CH₃, -CH₂CH₂CH₃, -CH(CH₃)₂, -CH₂ CH₂CH₂CH₃, -CH₂CH(CH₃)₂, -CH(CH₃)CH₂CH₃, - C(CH₃)₃ oder R9 und R10 zusammen eine chemiche Bindung oder eine Gruppe - (CR13R14)ₓ bedeuten, worin x für die Werte 1 oder 2 steht und R13 und R14 unabhängig voneinander ausgewählt sind aus -H, -CH₃, -CH₂CH₃, -CH₂CH₂CH₃,-CH(CH₃)₂, -CH₂ CH₂CH₂CH₃, -CH₂CH(CH₃)₂, -CH(CH₃)CH₂CH_{3,}-C(CH₃)₃
enthält.

6. Mundwasserzusammensetzung nach einem der Ansprüche 1 bis 5, **dadurch gekennzeichnet, daß** sie zusätzlich 0,05 bis 5 Gew.-%, vorzugsweise 0,1 bis 2,5 Gew.-% und insbesondere 0,15 bis 1,2 Gew.-% nichtionische(s) Tensid(e), vorzugsweise ethoxylierte(s) nichtionische(s) Tensid(e) und insbesondere ethoxylierte(s) nichtionische(s) Tensid(e) mit 40 bis 100, vorzugsweise 50 bis 90 und insbesondere 60 bis 80 EO-Gruppen, enthält.

7. Mundwasserzusammensetzung nach einem der Ansprüche 1 bis 6, **dadurch gekennzeichnet, daß** sie zusätzlich Antikaries-Wirkstoffe, vorzugsweise Fluorverbindung(en), insbesondere Natriumfluorid, Kaliumfluorid, Natriummonofluorphosphat, Zinkfluorid, Zinnfluorid und Natriumfluorosilikat, vorzugsweise in Mengen von 0,01 bis 2,5 Gew.%, vorzugsweise von 0,02 bis 1 Gew.% und insbesondere von 0,03 bis 0,5 Gew.%, jeweils bezogen auf das gesamte Mittel, enthält.

8. Mundwasserzusammensetzung nach einem der Ansprüche 1 bis 7, **dadurch gekennzeichnet, daß** sie die Unempfindlichkeit der Zähne steigernde Substanzen, vorzugsweise Kaliumsalze, besonders bevorzugt Kaliumnitrat und/oder Kaliumcitrat und/oder Kaliumchlorid und/oder Kaliumbicarbonat und/oder Kaliumoxalat, vorzugsweise in Mengen von 0,05 bis 5 Gew.%, besonders bevorzugt von 0,1 bis 2,5 Gew.%, weiter bevorzugt von 0,2 bis 1 Gew.-% und insbesondere von 0,25 bis 0,75 Gew.%, jeweils bezogen auf das gesamte Mittel, enthält.

9. Mundwasserzusammensetzung nach einem der Ansprüche 1 bis 8, **dadurch gekennzeichnet, daß** sie zusätzlich Antiplaque-Wirkstoffe, vorzugsweise p-Hydroxybenzoesäuremethyl-, -ethyl- oder propylester, Natriumsorbat, Natriumbenzoat, Bromchlorophen, Triclosan, Phenyl- Salicylsäureester, Biguanide z. B. Chlorhexidin, Thymol, vorzugsweise in Mengen von 0,01 bis 2,5 Gew.%, vorzugsweise von 0,05 bis 1 Gew.% und insbesondere von 0,1 bis 0,5 Gew.%, jeweils bezogen auf das gesamte Mittel, enthält.

10. Verfahren zur Reinigung der Mundhöhle, **dadurch gekennzeichnet, daß** eine gebrauchsfertige Mundwasserzusammensetzung nach einem der Ansprüche 1 bis 9 unverdünnt in die Mundhöhle eingebracht und nach einer Einwirkzeit wieder aus ihr entfernt wird.

11. Verwendung von gebrauchsfertigen Mundwasserzusammensetzungen nach einem der Ansprüche 1 bis 9
- zur Reinigung der Mundhöhle und/oder
- zur Reduzierung von Plaque und/oder
- zur Vorbeugung gegen und/oder Reduzierung von Mundgeruch und/oder
- zur prophylaktischen Vorbeugung vor Karies und/oder
- zur prophylaktischen Vorbeugung vor Gingivitis und/oder
- zur prophylaktischen Vorbeugung vor Periodontitis und/oder
- zur prophylaktischen Vorbeugung vor Aphthen und/oder
- zur prophylaktischen Vorbeugung vor Gebißreizungen und/oder
- zur prophylaktischen Vorbeugung vor Mucosainfektionen und/oder
- zur prophylaktischen Vorbeugung vor Herpes stomatitis.
